# EUROPEAN PATENT APPLICATION

(11) **EP 3 792 250 A1**
(43) Date of publication of application: **17.03.2021**
(21) Application number: 19197390.8
(22) Date of filing: 13.09.2019
(51) Int. Cl.: C07D 209/90, C07C 227/20, C07C 271/22, C07K 1/06, C07K 1/10, C07K 1/113, C07K 7/06, C07K 7/50

(54) **SYNTHESIS OF ALPHA-AMANITIN AND ITS DERIVATIVES**

(71) Applicant: Pure Bioorganics SIA, 3124 Pure Tukuma Novads (LV)
(72) Inventor: SIEGERT, Mary-Ann, 12157 Berlin (DE); KNITTEL, Caroline Herta, 10961 Berlin (DE); SÜSSMUTH, Roderich, 10629 Berlin (DE)
(74) Representative: Schulz Junghans Patentanwälte PartGmbB

(57) **Abstract**

The present invention relates to the chemical synthesis of α-amanitin and its derivatives. The present invention also relates to intermediate products of the α-amanitin synthesis.

## Description

The present invention relates to the chemical synthesis of α-amanitin and its derivatives. The present invention also relates to intermediate products of the α-amanitin synthesis.

### Description

The objective of the present invention is to provide means and methods to chemically synthesize amanitin or derivatives thereof. This objective is attained by the subject-matter of the independent claims of the present specification.

### Terms and definitions

Amino acid sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids.

The term "protecting group" in the context of the present specification relates to a moiety covalently attached to a functional group (particularly the carboxylic acid moiety, the amino moiety or the hydroxyl moiety of the molecules discussed herein) that can be selectively attached to the functional group and selectively removed without affecting the integrity or chiral orientation of the carbon backbone of the molecule the protecting group is attached to, nor cleaving particular other protecting groups attached to other protecting groups attached to the molecule.

The term "deprotection agent" in the context of the present specification relates to an agent which is able to cleave a certain protecting group. The skilled person is able to select the deprotection agent according to the protecting group. The conditions under which the protecting group is cleavable constitute the deprotection agent, e.g. if the protecting group is cleavable under acidic conditions, then the deprotection agent is an acid.

The term "preactivated carboxylic group" in the context of the present specification relates to a carboxylic moiety being reacted into an active ester susceptible for the nucleophilic attack of an amine group in order to form a peptide bond.

The term "preactivated amino group" in the context of the present specification relates to an amino group being reacted into a N-trimethylsilyl amine with increased nucleophilicity to attack a carboxylic acid moiety in order to form a peptide bond.

A comprehensive review of modern protecting group chemistry, particularly as it pertains to the compounds disclosed herein, is available in Peter G. M. Wuts, Greene's Protective Groups in Organic Synthesis, 5th Edition, Wiley 2014.

US 6693178 B2 - "Protecting groups useful in the synthesis of polysaccharides, natural products, and combinatorial libraries" and US 20160024143 A1 - "Deprotection method" are incorporated herein by reference.

Standard convention of organic chemistry, by which a non-designated position in a formula is deemed to be a saturated carbon, is followed herein.

A first aspect of the invention relates to a method for preparation of a compound of formula (Iox) 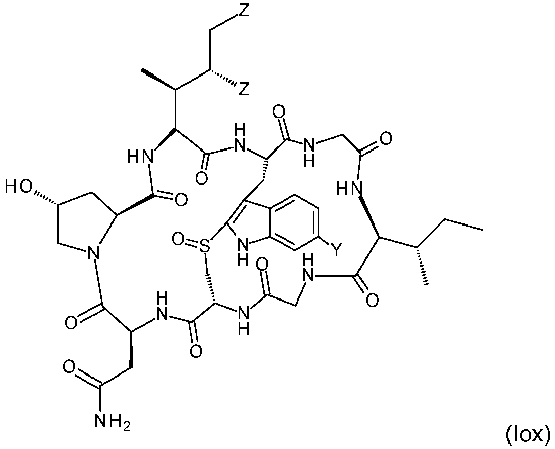 wherein
a) a compound of formula (IIox) 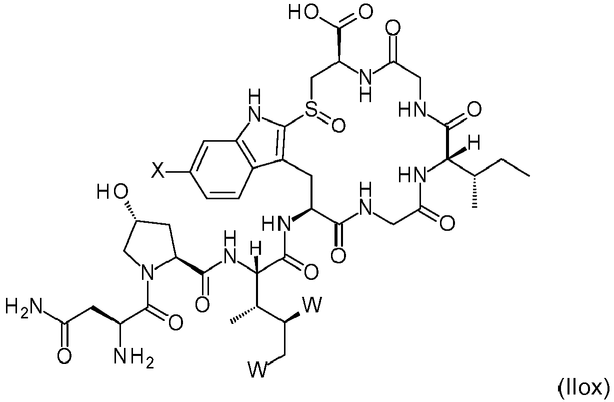 wherein
   X and Y are H, or
   Y is OH and X is OR^{PGP} wherein R^{PGP} is a protecting group for phenolic OH groups, particularly a phenolic OH-protecting group not acid- or alkali-labile, more particularly cleavable under reductive conditions, most particularly benzyl (Bn) or X and Y are selected from F, Cl, Br, and I,
   particularly X and Y are H or Y is OH and X is OR^{PGP}
   Z and W are H, or
   Z is OH and W is OR^{PGOH}, wherein R^{PGOH} is a protecting group for hydroxyl-groups, particularly a hydroxyl-protecting group cleavable with fluoride ions, more particularly TBS, TMS, TES, TBDPS, TIPS, or disiloxane, most particularly TBS,
   is reacted with a peptide bond forming reagent,
   particularly with a coupling reagent selected from a carbodiimide, an imidazolinium reagent, a phosphonium salt, an organo-phosphorous reagent, an uronium salt, a pyridinium reagent, and a phosphonic acid,
   more particularly with HATU [1-[bis(dimethylamino)methylene]-1H-1,2,3-triazolo[4,5-b]pyridinium 3-oxide hexafluorophosphate], COMU, HBTU, TBTU, TOMBU, COMBU, or HCTU,
   in a reaction step (a1),
   and for Y being OH and/or Z being OH, the compound is reacted with a deprotection agent removing R^{PGP} and/or R^{PGOH},
   or wherein
b) the compound of formula (II) 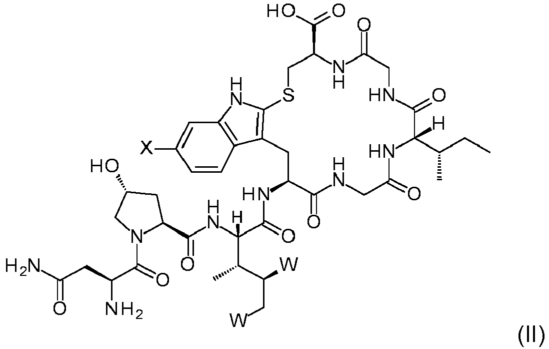 wherein
   X, Y, Z and W have the same meanings as defined above,
   is reacted with a peptide bond forming reagent,
   particularly with HATU
   in a reaction step (a2),
   yielding a compound of formula (I) 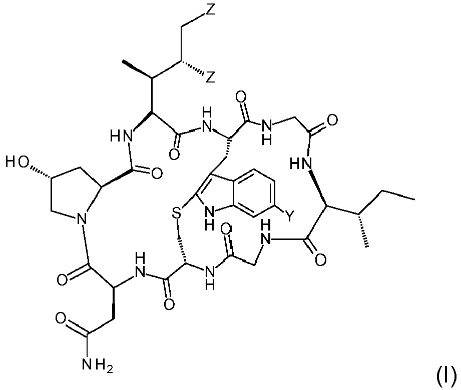 wherein the sulfur atom is subsequently oxidized,
   i. using manganese ions, more particularly the compound is reacted with a compound of formula (XXII) 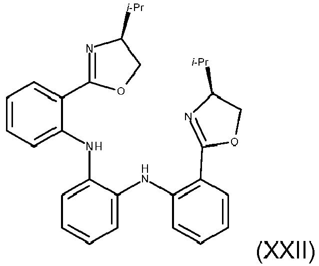 and with Mn(OTf)₂ and H₂O₂,
   ii. using PPO, dibenzyolperoxide, tert-butyl peroxybenzoate, or lauroyl peroxide; or
   iii. using iodine and oxygen;
in a reaction step (b2),
and for Y being OH and/or Z being OH, the compound is reacted with a deprotection agent removing R^{PGP} and/or R^{PGOH}, particularly for R^{PGP} with reductive conditions and for R^{PGOH} with fluoride ions,
to yield the compound characterized by (lox).

For cyclisation, the amide-NH₂ of compound (II) or (IIox) does not need to be protected. No significant side reactions were observed without protecting group.

In certain embodiments, the oxidation of the sulfur atom is performed using manganese ions. In certain embodiments, the chemoselective oxidation of the sulfur atom is performed using a compound of formula (XXII) 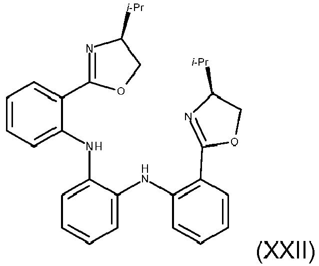 with Mn(OTf)₂ and H₂O₂.

In certain embodiments, the chemoselective oxidation of the sulfur atom is performed using PPO (Phthaloyl peroxide), dibenzyolperoxide, tert-butyl peroxybenzoate, or lauroyl peroxide. Preparation of PPO is described in (S. Gan, J. Yin, Y. Yao, Y. Liu, D. Chang, D. Zhu, L. Shi, Org. Biomol. Chem. 2017, 15, 2647-2654.).

In certain embodiments, the oxidation of the sulfur atom is performed with *m*CPBA (meta-chloroperoxybenzoic acid) in isopropanol/ethanol (8:3).

In certain embodiments, the oxidation of the sulfur atom is performed with an oxaziridinium salt as described in (Rio et al, Org. Lett. 2007, 9,12, 2265-2268).

In certain embodiments, the oxidation of the sulfur atom is performed with non-enantioselective agents or simply with oxygen or hydrogenperoxide.

In certain embodiments, the oxidation of the sulfur atom is performed with iodine and oxygen.

A second aspect relates to a method for preparation of a compound of formula (I) 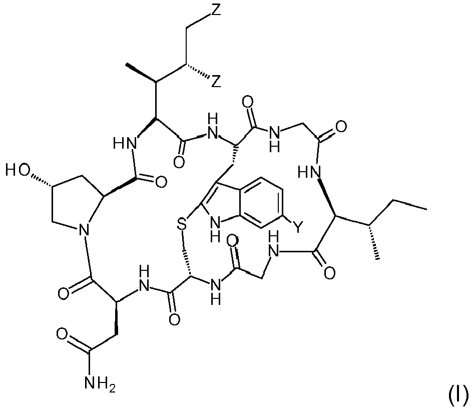 wherein a compound of formula (II) 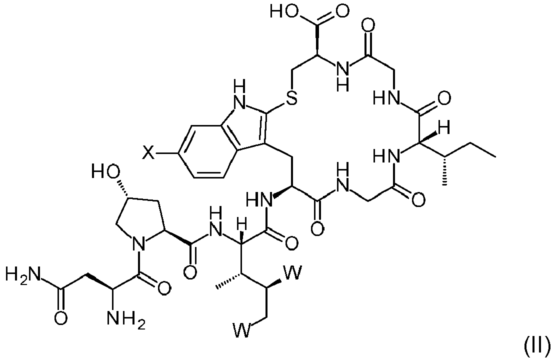 wherein
X and Y are H, or
Y is OH and X is OR^{PGP} wherein R^{PGP} is a protecting group for phenolic OH groups, particularly a phenolic OH-protecting group not acid- or alkali-labile, more particularly cleavable under reductive conditions, most particularly benzyl or
X and Y are selected from F, Cl, Br, and I,
particularly X and Y are H, or Y is OH and X is OR^{PGP},
Z and W are H, or
Z is OH and W is OR^{PGOH}, wherein R^{PGOH} is a protecting group for hydroxyl-groups, particularly a hydroxyl-protecting group cleavable with fluoride ions, more particularly TBS
is reacted with a coupling reagent selected from a carbodiimide, an imidazolinium reagent, a phosphonium salt, an organo-phosphorous reagent, an uronium salt, a pyridinium reagent, and a phosphonic acid,
particularly with a peptide bond forming reagent,
more particularly with HATU, COMU, HBTU, TBTU, TOMBU, COMBU, or HCTU
in a reaction step (a),
and for Y being OH and/or Z being OH, the compound is reacted with a deprotection agent removing R^{PGP} and/or R^{PGOH} in a reaction step (b)
to yield the compound characterized by (I).

In certain embodiments, a compound of formula (III) 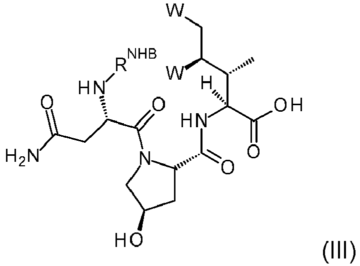 and a compound of formula (IV) or (IVox) 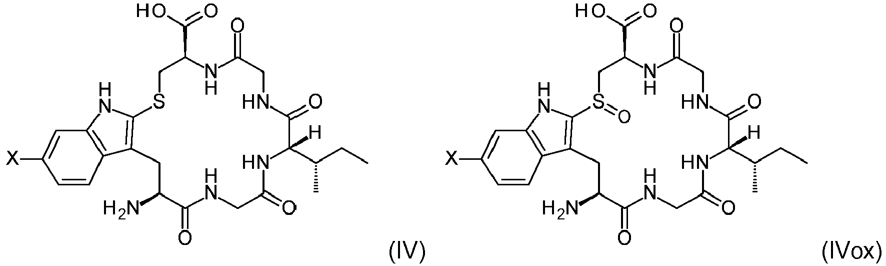 wherein
R^{NHB} is an amino protecting group, particularly an amino protecting group cleavable under alkaline conditions, more particularly Fmoc, or an amino protecting group cleavable with hydrogenolysis, particularly Cbz, most particularly R^{NHB} is Fmoc
W and X have the same meaning as outlined above,
wherein
the amino-group of (IV) or (IVox) is preactivated, particularly with MSA, and preactivated (IV) or preactivated (IVox) and (III) are reacted with a peptide bond forming reagent, particularly with HATU, or
the amino-group of (IV) or (IVox) is preactivated, particularly with MSA, and the carboxyl-group of compound (III) is preactivated, particularly with an O-PFP-ester, O-PCP-ester, or OSu-ester, and preactivated (IV) or preactivated (IVox) and preactivated (III) are reacted
in a reaction step (c),
and the compound is reacted with a deprotection agent removing R^{NHB} in a reaction step (d), particularly with a base if R^{NHB} is Fmoc, or with hydrogenolysis if R^{NHB} is Cbz, more particularly with Et₂NH, tris-2-amino-ethylamin, DBU, morpholine, or piperidine if R^{NHB} is Fmoc,
to yield the compound characterized by (II) or (IIox).

For coupling compounds (II) and (IV) or (IVox), the acid-COOH group of compound (IV) or (IVox) does not need to be protected. No significant side reactions were observed without protecting group.

In certain embodiments, a compound of formula (IV) 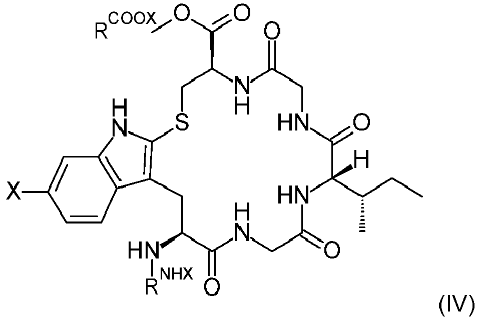 wherein
R^{COOX} is a carboxyl-protecting group, particularly *t*Butyl,
R^{NHX} is an amino-protecting group, particularly Teoc,
X has the same meaning as outlined above,
wherein the sulfur atom is subsequently oxidized, particularly
i. using manganese ions, more particularly the compound is reacted with a compound of formula (XXII) 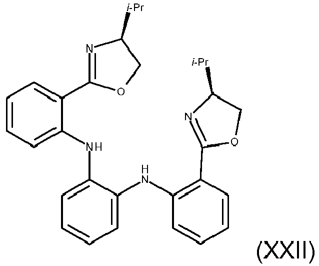 and with Mn(OTf)₂ and H₂O₂,
ii. using PPO, dibenzyolperoxide, tert-butyl peroxybenzoate, or lauroyl peroxide; or
iii. using iodine and oxygen;
and with Mn(OTf)₂ and H₂O₂ in a reaction step (d2),
and the compound is reacted with a deprotection agent removing R^{COOX} and R^{NHX}, particularly with a strong acid, more particularly at a pH of -3 to 2, most particularly with 80-95% TFA,
to yield the compound characterized by (IVox).

In certain embodiments, the oxidation of the sulfur atom is performed using manganese ions. In certain embodiments, the chemoselective oxidation of the sulfur atom is performed using a compound of formula (XXII) 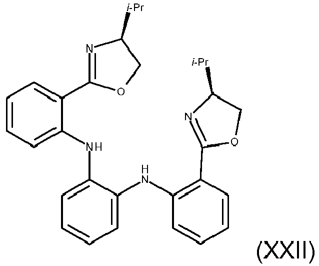 with Mn(OTf)₂ and H₂O₂.

In certain embodiments, the chemoselective oxidation of the sulfur atom is performed using PPO (Phthaloyl peroxide), dibenzyolperoxide, tert-butyl peroxybenzoate, or lauroyl peroxide. Preparation of PPO is described in (S. Gan, J. Yin, Y. Yao, Y. Liu, D. Chang, D. Zhu, L. Shi, Org. Biomol. Chem. 2017, 15, 2647-2654.).

In certain embodiments, the oxidation of the sulfur atom is performed with *m*CPBA (meta-chloroperoxybenzoic acid) in isopropanol/ethanol (8:3).

In certain embodiments, the oxidation of the sulfur atom is performed with an oxaziridinium salt as described in (Rio et al, Org. Lett. 2007, 9,12, 2265-2268).

In certain embodiments, the oxidation of the sulfur atom is performed with non-enantioselective agents or simply with oxygen or hydrogenperoxide.

In certain embodiments, the oxidation of the sulfur atom is performed with iodine and oxygen.

In certain embodiments, a compound of formula (V) 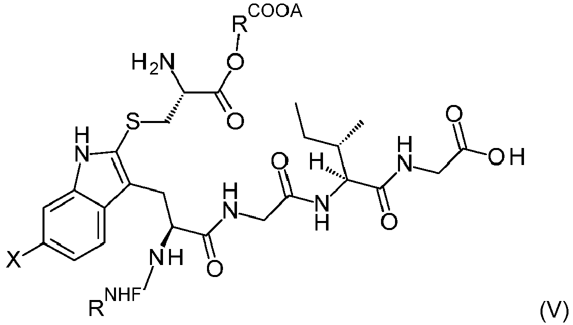 wherein
R^{NHF} is an amino protecting group, particularly an amino protecting group cleavable with fluoride ions or strong acids, more particularly Teoc,
R^{COOA} is a carboxyl-protecting group, particularly a carboxyl-protecting group cleavable under strongly acidic conditions, more particularly *tert*-butyl,
X has the same meaning as outlined above,
is reacted with a coupling reagent selected from a carbodiimide, an imidazolinium reagent, a phosphonium salt, an organo-phosphorous reagent, an uronium salt, a pyridinium reagent, and a phosphonic acid,
particularly a peptide bond forming reagent, more particularly with T3P, HATU, COMU, HBTU, TBTU, TOMBU, COMBU, or HCTU, in a reaction step (e),
and the compound is reacted with a deprotection agent removing R^{NHF} and R^{COOA} in a reaction step (f), particularly with TFA,
to yield the compound characterized by (IV).

In certain embodiments, a compound of formula (VI) 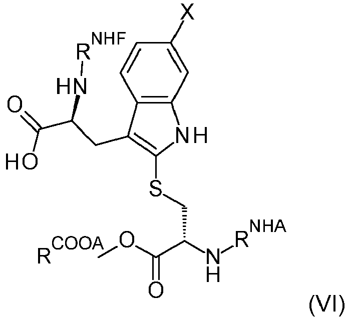 and a compound of formula (VII) 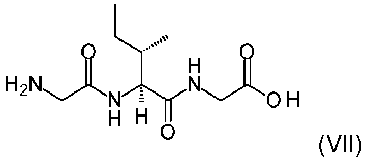 wherein
R^{NHA} is an amino protecting group, particularly an amino protecting group cleavable under acidic conditions, more particularly Boc,
R^{COOA} , R^{NHF} and X have the same meaning as outlined above,
wherein compound (VI) is
preactivated with a peptide bond forming reagent, particularly with HATU, COMU, HBTU, TBTU, TOMBU, COMBU, or HCTU, followed by a reaction with the silylated compound (VII), or
is preactivated as in OSu-ester, followed by a reaction with the compound (VII)
in a reaction step (g),
and the compound is reacted with a deprotection agent removing R^{NHA} in a reaction step (h), particularly with acidic conditions, more particularly at a pH of -3 to 0, even more particularly with HCI or p-toluenesulfonic acid, most particularly with 2 M HCI in Dioxan,
to yield the compound characterized by (V).

In certain embodiments, a compound of formula (VIII) 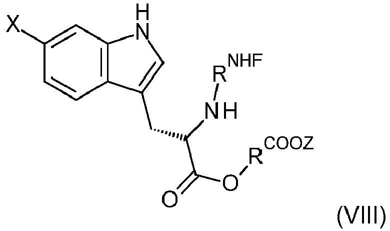 and a compound of formula (IX) 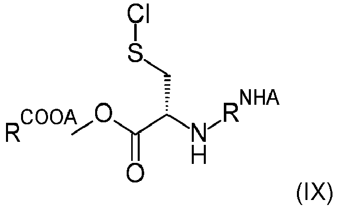 wherein
R^{COOZ} is a carboxyl-protecting group, particularly a carboxyl-protecting group cleavable with Zn, more particularly Tce, or R^{COOZ} is H,
R^{COOA}, R^{NHF}, R^{NHA} and X have the same meaning as outlined above,
are reacted in a reaction step (i), and if R^{COOZ} is a carboxyl-protecting group, the compound is reacted with a deprotection agent removing R^{COOZ} in a reaction step (j), particularly with Zn,
to yield the compound characterized by (VI).

A protection group strategy was applied that relies on acid stability. Decreasing pH values were used for deprotection. First, the Tce group of tryptophan (R^{COOZ} of compound VIII) was removed under reductive conditions using Zn with mildly acidic pH. Afterwards, the Boc group of cysteine (R^{NHA} of compound IX) was removed with p-toluenesulfonic acid. Last, Teoc (R^{NHF}) and tert-butyl (R^{COOA}) of compound (V) were removed concomitantly with 95%TFA.

In certain embodiments, a compound of formula (X) 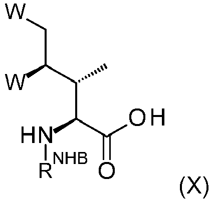 and a compound of formula (XI) 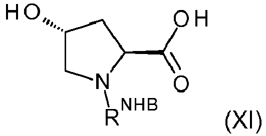 and a compound of formula (XII) 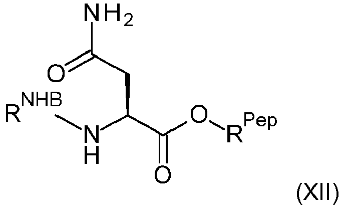 wherein
R^{Pep} is an active ester, particularly O-pentafluorophenol or OSu-ester,
R^{NHB} is an amino protecting group, particularly an amino protecting group cleavable under alkaline conditions, more particularly Fmoc,
are reacted with solid phase peptide synthesis in a reaction step (k), wherein the carboxyl-group of compound (XII) may be protected,
to yield the compound characterized by (III).

A third aspect relates to a method for preparation of a compound of formula (XIII), (XIIIC), (XIIIN), or (XIIICN) 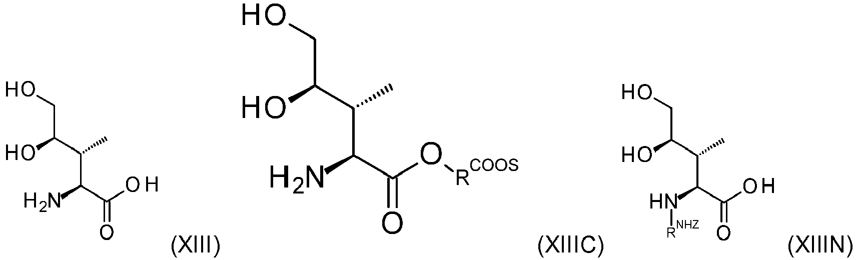 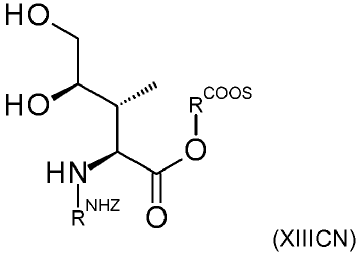 wherein a compound of formula (XIV) 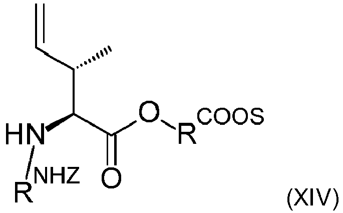 wherein
R^{COOS} is a carboxyl-protecting group, particularly a carboxyl-protecting group cleavable with silylating agents, more particularly *tert*-butyl,
R^{NHZ} is an amino protecting group, particularly an amino protecting group cleavable under alkaline conditions, more particularly Fmoc, or an amino protecting group cleavable under reductive conditions, more particularly trifluoroacetyl;
is reacted with Osmium(IV)-oxide in a reaction step (I), particularly in CHCl₃/H₂O, and optionally, the compound is reacted with a deprotection agent removing R^{NHR} and/or R^{COOS} in a reaction step (m), particularly with silylating agents for R^{COOS} and reductive conditions or alkaline conditions for R^{NHR}, more particularly with TMSOTf and Lutidine for R^{COOS} and/or sodium borohydride for R^{NHZ} [if R^{NHZ} is trifluoroacetyl] or alkaline conditions for R^{NHZ} [if R^{NHZ} is Fmoc],
to yield the compound characterized by (XIII), (XIIIC), (XIIIN), or (XIIICN).

When the compound of formula (XIII) is used in the synthesis of α-amanitin or its derivatives, either the deprotection of the amino-protecting group or of the carboxyl-protecting group is omitted, as shown in formula (XIIIC) or (XIIIN). In addition, the OH-groups of compound (XIII) are protected before proceeding with the synthesis.

The oxidation with Osmium(IV)-oxide is particularly stereoselective (2.5:1) in CHCl₃/H₂O. An Upjohn-dihydroxylation protocol is employed. Only the solvent influences the stereoselectivity here, as in e.g. *t*BuOH/H₂O mainly the opposite diastereomer of compound (XIII) is produced.

A fourth aspect relates to a method for preparation of a compound of formula (XV) 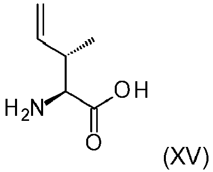 wherein a compound of formula (XVI) 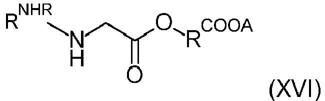 and a compound of formula (XVII) or (XVIIs) 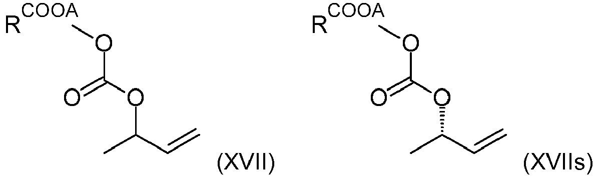 wherein
R^{NHR} an amino protecting group cleavable under reductive conditions, more particularly trifluoroacetyl,
R^{COOA} is a carboxyl-protecting group, particularly a carboxyl-protecting group cleavable under strongly acidic conditions, more particularly *tert*-butyl,
are reacted with [(p-cymene)RuCl₂]₂ in a reaction step (n) yielding the compound (XXIII) or (XXIV) 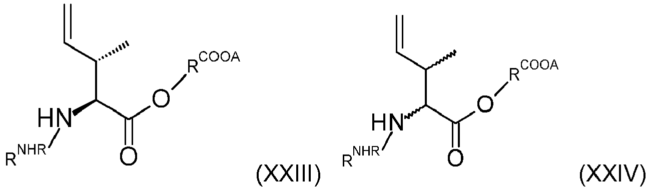
the compound (XXVI) is reacted with a deprotection agent removing R^{COOA} in a reaction step (o), and is reacted with acylase in a reaction step (p), or
the compound (XXIII) is reacted with a deprotection agent removing R^{COOA} in a reaction step (o), and is reacted with a deprotection agent removing R^{NHR} in a reaction step (q), particularly with reductive conditions, more particularly with sodium borohydride,
to yield the compound characterized by (XV).

The reaction with acylase in reaction step (p) removes the protecting group R^{NHR}.

In certain embodiments, the compound of formula (XXIII) is directly employed in the synthesis of the compound of formula (XIII), (XIIIC), or (XIIIN) without a deprotection step in between.

The chiral compound (XV) may be gained directly from the reaction with [(p-cymene)RuCl₂]₂. The reaction is particularly stereoselective when compound (XVIIs) is employed. Then, compound (XXIII) is gained and no acylase step is necessary. Stereoselectivity is improved compared to methods known from literature (e.g. A. Bayer, U. Kazmaier, Org. Lett. 2010, 12, 21, 4960-4963).

Otherwise, when using compound (XVII), compound (XXIV) in an 1:1 mixture of the (R,R) and the (S,S) diastereomers is gained. From this mixture, the correct diastereomer [compound (XV)] is gained with the use of acylase.

A fifth aspect relates to a method for preparation of a compound of formula (XVIII) 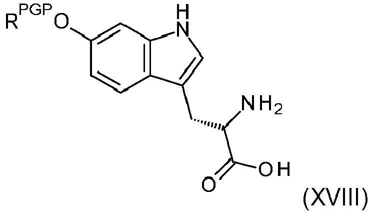 wherein a compound of formula (XIX) 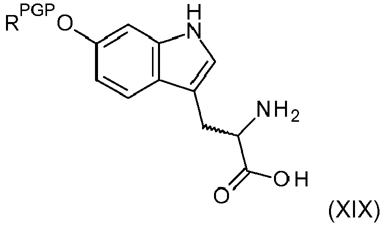 and a compound of formula (XX) 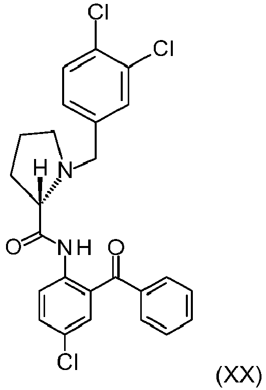 wherein
R^{PGP} is a protecting group for phenolic OH groups, particularly a phenolic OH-protecting group not acid- or alkali-labile, more particularly cleavable under reductive conditions, most particularly benzyl,
are reacted with Ni²⁺ in a reaction step (r)
to yield the compound characterized by (XVIII).

A sixth aspect relates to a method for preparation of a compound of formula (lox), wherein a compound of formula (I) 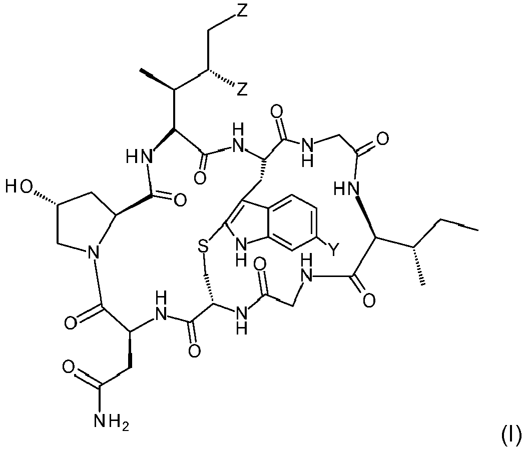 wherein the sulfur atom is oxidized,
i. using manganese ions, more particularly the compound is reacted with a compound of formula (XXII) 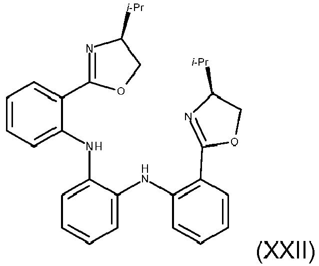 and with Mn(OTf)₂ and H₂O₂,
ii. using PPO, dibenzyolperoxide, tert-butyl peroxybenzoate, or lauroyl peroxide; or
iii. using iodine and oxygen;
yielding the compound (lox).

In certain embodiments, the oxidation of the sulfur atom is performed using manganese ions. In certain embodiments, the chemoselective oxidation of the sulfur atom is performed using a compound of formula (XXII) 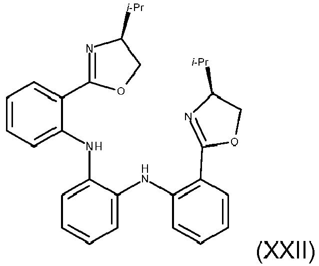 with Mn(OTf)₂ and H₂O₂.

In certain embodiments, the chemoselective oxidation of the sulfur atom is performed using PPO (Phthaloyl peroxide), dibenzyolperoxide, tert-butyl peroxybenzoate, or lauroyl peroxide. Preparation of PPO is described in (S. Gan, J. Yin, Y. Yao, Y. Liu, D. Chang, D. Zhu, L. Shi, Org. Biomol. Chem. 2017, 15, 2647-2654.).

In certain embodiments, the oxidation of the sulfur atom is performed with *m*CPBA (meta-chloroperoxybenzoic acid) in isopropanol/ethanol (8:3).

In certain embodiments, the oxidation of the sulfur atom is performed with an oxaziridinium salt as described in (Rio et al, Org. Lett. 2007, 9,12, 2265-2268).

In certain embodiments, the oxidation of the sulfur atom is performed with non-enantioselective agents or simply with oxygen or hydrogenperoxide.

In certain embodiments, the oxidation of the sulfur atom is performed with iodine and oxygen.

In certain embodiments, the method according to the third aspect is applied for the method of the first aspect. Compound (X) can be obtained from compound (XIII).

In certain embodiments, the method according to the fourth aspect is applied for the method of the third aspect. Compound (XIV) can be obtained from compound (XV).

In certain embodiments, the method according to the fifth aspect is applied for the method of the first aspect. Compound (VIII) can be obtained from compound (XVIII).

A seventh aspect of the invention relates to a method for preparation of a compound of formula (XXIII) or (XXIIIox) 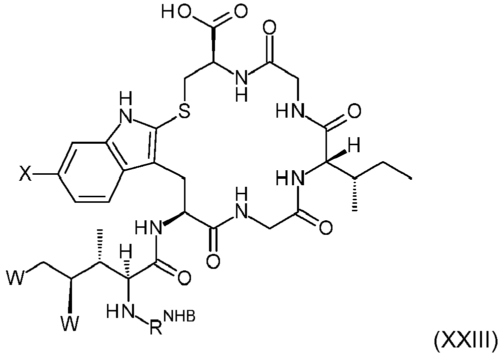 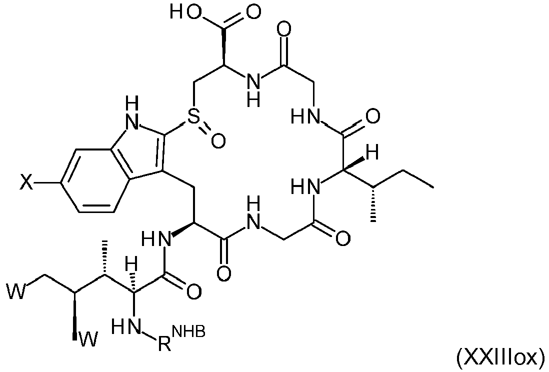 wherein a compound of formula (IV) or (IVox), respectively, 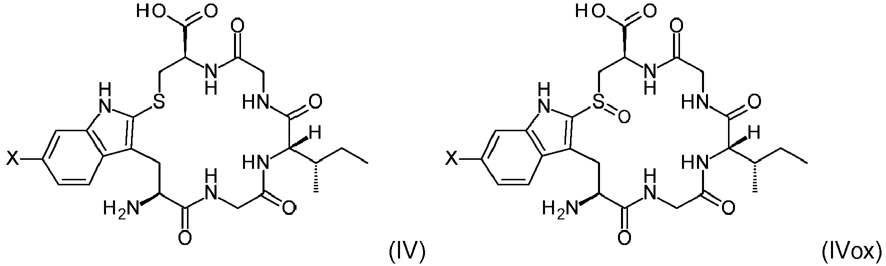 and a compound of formula (X) 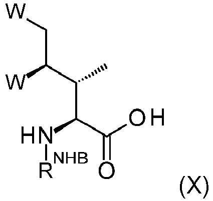 wherein X, W, and R^{NHB} have the same meanings as defined above,
wherein the amino-group of (IV) or (IVox) is preactivated, particularly with MSA, and preactivated (IV) or preactivated (IVox) and (X) are reacted with a peptide bond forming reagent, particularly with HATU, COMU, HBTU, TBTU, TOMBU, COMBU, or HCTU, more particularly with COMU, in a reaction step (s) to yield the compound (XXIII) or (XXIIIox), respectively.
An eighth aspect of the invention relates to a method for preparation of a compound of formula (XXVI) or (XXVIox) 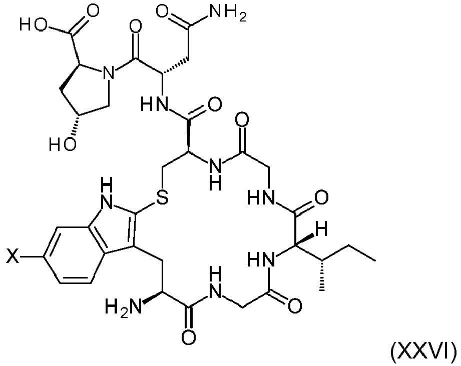 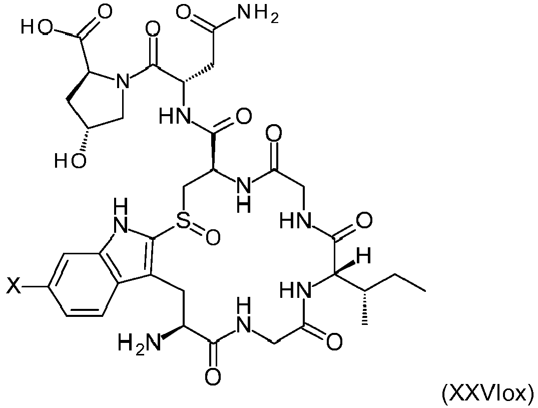 wherein a compound of formula (XXVIII) or (XXVIIIox), respectively, 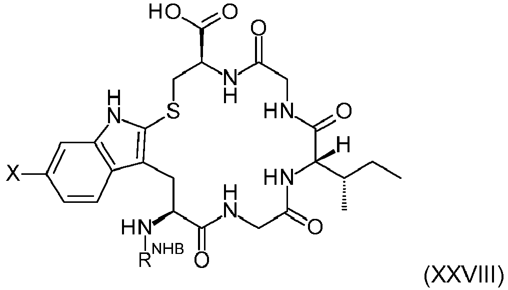 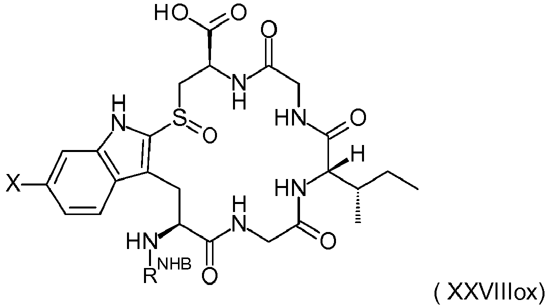 and a compound of formula (XXV) 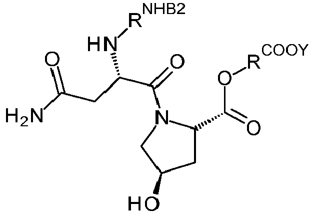 wherein
X, W, and R^{NHB} have the same meanings as defined above,
R^{NHB2} is an amino-protecting group, particularly an amino-protecting group cleavable under acidic conditions, more particularly Boc;
R^{COOY} is a carboxyl-protecting group, particularly fluorenylmethyl or benzyl, more particularly fluorenylmethyl;
wherein (IV) or (IVox) and (XXV) are reacted with a peptide bond forming reagent, particularly with HATU, COMU, HBTU, TBTU, TOMBU, COMBU, or HCTU, in a reaction step (t) to yield the compound (XXVI) or (XXVIox), respectively.

A ninth aspect of the invention relates to a method for preparation of a compound of formula (XXVII) or (XXVIIox) 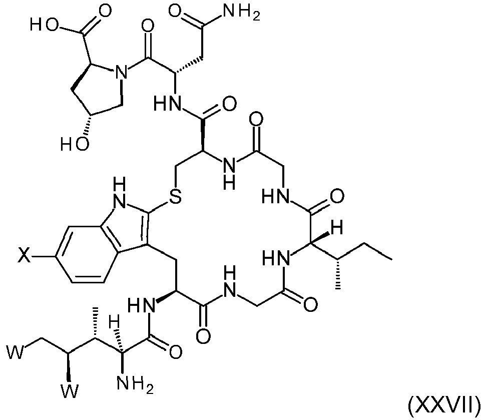 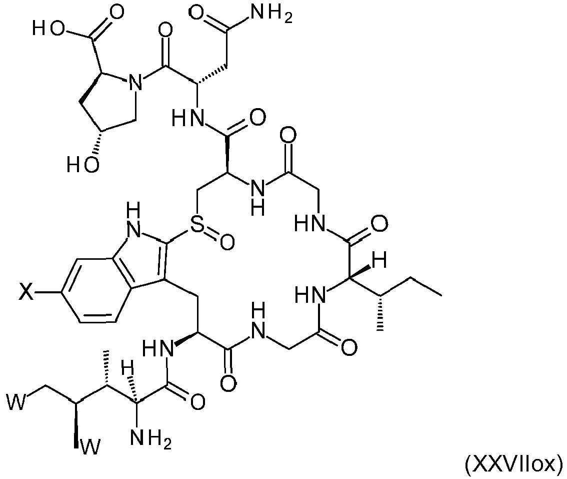 wherein
a compound of formula (IV) or (IVox), 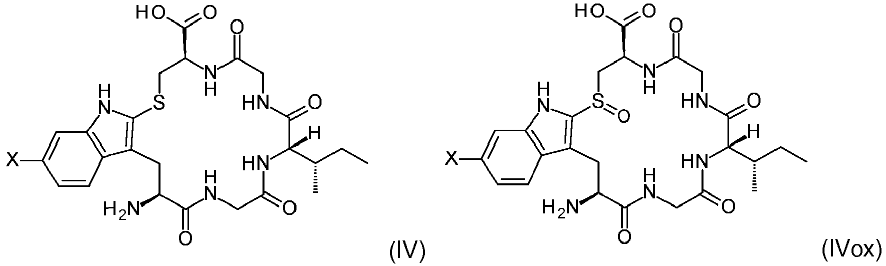 and a compound of formula (X) 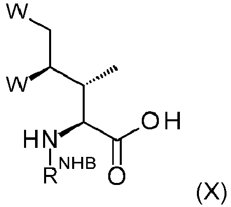
wherein X, W, and R^{NHB} have the same meanings as defined above,
wherein the amino-group of (IV) or (IVox) is preactivated, particularly with MSA, and preactivated (IV) or preactivated (IVox) and (X) are reacted with a peptide bond forming reagent, particularly with COMU, in a reaction step (s) to yield the compound (XXIII) or (XXIIIox), respectively,
and subsequently compound (XXIII) or (XXIIIox) and compound (XXV) are reacted with a peptide bond forming reagent, particularly with HATU, COMU, HBTU, TBTU, TOMBU, COMBU, or HCTU
in a reaction step (u) to yield the compound (XXVII) or (XXVIlox), respectively;
or
compound (XXIII) or (XXIIIox) and compound (XXV) are reacted with a peptide bond forming reagent, particularly with HATU, COMU, HBTU, TBTU, TOMBU, COMBU, or HCTU
in a reaction step (u) to yield the compound (XXVII) or (XXVIlox), respectively;
or
a compound of formula (XXVIII) or (XXVIIIox), respectively, 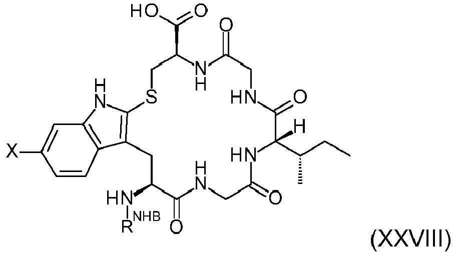 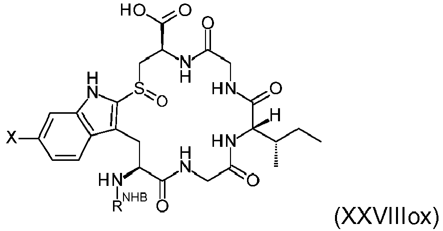 and a compound of formula (XXIX) 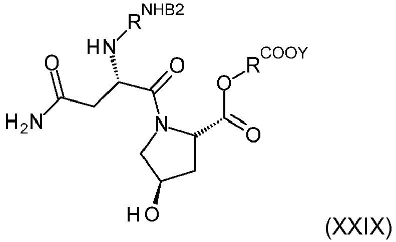 wherein
X, W, and R^{NHB} have the same meanings as defined above,
R^{NHB2} is an amino-protecting group, particularly an amino-protecting group cleavable under acidic conditions, more particularly Boc;
wherein R^{COOY} is a carboxyl-protecting group, particularly fluorenylmethyl or benzyl, more particularly fluorenylmethyl;
wherein (XXVIII) or (XXVIIIox) and (XXV) are reacted with a peptide bond forming reagent, particularly with HATU, COMU, HBTU, TBTU, TOMBU, COMBU, or HCTU,
in a reaction step (t) to yield the compound (XXVI) or (XXVIox), respectively and subsequently compound (XXVI) or (XXVIox) and compound (X) are reacted with a peptide bond forming reagent, particularly with HATU, in a reaction step (v) to yield the compound (XXVII) or (XXVIlox), respectively
or
compound (XXVI) or (XXVIox) and compound (X) are reacted with a peptide bond forming reagent, particularly with HATU, in a reaction step (v) to yield the compound (XXVII) or (XXVIIox), respectively.

A tenth aspect of the invention relates to a method for preparation of a compound of formula (I) or (lox), wherein a compound of formula (XXVII) or (XXVIlox) prepared according to the ninth aspect 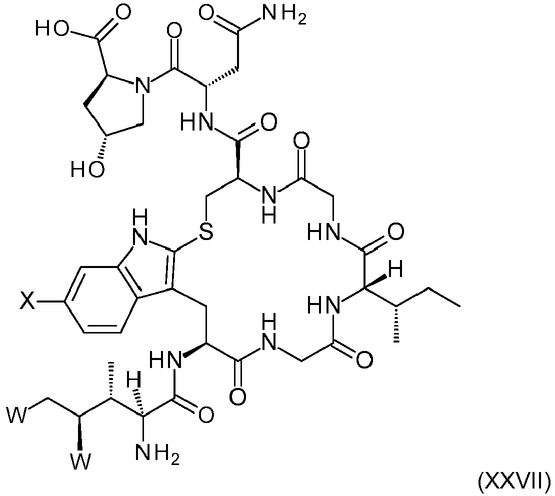 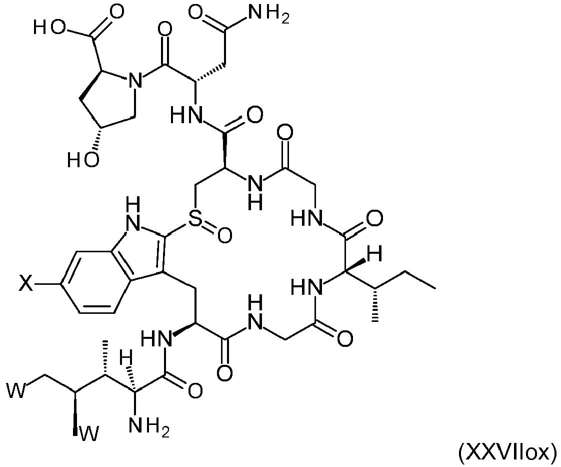 is reacted with a coupling reagent selected from a carbodiimide, an imidazolinium reagent, a phosphonium salt, an organo-phosphorous reagent, an uronium salt, a pyridinium reagent, and a phosphonic acid,
particularly a peptide bond forming reagent, more particularly with T3P, HATU, COMU, HBTU, TBTU, TOMBU, COMBU, or HCTU,
to yield compound (I) or (lox).

A further aspect relates to a compound of the general formula (I) 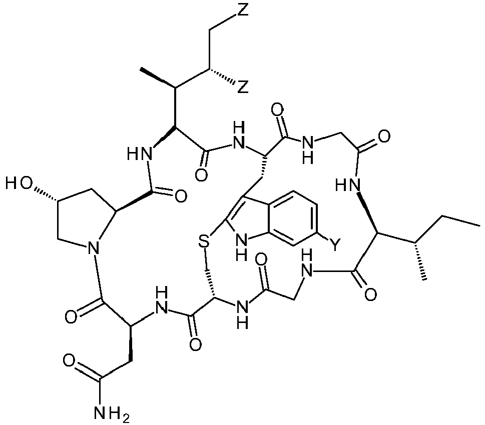 wherein
Y is H and Z is H,
Y is H and Z is OH,
Y is OH and Z is H
Y is F, Cl, I or Br and Z is OH, or
Y is F, Cl, I or Br and Z is H;
particularly Y and Z are independently selected from OH and H.

A further aspect relates to a compound of the general formula (II) 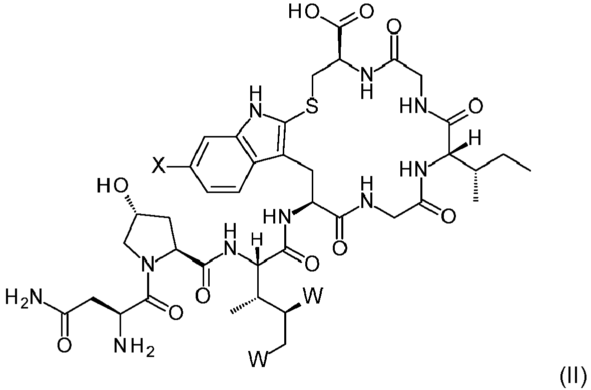 wherein
X is H and W is H,
X is OH and W is OH,
X is H and W is OH,
X is OH and W is H,
X is F, Cl, I or Br, and W is OH, or
X is F, Cl, I or Br, and W is H;
particularly X and W are independently selected from OH and H.

A further aspect relates to a compound of the general formula (IIox) 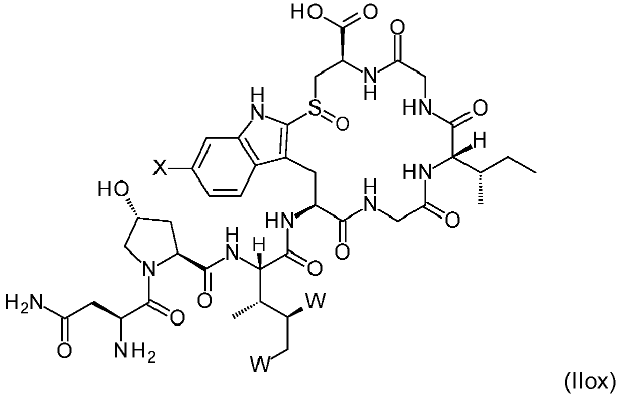 wherein
X is H and W is H,
X is OH and W is OH,
X is H and W is OH,
X is OH and W is H,
X is F, Cl, I or Br, and W is OH, or
X is F, Cl, I or Br, and W is H;
particularly X and W are independently selected from OH and H.

A further aspect relates to a compound of the general formula (IVox) 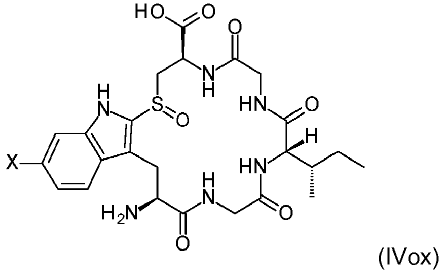 wherein
X is H or OH, or
X is F, Cl, I or Br
particularly X is selected from OH and H.

A further aspect relates to a compound of the general formula (XXVIII) 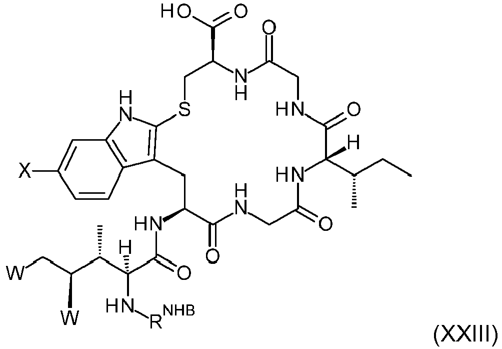 wherein
X is H and W is H,
X is OH and W is OH,
X is H and W is OH,
X is OH and W is H,
X is F, Cl, I or Br, and W is OH, or
X is F, Cl, I or Br, and W is H;
particularly X and W are independently selected from OH and H.

A further aspect relates to a compound of the general formula (XXVIIIox) 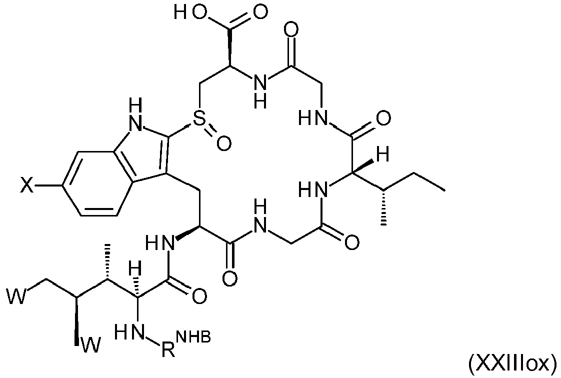 wherein
X is H and W is H,
X is OH and W is OH,
X is H and W is OH,
X is OH and W is H,
X is F, Cl, I or Br, and W is OH, or
X is F, Cl, I or Br, and W is H;
particularly X and W are independently selected from OH and H.

A further aspect relates to a compound of the general formula (XXVI) 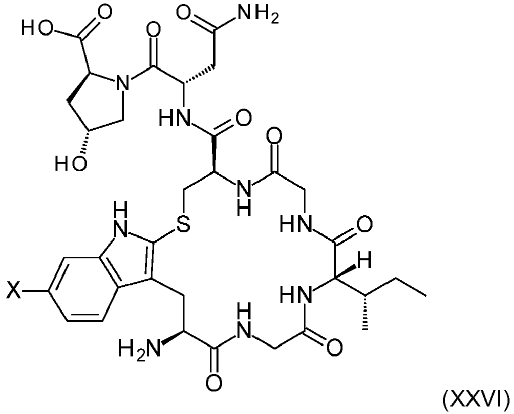 wherein
- X is H and W is H,
- X is H and W is OH,
- X is OH and W is H,
- X is F, Cl, I or Br, and W is OH, or
- X is F, Cl, I or Br, and W is H,
particularly X is H and W is H, or X is H and W is OH, or X is OH and W is H

A further aspect relates to a compound of the general formula (XXVIox) 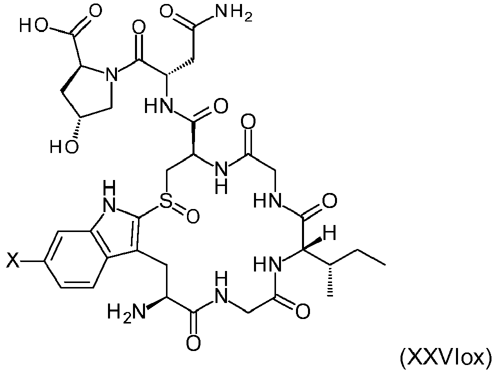 wherein
- X is H and W is H,
- X is OH and W is OH,
- X is H and W is OH,
- X is OH and W is H,
- X is F, Cl, I or Br, and W is OH, or
- X is F, Cl, I or Br, and W is H,
particularly X and W are independently selected from OH and H.

The invention is further illustrated by the following examples and figures, from which further embodiments and advantages can be drawn. These examples are meant to illustrate the invention but not to limit its scope.

### Brief description of the figures

Fig. 1 Synthesis of the Fmoc-protected (2*S*,3*R*,4*R*)-4,5-dihydroxyisoleucine derivatives *via* regioselective Ru-catalyzed allylic alkylation after Kazmaier et al. (Kazmaier et al., Chem. Eur. J. 2004, 20 ,10484-10491) and Sharpless dihydroxylation.
Fig. 2 Chiral GC MS chromatogram of the allylic alkylation product **5,** derivatized for GC-MS by methylation of the C-terminus.
Fig. 3 Synthesis of the (S)-configured allylic carbonate **(S)-4** suitable for the following asymmetric allylic alkylation reaction (Sharpless et al., J. Am. Chem. Soc. 1987, 109, 5765-5780., E. Balmer et al., J. Chem. Soc., Perkin Trans.1 1993, 399-400.).
Fig. 4 Synthesis of Fmoc-protected (2*S*,3*R*,4*R*)-4,5-dihydroxyisoleucine derivatives via asymmetric regioselective Ru-catalyzed allylic alkylation (A. Bayer, U. Kazmaier, Org. Lett. 2010, 12, 21, 4960-4963) and Upjohn-dihydroxylation.
Fig. 5 Synthesis of the Fmoc-protected (2*S*,3*R*,4*R*)-4,5-dihydroxyisoleucine derivative **13** by Tfa-deprotection using NaBH₄ (**shortcut 1**).
Fig. 6 Synthesis of the Fmoc-protected (2*S*,3*R*,4*R*)-4,5-dihydroxyisoleucine derivative **13** by direct Sharpless dihydroxylation of fully protected didehydroisoleucine **5** after allylic alkylation (**shortcut 2**).
Fig. 7 Chiral GC MS chromatogram of the asymmetric allylic alkylation product, derivatized for GC MS by methylation of the C-terminus.
Fig. 8 a) Synthesis of tridentate ligand **(*S*)-28**[1,2] b) Synthesis of (*S*)-6-hydroxytryptphan derivative **33** by dynamic kinetic resolution with a chiral tridentate ligand (Zhou et al., Angew. Chem. Int. Ed Engl. 2014, 53, 7883-7886; Nian et al., Angew. Chem. Int. Ed Engl. 2015, 54, 12918-12922).
Fig. 9 Synthesis of the tryptathionine building blocks.
Fig. 10 Synthesis of H-Gly-Ile-Gly-OH (**45**)
Fig. 11 Synthesis of Fmoc-Asn-Hyp-DHIL(TBS)₂-OH (**48**).
Fig. 12 Assembly of the peptide building blocks affording α-amanitin (**61**) and amaninamide (**62**).

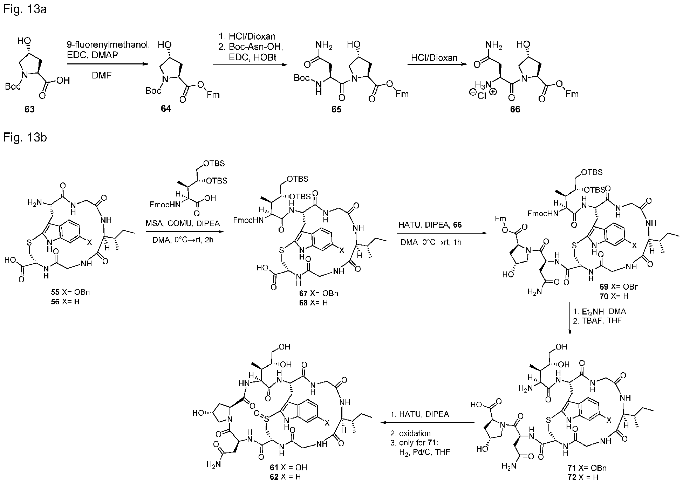

Fig. 13a Dipeptide synthesis of H₂N-Asn-Hyp-OFm.
Fig. 13b Alternative route for α-amanitin (**61**) and amanin amide (**62**).

### Examples

### Example 1: Strategy for the synthesis of (2S,3R,4R)-4,5-dihydroxyisoleucine derivatives

### 9 step synthesis via Ruthenium-catalyzed allylic alkylation

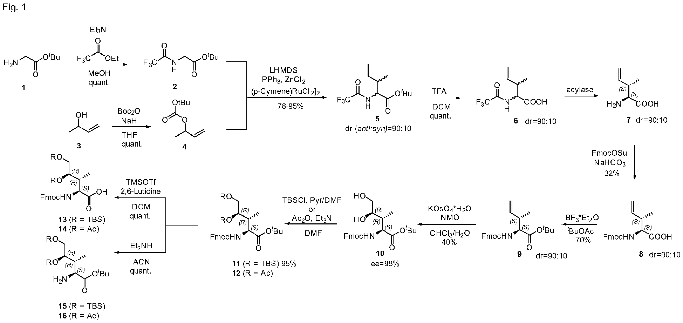

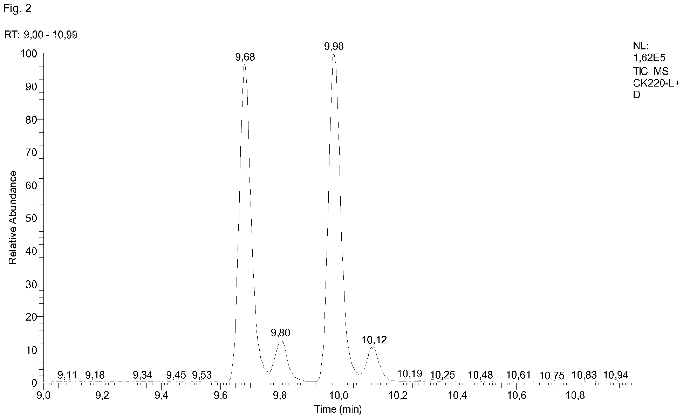

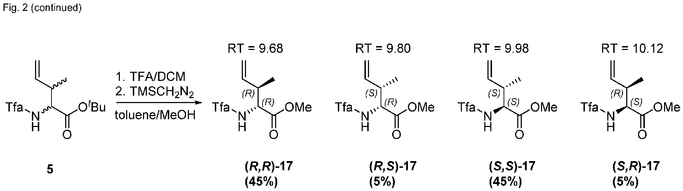

The (2S,3*R*,4R)-4,5-dihydroxyisoleucine derivative **13** was synthesized in 9 steps (Fig. 1) using glycine *tert*-butyl ester as starting material, which was *N*-terminally protected quantitatively in the first step by trifluoroacetylation of the amino group. The fully protected glycine derivative **8** was then submitted to regioselective Ruthenium-catalyzed asymmetric allylic alkylation after Kazmaier *et al.* (Kazmaier et al., Chem. Eur. J. 2004, 20 ,10484-10491) The alkylating reagent was a terminal alkene (**4**) bearing *tert-butyl* carbonate as leaving group, easily accessible by Boc-protection of the racemic allylic alcohol **3** using Boc₂O and NaH. The allylic alkylation reaction led to the mainly *anti*-directed formation of a fully protected didehydroisoleucine derivative **5** with a diastereomeric ratio (dr) of 90:10, calculated by submission of **5** to chiral GC MS after *t*Bu-deprotection of the carboxylic moiety and methylation using TMSCHN₂ (Fig. 2).

Separation of the desired L-configured enantiomers from the remaining D-configured enantiomers was performed by enzymatic kinetic resolution using the enzyme Acylase I from *Aspergillus melleus.* Previous deprotection of the *t*-butyl protecting group was inevitable in order for the enzymatic reaction to take place. The resulting didehydroisoleucine **7** was then Fmoc-protected at the *N*-terminus and refurnished with the *t*Bu-protecting group at the C-terminus prior to the asymmetric dihydroxylation of the terminal double bond. While Fmoc was the protecting group of choice in order to submit the final derivative to SPPS, the *t*Bu protecting group proved to be essential in order to avoid the formation of a highly stable lactone during the dihydroxylation reaction and the subsequent separation of diastereomers using column chromatography on silica gel. The asymmetric dihydroxylation of the fully protected didehydroisoleucine derivative **9** was performed in a biphasic system of water and CHCl₃, which led to the formation mainly of the 2S,3R,4R-configured dihydroxyisoleucine (**10**). Separation of all four diastereomers was easily achieved by a purification step on silica gel at this stage. Finally, the hydroxy groups of the side chain were protected prior to deprotection of the C-terminus. The protecting group of choice was the TBS-protecting group, allowing the mild *t*Bu cleavage in quantitative yield using an excess of TMSOTf in the final step affording the final Fmoc-protected (2*S*,3*R*,4*R*)-4,5-dihydroxyisoleucine derivative. The overall yield after 9 steps was calculated to be ∼7%.

### Asymmetric synthesis strategies via regioselective Ruthenium-catalyzed allylic alkylation and possible shortcuts

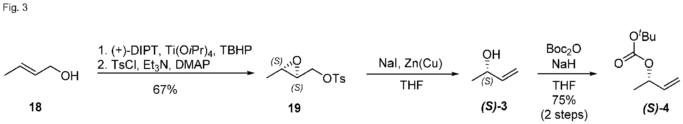

In order to achieve a higher overall yield of the synthesis route described in the previous section a chiral alkylating reagent during the allylic alkylation reaction was used (Fig. 3). Employing a chiral transfer during the allylic alkylation, the desired (2*S*,3*S*)-configured didehydroisoleucine is preferably formed. The chiral allylic alcohol but-3-en-2-ol (*S*)-**3** was synthesized according to published literature procedures *via* Sharpless epoxidation of (*E*)-crotyl alcohol **18** followed by *in situ* tosylation of the hydroxy group affording epoxide **19.** The chiral carbonate (*S*)-**4** was then formed by reductive elimination using Nal and a Zinc-Copper-couple after Balmer *et al.* followed by Boc-protection of the hydroxyl group.

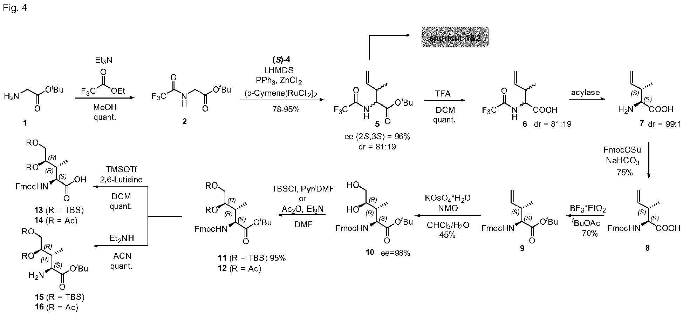

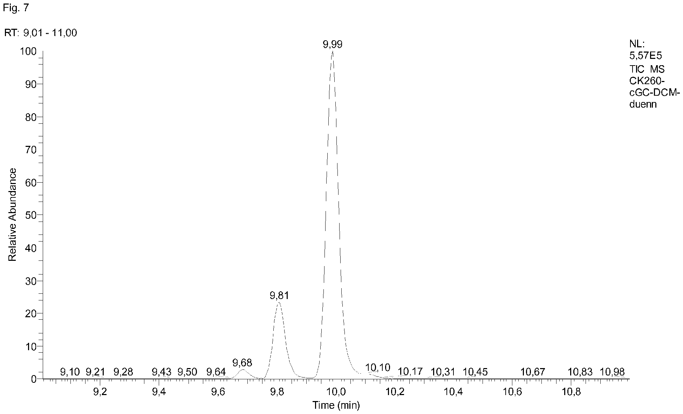

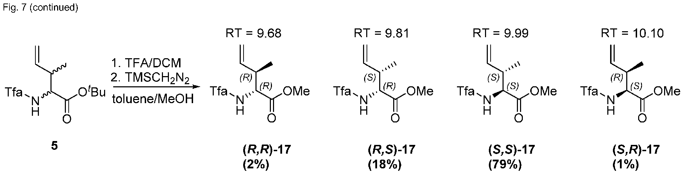

The overall synthesis strategy for the enantiomerically pure Fmoc-protected (2*S*,3*R*,4*R*)-4,5-dihydroxyisoleucine derivative (Fig. 4) followed the same route as described in the previous section. The only difference was the use of the chiral allylic carbonate (*S*)-**4** during the Ruthenium-catalyzed allylic alkylation which resulted in the formation of the fully protected (2*S*,3*S*)-didehydroisoleucine **5** with an enantiomeric excess of 98% due to a chiral transfer of the alkylating reagent (Fig. 7).

The diastereomeric ratio (dr) was calculated to be 86:14 towards the (2*R*,3*S*)-diastereomer and 99:1 towards the (2*S*,3*R*)-configured diastereomer. The former was separated conveniently by the following acylase reaction which led to the formation of a enantiomerically pure didehydroisoleucine **7** with a dr of 99:1. Because of the enantiomeric purity the asymmetric dihydroxylation also resulted in a higher yield as there were only two diastereomers that needed separation afterwards instead of four. The overall yield starting from glycine *tert*-butyl ester (**1**) after 9 steps was calculated to be 17-21%.

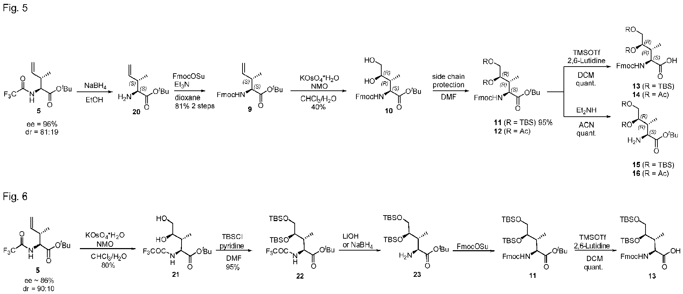

The high enantiomeric excess of **5** also made it possible to take two shortcuts resulting in a higher yield (Fig. 5 and 6).

One shortcut was the direct Tfa-deprotection of didehydroisoleucine **5** followed by Fmoc-protection. This way, the tBu-cleavage and reattachment was omitted resulting in the formation of the dihydroxylation substrate within two steps instead of four. The enantiomeric excess of **13** following **shortcut 1** was calculated by chiral HPLC and resulted to be 95%. The overall yield was calculated to be 24-29%.

A second shortcut was the direct Sharpless dihydroxylation of the allylic alkylation product **5** followed by the protection of the side chain with the TBS protecting groups. The diastereomers were separated by column chromatography on silica gel after cleavage of the Tfa protecting group using LiOH and Fmoc-protection of the C-terminus (**11**). The enantiomeric excess of **13** following **shortcut 2** was calculated by chiral HPLC and resulted to be 70%. Both shortcuts enable the synthesis of Fmoc-4,5-dihydroxyisoleucine in 7 steps instead of 9 and provided higher overall yields.

### Example 2: Strategy for the synthesis of (S)-6-hydroxytryptophan derivatives

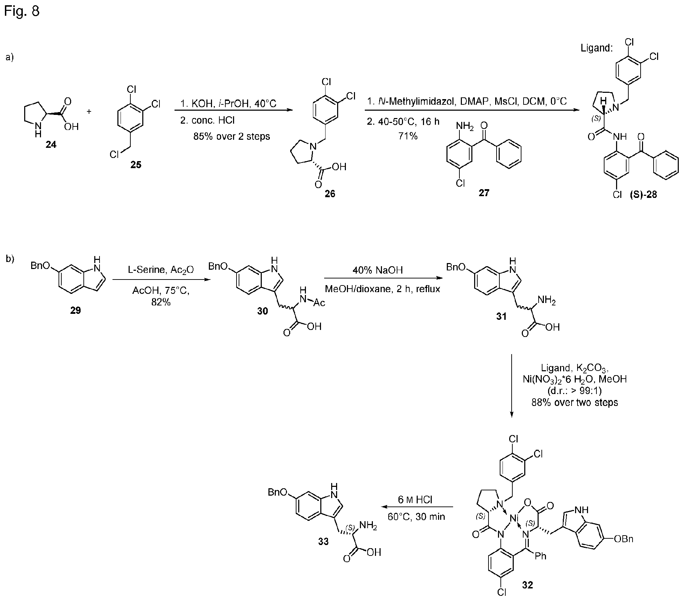

The (*S*)-6-hydroxytryptophan derivative **33** was synthesized in four steps, starting with an alkylation of the commercially available 6-benzoxyindol (**29**) using L-serine and acetic anhydride in acetic acid, which leads to the racemic *N*-acetyl-6-benzoxytryptophan (**30**) in moderate yields (Blaser, et al., Tetrahedron Lett.2008, 2795-2798). After deacetylation with 40% NaOH in MeOH/dioxane the racemic 6-benzoxytryptophan (**31**) was obtained, which was submitted to a dynamic kinetic resolution following a protocol from Zhou *et al.* and Nian *et al.* (Zhou et al., Angew. Chem. Int. Ed Engl. 2014, 53, 7883-7886; Nian et al., Angew. Chem. Int. Ed Engl. 2015, 54, 12918-12922). Therefore, the tridentate ligand **(*S*)-28** was synthesized in two steps according to the literature (Zhou et al., Angew. Chem. Int. Ed Engl. 2014, 53, 7883-7886; Nian et al., Angew. Chem. Int. Ed Engl. 2015, 54, 12918-12922). The racemic 6-benzoxytryptophan (**31**) was treated with the ligand **(*S*)-28,** K₂CO₃ and Ni(NO₃)₂^{∗}6H₂O as a nickel source, which gave the Ni(II)-complex **32.** The diastereomeric ratio of >99% was determined by chiral HPLC with a CHIRALPAK AD-H column (hexane/isopropanol = 55/45, λ = 280 nm, 0.8 mL/min). After separation of the two diastereoisomers by silica gel column chromatography, the absolute stereochemistry was undoubtedly determined by single-crystal X-ray diffraction. Disassembly of the complex **32** under acidic conditions resulted in the target enantiomerically pure (*S*)-6-hydroxytryptophan derivative **33** (Fig. 8).

### 3-(6-Benzoxy-1H-indol-3-yl)-2-acetylaminopropionic acid (30):

**¹H NMR** (400 MHz, DMSO-*d*₆): δ (ppm) = 1.79 (s, 3 H) 2.92 (dd, *J*=14.68, 8.66 Hz, 1 H) 3.09 (dd, *J*=14.68, 4.89 Hz, 1 H) 4.42 (td, *J*=8.22, 5.14 Hz, 1 H) 5.09 (s, 2 H) 6.72 (d, *J*=6.27 Hz, 1 H) 6.89 (d, *J*=2.01 Hz, 1 H) 6.98 (d, *J*=2.01 Hz, 1 H) 7.27 - 7.33 (m, 1 H) 7.35 - 7.41 (m, 3 H) 7.42 - 7.48 (m, 2 H) 8.11 (d, *J*=7.78 Hz, 1 H) 10.64 (s, 1 H).
**¹³C NMR** (100 MHz, DMSO-*d*₆): δ (ppm) = 173.59, 169.20, 154.45, 137.68, 136.67, 128.39, 127.62, 127.48, 122.27, 121.85, 118.78, 110.00, 109.21, 95.95, 69.49, 52.98, 27.22, 22.42.
**HRMS** (ESI): *m*/*z* calc. für C₂₀H₂₀N₂O₄ (M+H)⁺353.1496, found 353.1487.

### (S)-3-amino-3-(6-(benzoxy)-1H-indol-3-yl)propanoic acid - Schiff Base Complex (32):

**1H NMR** (500 MHz, C*D*Cl₃): δ (ppm) = 1.27 - 1.39 (m, 1 H) 1.61 - 1.74 (m, 2 H) 1.76 - 1.88 (m, 1 H) 2.00 - 2.13 (m, 1 H) 2.71 - 3.05 (m, 4 H) 3.23 (dd, *J*=14.65, 3.97 Hz, 1 H) 4.01 (d, *J*=12.51 Hz, 1 H) 4.20 (t, *J*=4.65 Hz, 1 H) 5.00 (s, 2 H) 6.55 (d, *J*=2.44 Hz, 1 H) 6.62 - 6.74 (m, 2 H) 6.79 (s, 1 H) 6.88 (d, J=1.83 Hz, 1 H) 7.03 (dd, *J*=9.31, 2.44 Hz, 1 H) 7.12 (d, *J*=8.70 Hz, 1 H) 7.16 - 7.33 (m, 7 H) 7.34 - 7.57 (m, 5 H) 8.09 (d, *J*=9.31 Hz, 3 H) 8.38 (br. s., 1 H) 8.80 (d, *J*=1.68 Hz, 1 H).
**13C NMR** (100 MHz, C*D*Cl3): δ (ppm) =179.92, 156.24, 141.28, 137.77, 137.47, 135.19, 134.01, 133.58, 133.51, 132.64, 131.17, 130.41, 130.04, 129.64, 129.32, 128.84, 128.13, 127.73, 125.83, 124.16, 123.53, 123.36, 120.68, 110.92, 110.18, 96.86, 71.78, 70.98, 63.47, 58.66, 30.95, 22.83.
**HRMS** (ESI): *m*/*z* calc.C₄₃H₃₅Cl₃N₄NiO₄ (M+H)⁺835.1150, found 835.1159.

The *dr* was determined by chiral HPLC with a CHIRALPAK AD-H column (hexane/isopropanol = 55/45, λ = 280 nm, 0.8 mL/min). t_{R} (major diastereomer) = 13.29 min, >99:1 *dr*, t_{R}(minor diastereomer) = 17.28 min.

### (S)-6-benzoxytryptophan (33):

**HRMS** (ESI): *m*/*z* calc. C₁₈H₁₈N₂O₃ (M+H)⁺ 311.1390, found 311.1391.

### Example 3: Synthesis of α-amanitin and amaninamide

### Synthesis of the peptide building blocks

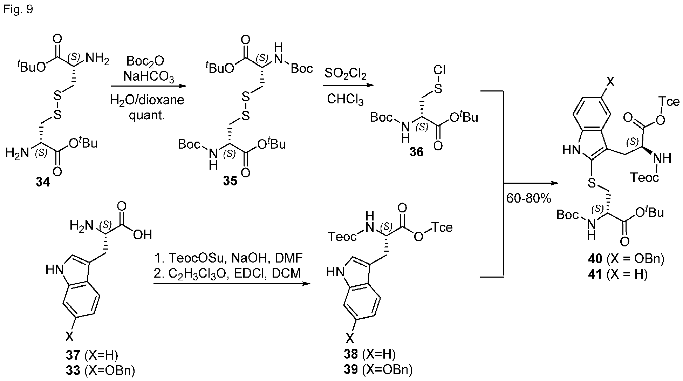

The thioether building units **40** and **41** were readily established by treatment of a fully protected L-cystine derivative (**35**) with sulfuryl chloride. Cleavage of the disulfide afforded the highly reactive sulfenyl chloride monomer **36,** which in the following step is susceptible for an electrophilic aromatic substitution (S_{E}Ar) either solely *N*-terminally protected or fully protected 6-hydroxytryptophan and tryptophan derivative **38** and **39.** The use of the TCE-protecting group at the C-terminus helped to suppress the formation of undesired side-products with residual sulfuryl chloride from the sulfenyl chloride formation, but was not imperative for the reaction to take place (Fig. 9).

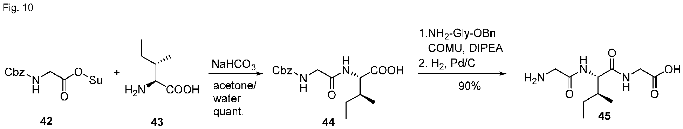

The tripeptide building block H-Gly-Ile-Gly-OH (**45**) was synthesized in solution phase by first synthesizing a *N*-terminally Cbz- and C-terminally Bn-protected tripeptide, followed by simultaneous Cbz- and Bn-deprotection by hydrogenolysis using H₂ and Pd/C as catalyst. (Fig. 10).

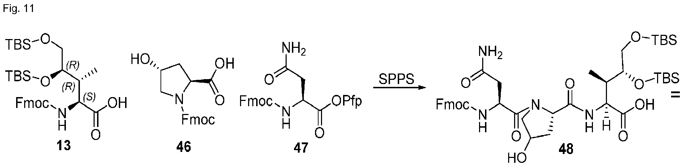

The Fmoc-Asn-Hyp-DHIL(TBS)₂-OH tripeptide **48** was synthesized on solid phase using the CTC-resin. The use of Fmoc-Asn-OPfp (**47**) during the coupling of asparagine with no protecting group at the side chain suppressed the formation of the dehydration product (a tripeptide containing Fmoc-β-cyanoalanine) to the extent of only 10% (Fig. 11).

A C-terminally 9-Fluorenylmethyl ester-protected dipeptide building block **66** was synthesized by esterification of *trans*-*N*-(Boc)-4-hydroxy-L-proline (**63**) with 9-fluorenylmethanol affording fully protected 4-hydroxy-L-proline **64.** Boc-deprotection under acidic conditions, followed by coupling with Boc-Asn-OH using EDC and HOBt and repeated Boc-deprotection under acidic conditions led to the formation of dipeptide building block **66** with no formation of the dehydration side product present.

*Assembly of the peptide building blocks towards (S)-Deoxy-(O)-Benzyl α-amanitin and (S)-Deoxy amaninamide.*

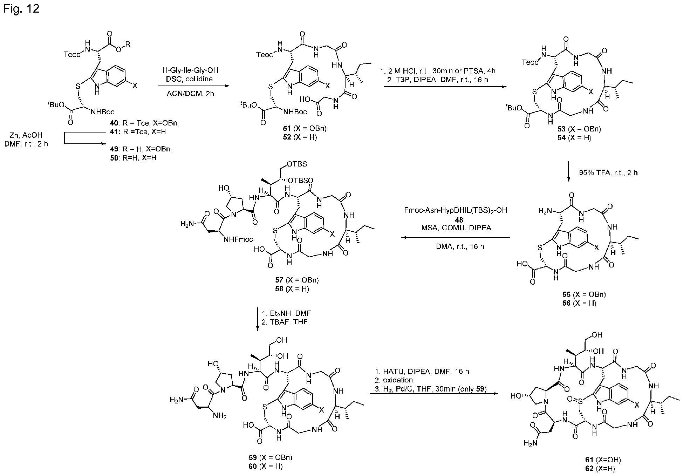

First, monocyclic thioethers **55** and **56** were synthesized in order to obtain the bicyclic structures of (*S*)-Deoxy (O)-benzyl-α-amanitin and (S)-Deoxy amaninamide (Fig. 12). In order to do so, the thioether building blocks **51** and **52** were deprotected using Zn and AcOH in DMF, transformed into an active ester using *N,N'*-disuccinimidyl carbonate, followed by coupling of the *C*- and *N*-terminally deprotected tripeptide building block **45.** After deprotection with p-toluoenesulfonic acid or 2 M HCl, the peptides were cyclized with T3P and DIPEA in DMF/DCM within 3 h. Afterwards, monocyclic pentapeptides **53** and **54** were deprotected using 80% TFA in DCM and coupled to tripeptide **48** using a protocol activating not only the carboxylic function of the tripeptide by an active ester forming agent, but also the amino group of monocyclic pentapeptides by a silylating agent. Octapeptides **57** and **58** were then *N*-terminally Fmoc-deprotected and cyclized using HATU in DMF. The TBS protecting groups were cleaved from the DHIL residue by treatment of the peptides with 1 M TBAF for 2h.

A second pathway leading to the formation of (*S*)-Deoxy-(*O*)-benzyl-α-amanitin and (*S*)-Deoxy amaninamide was the direct coupling of DHIL-derivative **13** to the fully deprotected monocyclic pentapeptides **55** and **56** by using a silylating agent for the *N*-terminus of the pentapeptides and an active ester forming agent for the carboxylic function of the DHIIe derivative, analogous to the method described above. Monocyclic hexapeptides **67** and **68** could then be coupled to the C-terminally Fm-protected dipeptide building block **66** leading to the formation monocyclic octapeptides **69** and **70.** The final cyclization was then performed after simultaneous Fmoc and Fm-cleavage and subsequent TBS-deprotection from the DHIL residue.

### Sulfide oxidation affording α-amanitin and amaninamide

The asymmetric oxidation of the tryptathionine moiety leading to (*O*)-Benzyl-α-amanitin and amaninamide (**62**) was achieved by using a manganese complex as a catalyst with a porphyrine inspired chiral ligand, following a protocol reported by Gao *et al (*D. Wen, L. Jun, S. Gao, Org. Lett. 2013, 15, 22, 5658-61). Hydrogenolysis with H₂ and Pd/C of (*O*)-Benzyl-α-amanitin in THF afforded the natural product α-amanitin (**61**) after 30 min of reaction time.

### Materials and methods

### tert-Butyl (2,2,2-trifluoroacetyl)glycinate (2):

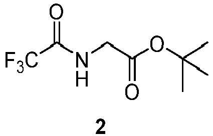

To a solution of glycine *tert-butyl* ester hydrochloride (10.0 g, 137 mmol, 1.00 eq) in MeOH (150 ml) triethylamine (17.4 ml, 125 mmol, 2.10 eq) was added dropwise. After stirring for 5 min ethyl trifluoroacetate (16.4 ml, 137 mmol, 2.3 eq) was added and the mixture was stirred for 16 h at room temperature during which time a clear solution formed. Then, the reaction mixture was concentrated under reduced pressure and the resulting residue acidified with 2 N HCI before being extracted with EtOAc (3x100 ml). The organic layers were combined, then washed with sat. NaHCO₃ (2x100 ml), distilled H₂O (2x100 ml) and brine (2x100 ml) and dried over MgSO₄. The solvent was removed *in vacuo* to give the product (**2**) as a yellow oil (13.5 g, quant.).
**¹H NMR** (CDCl₃-d¹, 400 MHz): δ = 1.50 (s, 9 H), 4.02 (d, *J*=5.02 Hz, 2 H), 6.89 ppm (br s, 1 H).
**¹³C NMR** (CDCl₃-d¹, 100 MHz): δ = 27.91, 41.94, 83.52, 115.60 (q, *J*=287.28 Hz), 157.06
(q, *J*=39.60 Hz), 167.28 ppm.
**HRMS (ESI):** m/z calculated: C₈H₁₂F₃NO₃ (M-H)⁻ 226.0686, found 226.0693.

### But-3-en-2-yl tert-butyl carbonate (4):

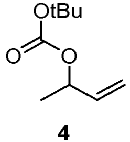

But-3-en-2-ol (1.50 g, 20.8mmol, 1.00 eq) was added slowly to a solution of NaH (1.50 g, 62.4 mmol, 3.00 eq) in dry THF (40 ml) at 0°C. Then Boc₂O (5.9 g, 27 mmol, 1.3eq) was added in portions over 10 min under vigorous stirring at this temperature. The reaction mixture was allowed to warm to room temperature overnight and then diluted with Et₂O after 16h of vigorous stirring. Excess sodium hydride was quenched by the slow addition of water. The resulting mixture was then extracted with diethyl ether (3x50 ml). The combined organic extracts were washed with brine (1x50 ml), dried over MgSO₄ and concentrated under reduced pressure. After purification by column chromatography on silica gel (hexane/EtOAc, 10:1) the product (**4**) was obtained as a colourless liquid (3.6 g, 20.3 mmol, quant.).
**¹H NMR** (CDCl₃-d¹, 400 MHz): δ = 1.36 (d, *J* = 6.53 Hz, 3 H), 1.49 (s, 9 H), 5.13 - 5.17 (m, 2 H), 5.25 - 5.31 (m, 1 H), 5.83 - 5.91 ppm (m, 1 H).
**¹³C NMR** (CDCl₃-d¹, 100 MHz): δ = 19.72, 27.48, 73.72, 81.62, 115.78, 137.78, 137.17, 152.53 ppm.
**HRMS (ESI):** m/z calculated: C₉H₁₆O₃ (M+H)⁺ 173.1172, found 173.1171.

### tert-Butyl 3-methyl-2-(2,2,2-trifluoroacetamido)pent-4-enoate (Trifluoroacetyl 4,5-didehydroisoleucine tert-butyl ester) (5):

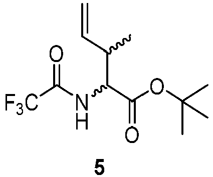

LHMDS (1 M in THF, 11 mmol, 2.5eq) was added slowly to a solution of trifluoroacetyl glycine *tert*-butylester (**2,** 1.0 g, 4.4 mmol, 1.5 eq) in dry THF (12 ml) at -78°C. After stirring for 10 min a solution of dried ZnCl₂ (720 g, 5.30 mmol, 1.20eq) in dry THF (6 ml) was added at this temperature and stirring was continured for another 30 min at -78°C. Meanwhile a solution was prepared from [(*p*-cymene)RuCl₂]₂ (37 mg, 0.06mmol, 0.02eq) and triphenylphosphine (32 mg, 0.12mmol, 0.04eq) in dry THF (6ml) and stirred for 10 min at room temperature. Then, the allylic carbonate (**4**) (517 mg, 3.00 mmol, 1.00eq) was added and the resulting solution was added to the chelated enolate at -78°C. The reaction mixture was allowed to warm to room temperature overnight. After diluting with EtO₂ (100 ml) the reaction mixture was hydrolyzed by addition of 1 M KHSO₄ until the precipitate was fully dissolved in the organic layer. Then, the layers were separated, the aqueous layer was extracted with diethyl ether (3x50 ml) and the combined organic layers were washed with brine (100ml) and dried over MgSO₄. The solvent was removed *in vacuo* and the crude product was purified by column chromatography on silica gel (hexane/EtOAc, 10:1), which afforded the product (**5**) as a colourless oil (740 mg, 88%).
**¹H NMR** (CDCl₃-d¹, 400 MHz): δ = 1.10 (d, *J*=7.03 Hz, 3 H), 1.49 (s, 9 H), 2.82 - 2.89 (m, 1 H), 4.48 - 4.54 (m, 1 H), 5.07 - 5.20 (m,2 H), 5.70 (s, 1 H), 6.70 ppm (d, *J*=7.53 Hz, 1 H).
**¹³C NMR** (CDCl₃-d¹, 100 MHz): δ = 15.75, 27.97, 40.21, 56.71, 83.35, 117.15 (q, *J*=273.2 Hz), 117.46, 136.78, 157.03 (q, *J*=39.60 Hz), 168.89 ppm.

### 3-methyl-2-(2,2,2-trifluoroacetamido)pent-4-enoic acid (Trifluoroacetyl 4,5-didehydroisoleucine) (6):

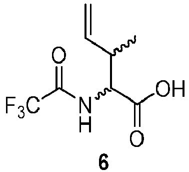

Fully protected didehydroisoleucine (**5**) (1.0 g, 3.6 mmol, 1.0eq) wasdissolved in a solution of 95% TFA in DCM. After stirring for 2 h at roomtemperature the solvent was evaporated *in vacuo* to afford the trifluoroacetylated didehydroisoleucine (**6**) (800 mg, quant.) as a white solid.
**¹H-NMR** (400 MHz, CDCl₃): δ (ppm) = 6.63 (br, 1 H), 5.66 - 5.77 (m, 1 H) 5.19 - 5.27 (m, 2 H), 4.66 (q, *J*=4.2 Hz, 1 H), 2.90 - 2.99 (m, 1 H), 1.15 (d, *J*=7.04 Hz, 3 H)
**¹³C-NMR** (100 MHz, CDCl₃): δ (ppm) = 175.37, 157.67, 136.37, 118.37, 117.00, 56.43, 39.56, 16.32
**HRMS** (ESI): *m*/*z* calc for C₈H₉F₃NO₃ (M-H)⁻ 224.0529, found 224.0536.

### (2S,3S)-2-amino-3-methylpent-4-enoic acid (4,5-Didehydroisoleucine) (7)

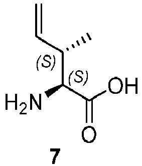

To a solution of the racemic trifluoroacetylated didehydroisoleucine **6** (700 mg, 3.10 mmol, 1.00 eq) in Sørensen phosphate buffer (15 ml, pH 7.5) 4 M KOH (777 µl, 3.10 mmol, 1.00 eq) and acylase I from *Aspergillus melleus* (300 mg) was added. After 6 h at 36°C the reaction mixture was filtered through a Amicon Ultra centrifugal filter unit (cut off 10 kDa). The resulting the amino acid buffer mixture was then submitted to the subsequent Fmoc protection without any further purification.
**HRMS** (ESI): *m*/*z* calc for C₆H₁₁NO₂ (M+H)⁺ 130.0863, found 130.0858.

### Fmoc-4,5-didehydroisoleucine (8)

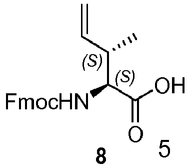

To a stirring solution of 4,5-didehydroisoleucine **8** in Sørensen phosphate buffer (15 ml) was added a solution of Fmoc-OSu (823 mg, 2.44 mmol, 1.05 eq) in acetone (10ml). After stirring for 16 h the reaction mixture was diluted with 50 ml water, acidified to pH 2 with 1 N HCI and extracted with ethyl acetate (3x30 ml). The combined organic layers were washed with brine, dried over MgSO₄ and concentrated under reduced pressure. The crude product was purified by column chromatography on silica gel (1% MeOH/DCM) to obtain the Fmoc didehydroisoleucine **8** as a white solid (810 mg, 75%, 2 steps).
**¹H-NMR** (400 MHz, DMSO-d₆): δ (ppm) = 8.49 (br, 1 H), 7.68 (d, *J*=7.5 Hz, 2 H), 7.44 - 7.54 (m, 2 H), 7.32 (t, *J*=7.02 Hz, 2 H), 7.23 (t, *J*=7.02 Hz, 2 H), 5.45 - 5.73 (m, 1 H), 5.18 - 5.32 (m, 1 H), 5.01 - 5.17 (m, 1 H), 4.30 - 4.37 (m, 2 H), 4.12- 4.19 (m, 1 H), 2.39- 2.54 (m, 1 H), 2.44 - 2.52 (m, 1 H), 1.60 (d, *J*=6.0 Hz, 2 H), 1.05 (d, *J*=7.0 Hz, 1 H)
**¹³C-NMR** (100 MHz, DMSO-d₆): δ (ppm) = 177.01, 156.04, 143.58, 141.45, 137.20, 130.67, 127.93, 127.02, 125.20, 124.13, 120.18, 117.60, 67.46, 58.04, 53.33, 47.25, 39.65, 35.09
**HRMS** (ESI): *m*/*z* calc for C₂₁H₂₁NO₄ (M+H)⁺ 374.1363, found 374.1360.

### Fmoc-4,5-didehydroisoleucine tert-butyl ester (9)

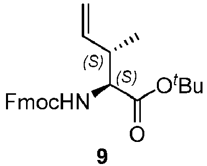

BF₃*OEt₂ (1.69 ml, 13.7 mmol, 6.0 eq) was added to a stirring solution of Fmoc-protected 4,5-didehydroisoleucine (**8**) (800 mg, 2.28 mmol, 1.00 eq) in 10 ml *^{t}*BuOAc. The reaction mixture was stirred at rt for 5 min, then cooled to 0°C and neutralized with saturated NaHCO₃. The reaction mixture was then extracted with EtOAc (3^{∗}50 ml), washed with 1 M HCI (3x20ml) and brine (2x20ml) and dried over MgSO₄. After evaporation of the solvent under reduced pressure the crude product was purified by column chromatography on silica gel (hexane/EtOAc, 10:1) to obtain the fully protected 4,5-didehydroisoleucine (**9**) as a colourless oil (650 mg, 70%).
**¹H NMR** (400 MHz, CDCl₃): δ (ppm) = 1.07 - 1.14 (m, 3 H), 1.49 (s, 9 H), 2.74 - 2.85 (m, 1 H), 4.21 - 4.34 (m, 2 H), 4.39 (m, J=6.90, 6.90 Hz, 2 H), 5.08 - 5.18 (m, 2 H), 5.24 (s, 1 H), 5.68 - 5.79 (m 1 H), 7.33 (t, J=7.53 Hz, 2 H), 7.41 (t, J=7.53 Hz, 2 H), 7.61 (d, J=7.53 Hz, 2 H), 7.78 (d, J=7.53 Hz, 2 H)
**¹³C NMR** (100 MHz, CDCl₃): δ (ppm) = 15.66, 27.75, 40.07, 46.86, 58.02, 66.69, 81.93, 116.32, 119.64, 124.81, 126.72, 127.36, 137.45, 140.97, 143.56, 155.92, 170.27
**HRMS** (ESI): *m*/*z* calc for C₂₅H₂₉NO₄ (M+H)⁺ 430.1989, found 430.1982.

### Fmoc-4,5-dihydroxyisoleucine tert-butyl ester (10)

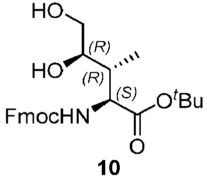

*N*-methylmorpholine-*N*-oxide (287 mg, 2.45 mmol, 1.30 eq) was added to a stirring solution of **9** and potassium osmate dihydrate (45.2 mg, 122 µmol, 0.05eq) in 15ml of a 4:1 mixture of CHCl₃ and water and stirred for 20 min at rt. Then, Fmoc-protected 4,5-dihydroxyisoleucine *tert*-butyl ester (**10**) (1.00 g, 2.45 mmol, 1.00 eq) was added to the biphasic mixture. The resulting mixture was stirred at rt for 16 h and diluted with 100 ml DCM. Afterwards, saturated sodium metabisulfite solution (20ml) was added and extracted with DCM (3x10 ml) after being stirred for 30 min. the combined organic phases were dried over NaSO₄ and evaporated under reduced pressure. The crude product was purified by column chromatography on silica gel (0.6% MeOH/DCM to 1.0% MeOH/DCM gradient) to obtain the 4,5-dihydroxyisoleucine derivative **10** as white crystals (439 mg, 40%).
**¹H NMR** (CDCl₃-d¹, 400 MHz): δ = 1.00 (d, *J*=7.03 Hz, 3 H), 1.50 (s, 9 H), 1.94 - 2.04 (m, 3 H), 3.57 (dd, *J*=10.79, 3.01 Hz, 1 H), 3.72 (t, *J*=9.50 Hz, 1 H), 3.76 - 3.82 (m, 1 H), 4.18 - 4.27 (m, 2 H), 4.37 - 4.47 (m, 2 H), 5.90 (d, *J*=8.28 Hz, 1 H), 7.33 (t, *J*=7.03 Hz, 2 H), 7.41 (t, *J*=7.28 Hz, 2 H), 7.61 (d, *J*=7.28 Hz, 2 H), 7.77 ppm (d, *J*=7.53 Hz, 2 H)
**¹³C NMR** (CDCl₃-d¹, 100 MHz): δ = 10.41, 27.99, 38.32, 47.14, 57.85, 64.71, 67.18, 71.70, 82.74, 119.96, 125.02, 127.06, 127.71, 141.27, 143.63, 156.82 ppm
**HRMS** (ESI): *m*/*z* calc for C₂₅H₃₁NO₆ (M+H)⁺ 442.2224, found 442.2222.

### tert-butyl(2S, 3R, 4R)-2-((((9H-fluoren-9-yl)methoxy)carbonyl)amino)-4,5-bis((tert-butyldimethylsilyl)oxy)-3-methylpentanoate (11)

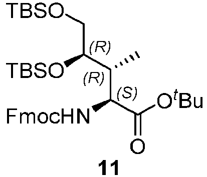

Under a nitrogen atmosphere *tert*-butyldimethylsilyl chloride (546 mg, 3.62 mmol, 8.0 eq) was added to a stirring solution of Fmoc-4,5-dihydroxyisoleucine *tert-butyl* ester (**10,** 200 mg, 452 µmol, 1.00 eq) in 4 ml of a 1:1 mixture of dry DMF and pyridine. Then, DMAP (8.3 mg, 68 µmol, 0.15 eq) was added and the resulting mixture was stirred for 24 h at rt. Afterwards, the reaction mixture was diluted with 50 ml EtOAc and washed with 1 M HCI (3x20ml) and brine (2x20ml), dried over MgSO₄ and evaporated under reduced pressure. The crude product was purified by column chromatography on silica gel (hexane/EtOAc, 19:1) to obtain the fully protected 4,5-dihydroxyisoleucine (**9**) as a colourless oil (271 mg, 90%).
**¹H NMR** (CDCl₃-d¹, 500 MHz): δ = 0.06 - 0.09 (m, 6 H), 0.11 (s, 3 H), 0.17 (s, 3 H), 0.90 - 0.95 (m, 18 H), 1.00 (d, *J*=7.02 Hz, 3 H), 1.49 (s, 9 H), 2.35 - 2.44 (m, 1 H), 3.44 (dd, *J*=9.69, 8.32 Hz, 1 H), 3.52 - 3.57 (m, 1 H), 3.82 - 3.86 (m, 1 H), 4.19 - 4.26 (m, 2 H), 4.39 (d, *J*=7.02 Hz, 2 H), 5.99 (d, *J*=8.24 Hz, 1 H), 7.30 (t, *J*=7.78 Hz, 2 H), 7.40 (t, *J*=7.40 Hz, 2 H), 7.63 (dd, *J*=9.77, 7.78 Hz, 2 H), 7.76 (d, J=7.63 Hz, 2 H)
**¹³C NMR** (CDCl₃-d¹, 126 MHz): -5.50, -5.38, -4.64, -4.09, 10.40, 18.05, 18.26, 25.87, 25.90, 28.04, 35.51, 47.27, 59.12, 64.18, 66.80, 73.74, 81.38, 119.88, 125.20, 126.98, 127.55, 141.28, 144.02, 144.17, 156.46, 171.22
**HRMS (ESI):** m/z calculated: C₃₇H₅₉NO₆Si₂ (M+H)⁺ 670.3953, found 670.3945.

### (2S,3R,4R)-2-((((9H-fluoren-9-yl)methoxy)carbonyl)amino)-4,5-bis((tert-butyldimethylsilyl)oxy)-3-methylpentanoic acid (13)

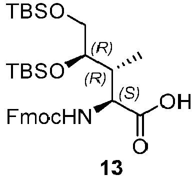

A solution of fully protected 4,5-dihydroxyisoleucine **11** (100 mg, 149 µmol, 1.0 eq) was dissolved in 2 ml dry DCM, treated with 2,6 lutidine (173 µl, 1.49 mmol, 10.0 eq) and cooled to 0°C. Then, TMSOTf (135 µl, 746 µmol, 5.0 eq) was added and the reaction mixture was allowed to warm to room temperature overnight. The solution was diluted with Et₂O (10ml) followed by the addition of Sorensen phosphate buffer (pH=7; 5ml). Afterwards, the pH of the mixture was adjusted to 2 by the dropwise addition of NaHSO₄-solution (10%). The phases were separated and the aqueous phase was extracted with Et₂O (3x10ml). The combined organic phases were washed with brine, dried over NaSO₄ and evaporated under reduced pressure. The crude product was purified by column chromatography on silica gel (1% MeOH/DCM) to furnish the final product (**13**) as a colourless oil (90.5 mg, quant.).
**¹H NMR** (CDCl₃-d¹, 500 MHz): δ = 0.02 - 0.14 (m, 12 H), 1.04 (d, *J*=7.02 Hz, 3 H), 2.36 - 2.47 (m, 1 H), 3.55 (t, *J*=8.24 Hz, 1 H), 3.58 - 3.64 (m, 1 H), 3.79 - 3.88 (m, 1 H), 4.24 (t, *J*=6.79 Hz, 1 H), 4.37 - 4.49 (m, 3 H), 6.10 (d, *J*=7.32 Hz, 1 H), 7.31 (t, *J*=7.48 Hz, 2 H), 7.40 (t, *J*=7.32 Hz, 2 H), 7.61 (t, *J*=7.63 Hz, 2 H), 7.76 (d, *J*=7.48 Hz, 2 H)
**¹³C NMR** (CDCl₃-d¹, 126 MHz): -5.48, -5.41, -4.74, -4.07, 11.07, 18.00, 18.25, 25.79, 25.87, 37.01, 47.23, 57.77, 64.68, 67.00, 73.97, 119.95, 125.05, 125.12, 127.03, 127.64, 141.33, 143.86, 156.43
**HRMS (ESI):** m/z calculated: C₃₃H₅₁NO₆Si₂ (M+H)⁺ 614.3328, found 614.3324.

### ((2S,3S)-3-methyloxiran-2-yl)methyl 4-methylbenzenesulfonate ((S,S)-19)

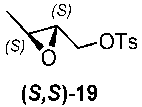

A flame dried flask was charged with 10 g of crushed activated 3 Å molecular sieves and flushed with nitrogen for several minutes. Then, DCM (200 ml) was added and the flask was cooled to -20°C. (+)-Diisopropyl tartrate (DIPT) (1.75 g, 7.49 mmol, 0.06 eq), crotyl alcohol (9.00 g, 125 mmol, 1.00 eq) and Ti(O*i*Pr)₄ (1.77 g, 6.24 mmol, 0.05 eq) were added sequentially at this temperature. The resulting mixture was stirred for 15 min at -20°C, then a solution of tert-butyl hydroperoxide (TBHP, 5 M in DCM) (50.0 ml, 150 mmol, 2.00 eq) was added dropwise. The reaction mixture was stirred for 2 h at this temperature. Careful quenching of the excess TBHP was carried out by careful addition of trimethyl phosphite (22.0 ml, 187 mmol, 1.50 eq) at -20°C after which trimethyl amine (26.1 ml, 187 mmol, 1.50 eq), DMAP (1.83 g, 15.0 mmol, 0.12 eq) and a solution of p-toluenesulfonyl chloride (23.8 g, 125 mmol, 1.00 eq) in DCM (100ml) was added sequentially. The temperature was raised to -10°C and the reaction mixture stirred for 36 h. Afterwards the mixture was filtered through a pad of Celite and washed with DCM. The filtrate was then washed with 10 % tartaric acid (2x100ml), saturated NaHCO₃ (2x100ml) and brine (2x100ml). The organic phase was dried over MgSO₄ and evaporated under reduced pressure. The crude product was purified by column chromatography on silica gel (hexane /EtOAc, 2:1) and recrystallized (Et₂O/hexane) in order to afford the tosylate **(S,S-19))**) as white needles (20.2 g, 67%).
**¹H NMR** (CDCl₃-d¹, 400 MHz): δ = 1.30 (d, *J*=5.27 Hz, 3 H), 2.46 (s, 3 H), 2.87 - 2.94 (m, 1 H), 2.92 (s, 1 H), 3.98 (dd, *J*=11.42, 5.90 Hz, 1 H), 4.18 (dd, *J*=11.42, 3.89 Hz, 1 H), 7.36 (d, *J*=8.28 Hz, 2 H), 7.80 ppm (d, *J*=8.53 Hz, 2 H).
**¹³C NMR** (CDCl₃-d¹, 100 MHz): δ = 16.95, 21.64, 52.77, 55.45, 70.03, 127.94, 129.89, 132.70, 145.06 ppm.

**HRMS (ESI):** m/z calculated: C₁₁H₁₄O₄S (M+H)⁺ 243.0686, found 243.0678.

### (S)-But-3-en-2-ol ((S)-3):

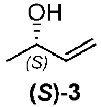

A solution of tosylate **(*S,S*)-19** in dry THF (20ml) was added to a suspension of Zinc-Copper-couple (6g) and dry Nal (22.3 g, 149 mmol, 3.00 eq) in dry THF (150ml). The resulting suspension was stirred for 2h at 70°C, cooled to room temperature and filtered through a pad of silica. Afterwards the THF-butenol mixture was distilled under reduced pressure (200 mbar, 100°C) while the collecting flask was cooled to -78°C (dry ice). The THF-butenol solution was then submitted to the next step without further purification.

### (S)-But-3-en-2-yl tert-butyl carbonate ((S)-4):

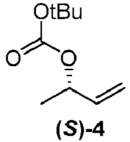

The butenol-THF solution of cooled to 0°C and NaH (5.82 g, 145 mmol, 3.00 eq) was carefully added under a nitrogen atmosphere in small portions. Then Boc₂O (11.7 g, 53.4 mmol, 1.1 eq) was added in portion-wise over 10 min under vigorous stirring at this temperature. The reaction mixture was allowed to warm to room temperature overnight and then diluted with Et₂O after 16h of vigorous stirring. Excess sodium hydride was quenched by the slow addition of water. The resulting mixture was then extracted with diethyl ether (3x50 ml). The combined organic extracts were washed with brine (1x50 ml), dried over MgSO₄ and concentrated under reduced pressure. After purification by column chromatography on silica gel (hexane/EtOAc, 10:1) the product **(*S*)-4** was obtained as a colourless liquid (6.25 g, 75% two steps).

### 3-(6-Benzoxy-1H-indol-3-yl)-2-acetylaminopropionic acid (30):

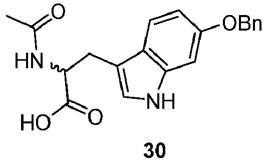

A suspension of L-serine (942 mg, 8.96 mmol, 4.00 eq.) in Ac₂O (4.02 mL, 42.6 mmol, 9.5 eq.) and AcOH (22 mL) was stirred for 16 h at r.t. 6-benzoxyindol **29** was added and after a reaction time of 2 h at 75°C the solvent was removed under reduced pressure. After the residue was taken up in water (50 mL) and the pH was adjusted to 11, the aqueous layer was washed with MTBE (2x50 mL) and acidified to pH = 3. The aqueous layer was extracted with EtOAc (4 x 50 mL), dried over Na₂SO₄ and the solvent was removed under reduced pressure. The crude product was recrystallized in MeOH, which gave compound **30** as a light brown solid (620 mg, 82%).

### (S)-3-amino-3-(6-(benzoxy)-1H-indol-3-yl)propanoic acid - Schiff Base Complex (32):

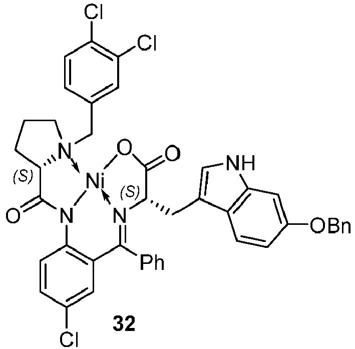

A suspension of **30** (1.03 g, 3.32 mmol) in 40% NaOH (H₂O/MeOHv/v = 1:1, 14 mL/mmol) was stirred at 110°C for 4 h, neutralized with conc. HCI to pH =7 and the solvent was removed *in vacuo.* The crude product was dissolved in MeOH (20 mL/mmol) and Ni(OAc)₂^{∗}6H₂O (966 mg, 3.32 mmol) and (S)-28 (1.78 g, 3.65 mmol) were added followed by K₂CO₃ (2.09 g, 15.1 mmol). The resulting mixture was refluxed for 16 h and the precipitate was filtered of and washed with DCM. The solvent of the filtrate was removed *in vacuo* and the crude product was suspended in DCM, washed with H₂O and dried with Na₂SO₄. After removal of the solvent *in vacuo* the crude product was purified with DCM/MeOH (v/v = 40:1) to give the product **32** as an orange solid (2.44 g, 88%).

### (S)-6-benzoxytryptophan (33):

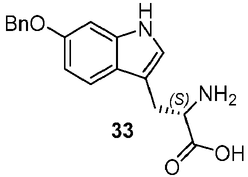

To a solution of **32** (180mg, 0.51 mmol) in MeOH was added 6 M HCI (15 mL) The solution was stirred for 45 min at 70°C and conc. NH₄OH-solution was added until pH=7 was reached. The aqueous layer was washed with EtOAc (2 x 15 mL), and the pH was adjusted to 10. The precipitate was centrifuged and washed two times with water (pH = 10), to give (S)-6-benzoxytryptophan as a white solid.

### (S)-3-(6-(benzyloxy)-1H-indol-3-yl)-2-(((2-(trimethylsilyl)ethoxy)carbonyl)amino)propanoic acid (33a)

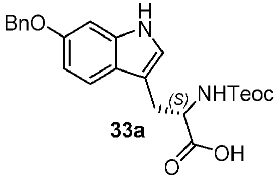

Triethylamine (446 µL g, 3.22 mmol, 2.00 eq.) and TeocOSu (626 mg, 2.42 mmol, 1.50 eq.) was added successively to a solution of L-6-benzoxytryptophan (**33,** 0.5 g, 1.61 mmol, 1.00 eq.) in DMF (20 mL). The reaction mixture was stirred at r.t. for 2 h, then concentrated under reduced pressure .The aqueous layer was carefully acidified to pH = 4 by dropwise addition of 1 M HCI and extracted with EtOAc (3 x 100 mL). The organic phase was washed with brine (2 x 100 mL), dried over Na₂SO₄ and evaporated under reduced pressure to afford the product **33a** as a white solid (665 mg, 91%).
**HRMS** (ESI): *m*/*z* calc. for C₁₇H₂₄N₂O₄Si (M+H)⁺ 455.1997, found 455.1999.

### ((2-(trimethylsilyl)ethoxy)carbonyl)-L-tryptophan (37a)

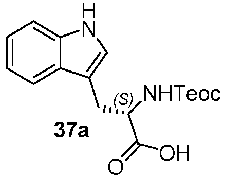

Triethylamine (11.3 mL g, 80.9 mmol, 1.5 eq.) and TeocOSu (18.2 g, 70.0 mmol, 1.30 eq.) was added successively to a solution of L-tryptophan (**37,** 11.0 g, 53.9 mmol, 1.00 eq.) in a 1:1 mixture of dioxane/water (200 mL). The reaction mixture was stirred at r.t. for 2 h, then concentrated under reduced pressure .The aqueous layer was carefully acidified to pH = 4 by dropwise addition of 1 M HCI and extracted with EtOAc (3 x 100 mL). The organic phase was washed with brine (2 x 100 mL), dried over Na₂SO₄ and evaporated under reduced pressure to afford the product **37a** as a white solid (18.2 g, 97%).
**HRMS** (ESI): *m*/*z* calc. for C₁₇H₂₄N₂O₄Si (M+H)⁺ 349.1578, found 349.1582.

### 2,2,2-trichloroethyl ((2-(trimethylsilyl)ethoxy)carbonyl)-L-tryptophanate (38)

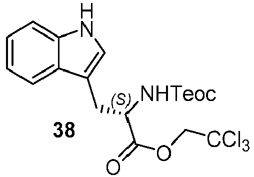

To a solution of N-Teoc protected L-tryptophane (**37a,** 2.80 g, 8.04 mmol, 1.00 eq.) in DCM (32 mL) at 0°C was added DMAP (147 mg, 1.21 mmol, 0.15 eq.) and EDC*HCl (2.00 g, 10.4 mmol, 1.30 eq.) successively . After stirring at 0°C for 10 min 2,2,2-trichloroethanol (1.54 mL, 16.1 mmol, 2.00 eq.) was added and the solution was stirred for 2 h at r.t. The reaction mixture was diluted with DCM (100 mL), washed with 0.5 M HCI (2 x 50 mL), sat. NaHCO₃ solution (50 mL) and brine (50 mL). After drying over NaSO₄ and removal of the solvent under reduced pressure the crude product was purified by column chromatography on silica gel (1% MeOH/DCM) to afford compound **37a** as a pale yellow solid (3.51 g, 91%).
**HRMS** (ESI): *m*/*z* calc. for C₁₉H₂₅Cl₃N₂O₄Si (M+H)⁺ 479.0722, found 479.0721.

### 2,2,2-trichloroethyl(S)-3-(6-(benzyloxy)-1H-indol-3-yl)-2-(((2-(trimethylsilyl)ethoxy)carbonyl)-amino)propanoate (39)

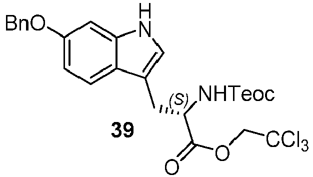

To a solution of compound **33a** (1.0 g, 2.2 mmol, 1.0 eq.) in DCM (8.8 mL) at 0°C was added DMAP (40.3 mg, 0.329 mmol, 0.15 eq.) and EDC*HCl (548 mg, 2.86 mmol, 1.30 eq.) successively. After stirring at 0°C for 10 min 2,2,2-trichloroethanol (0.42 mL, 4.4 mmol, 2.00 eq.) was added and the solution was stirred for 2 h at r.t. The reaction mixture was diluted with DCM (50 mL), washed with 0.5 M HCI (2 x 25 mL), sat. NaHCO₃ solution (25 mL) and brine (25 mL). After drying over Na₂SO₄ and removal of the solvent under reduced pressure the crude product was purified by column chromatography on silica gel (0.5% MeOH/DCM) to afford compound **39** as a yellow oil (1.1 g, 85%).
**HRMS** (ESI): *m*/*z* calc for C₂₆H₃₁Cl₃N₂O₅Si (M+H)⁺ 585.1141, found 585.1139.

### Synthesis of (N-Boc)₂-cystine-(OtBu)₂ (35)

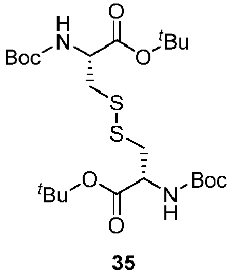

A solution of L-cystine-(OtBu)₂ (**34,** 10 g, 24 mmol, 1.0 eq.) in a 1:1 mixture of H₂O/dioxane (240 mL) was treated with NaHCO₃ (8.06 g, 96.0 mmol, 4.00 eq.) and Boc₂O (10.1 mL, 47.0 mmol, 2.00 eq.) and the reaction mixture was stirred for 16 h at r.t. The reaction mixture was concentrated under reduced pressure and the aqueous layer was extracted with EtOAc (3 x 120 mL). The organic layer was washed with brine (100 mL), dried over Na₂SO₄ and the solvent was removed under reduced pressure to afford **35** (13.2 g, 24.0 mmol, quant.) as a pale yellow solid.
**HRMS** (ESI): *m*/*z* calc for C₂₄H₄₄N₂O₈S₂ (M+H)⁺ 553.2612, found 553.2615.

### tert-butyl S-(6-(benzyloxy)-3-((S)-3-oxo-3-(2,2,2-trichloroethoxy)-2-(((2-(trimethylsilyl)ethoxy) carbonyl)amino)propyl)-1 H-indol-2-yl)-N-(tert-butoxycarbonyl)-L-cysteinate (40)

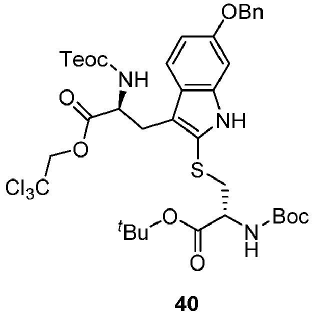

To a solution of (*N*-BOC)₂-L-Cystin-(OtBu)₂ (**35,** 900 mg, 1.63 mmol, 1.00 eq.) in CHCl₃ (16.3 mL) was added SO₂Cl₂ (263 µL, 3.26 mmol, 2.00 eq.). After the reaction mixture was stirred for 1 h at r.t. the solvent was removed under reduced pressure. The residue was redissolved in CHCl₃ (16.3 mL) and cooled to 0°C and added to an ice cold solution of **39** (800 mg, 1.67 mmol, 1.00 eq.) and NaHCO₃ (420 mg, 5.00 mmol, 3.00 eq.) in CHCl₃ (16.7 mL) dropwise over a periode of 10 min. Afterwards the reaction mixture was stirred for 15 min at 0°C and 1 h at r.t.. The organic layer was washed with H₂O (10 mL) and sat. NaHCO₃-solution (10 mL). After drying of the organic layer with Na₂SO₄ and removal of the solvent under reduced pressure the crude product of **40** was used in the next step without further purification.
**HRMS** (ESI): *m*/*z* calc. for C₃₈H₅₂Cl₃N₃O₉SSi (M+H)⁺ 860.2332, found 860.2323.

### tert-butyl N-(tert-butoxycarbonyl)-S-(3-((S)-3-oxo-3-(2,2,2-trichloroethoxy)-2-(((2-(trimethylsilyl)ethoxy)carbonyl)amino)propyl)-1H-indol-2-yl)-L-cysteinate (41)

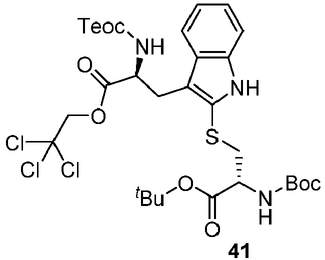

To a solution of (*N*-BOC)₂-L-Cystin-(OtBu)₂ (**39,** 5.06 g, 9.15 mmol, 1.00 eq.) in CHCl₃ (92 mL) was added SO₂Cl₂ (1.48 mL, 18.3 mmol, 2.00 eq.). After the reaction mixture was stirred for 1 h at r.t. the solvent was removed under reduced pressure. The residue was redissolved in CHCl₃ (92 mL), cooled to 0°C and added dropwise to an ice cold solution of **38** (4.4 g, 9.17 mmol 1.00 eq.) and NaHCO₃ (2.31 g, 27.5 mmol, 3.00 eq.) in CHCl₃ (92 mL) over a periode of 10 min. Afterwards the reaction mixture was stirred for 15 min at 0°C and 1 h at r.t. The organic layer was washed with H₂O (2 x 100 mL) and sat. NaHCO₃-solution (2 x 80 mL). After drying of the organic layer with Na₂SO₄ and removal of the solvent under reduced pressure the crude product of **41** was used in the next step without further purification.
HRMS (ESI): *m*/*z* calc. for C₃₁H₄₆Cl₃N₃O₉SSi (M+H)⁺ 754.1913, found 754.1917.

### (S)-3-(6-(benzyloxy)-2-(((R)-3-(tert-butoxy)-2-((tert-butoxycarbonyl)amino)-3-oxopropyl)thio)-1H-indol-3-yl)-2-(((2-(trimethylsilyl)ethoxy)carbonyl)amino)propanoic acid (49):

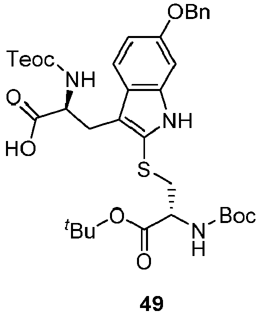

A solution of tryptathionine derivative **39** (1.63 mmol, 1.00 eq.) in DMF (8.4 mL) was treated with CH₃COOH (0.8 mL) and zinc (3.51 g, 53.6 mmol, 33.0 eq.) for 2 h at 45°C. The reaction mixture was filtered over Celite and the solvent was removed under reduced pressure. The crude product was dissolved in EtOAc (50 mL) and washed with 10% KHSO₄ solution (2 x 25 mL) and brine (2 x 25 mL). After drying over Na₂SO₄ and removing of the solvent under reduced pressure, the crude product was purified by C18 reverse phase chromatography (AcN/H₂O 50% to 100% gradient) to give compound 4**9** as a yellow solid (840 mg, 83% over 2 steps).
**HRMS** (ESI): *m*/*z* calc. for C₃₆H₅₁N₃O₉SSᵢ (M+H)⁺ 730.3183, found 730.3188.

### (S)-3-(2-(((R)-3-(tert-butoxy)- 2-((tert-butoxycarbonyl)amino)-3-oxopropyl)thio)-1H-indol-3-yl)-2-(((2-(trimethylsilyl)ethoxy)carbonyl)amino)propanoic acid (50)

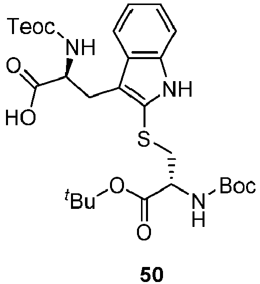

A solution of tryptathionine derivative **38** (9.15 mmol, 1.00 eq.) in DMF (40 mL) was treated with CH₃COOH (4 mL) and zinc (20.0 g, 302 mmol, 33.0 eq.) for 2 h at 45°C. The reaction mixture was filtered over Celite and the solvent was removed under reduced pressure. The crude product was dissolved in EtOAc (200 mL) and washed with 10% KHSO₄ solution (2 x 50 mL) and brine (2 x 50 mL). After drying over Na₂SO₄ and removing of the solvent under reduced pressure, the crude product was purified by C18 reverse phase chromatography (AcN/H₂O 50% to 100% gradient) to afford compound **50** as a yellow oil (5.0 g, 88%. over 2 steps).
**HRMS** (ESI): *m*/*z* calc. for C₃₁H₄₆Cl₃N₃O₈SSi (M+H)⁺ 624.2769, found 624.2775.

### ((benzyloxy)carbonyl)glycyl-L-isoleucine (44)

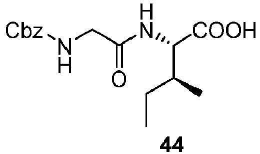

To a solution of Cbz-glycine (**42,** 10.0 g, 32.7 mmol, 1.00 eq.) in acetone (100 mL) was added a suspension of L-isoleucine (4.71 g, 35.9 mmol, 1.10 eq.) and NaHCO₃ (8.23 g, 87.9 mmol, 3.00 eq.) in water (100 mL). The reaction mixture was stirred at r.t. for 3 h and concentrated under reduced pressure .The aqueous layer was carefully acidified to pH = 4 by dropwise addition of 1 M HCI and extracted with EtOAc (3x150 mL). The organic phase was then washed with brine (2 x 100 mL), dried over Na₂SO₄ and evaporated under reduced pressure to afford the product **44** as a colourless oil (10.1 g, 96%).
**HRMS** (ESI): *m*/*z* calc. for C₁₆H₂₂N₂O₅ (M+H)⁺ 323.1601, found 323.1606.

### Glycyl-L-isoleucylglycine (45):

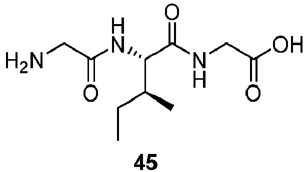

Dipeptide **44** (10.1 g, 31.3 mmol, 1.00 eq.) and benzyl glycinate (8.21 g, 40.7 mmol, 1.30 eq.) were dissolved in dry DMF (125 mL). Then, COMU (17.4 g, 40.7 mmol, 1.30 eq.) and DIPEA (12.6 mL, 72.1 mmol, 3.00 eq.) were added at 0°C. The reaction mixture was allowed to warm to r.t. overnight and diluted with EtOAc (300 mL) afterwards. After washing with a solution of 10% KHSO₄-solution (2x100 mL) the fully protected tripeptide precipitated in the organic phase. The organic phase was cooled to 4°C for 4 h in order for the peptide to precipitate, then the precipitate was filtered and washed with cold EtOAc. The precipitate was redissolved in a 1:1 mixture of water and THF (260 mL). Pd/C (1 g) was added to the solution after degassing with N₂ for 30 min. Then, the reaction mixture was degassed with hydrogen for 1 h. After vigorous stirring at room temperature under 1.0 atm of hydrogen overnight, the catalyst was filtered through a pad of Celite. Afterwards, the mixture was concentrated under reduced pressure to obtain the product **45** as a white solid (5.71 g, 74 %)
**HRMS** (ESI): *m*/*z* calc. for C₁₀H₁₉N₃O₄ (M+H)⁺ 246.1448, found 246.1440.

### Synthesis of pentapeptide 51:

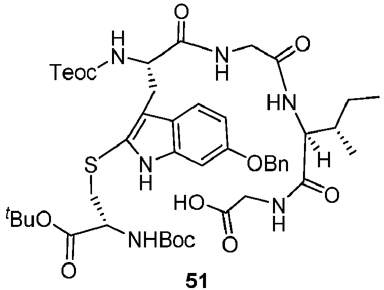

A solution of thioether building block **49** (111 mg, 0.14 mmol, 1.00 eq.) in AcN (0.7 mL) was treated with collidine (37 µL, 0.27 mmol, 2.0 eq) and *N,N'*-disuccinimidyl carbonate (39 mg, 0.15 mmol, 1.1 eq.) and stirred for 1 h at r.t.. A solution of tripeptide **45** (44 mg, 0.18 mmol, 1.3 eq) in a 1:4 mixture of AcN/H₂O (1 mL) was added and the reaction mixture was stirred for 2 h at r.t.. Afterwards, the mixture was diluted with EtOAc (20 mL), 10% KHSO₄-solution (20 mL) was added and the aqueous layer was extracted with EtOAc (2 x 20 mL). The organic layer was washed with brine (2 x 20 mL), dried over Na₂SO₄ and evaporated under reduced pressure which afforded pentapeptide **51** as a yellow solid (115 mg, 90%).
HRMS (ESI): *m*/*z* calc. for C₄₆H₆₈N₆O₁₂SSi (M+H)⁺ 957.4458, found 957.4457.

### Synthesis of pentapeptide 52:

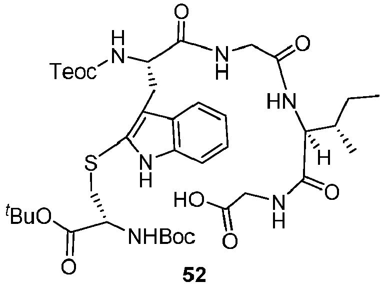

A solution of tryptathionine building block **50** (2.0 g, 2.5 mmol, 1.0 eq.) in AcN (10 mL) was treated with collidine (659 µL, 4.95 mmol, 2.00 eq) and *N,N'*-disuccinimidyl carbonate (697 mg, 2.72 mmol, 1.10 eq.) and stirred for 1 h at r.t.. A solution of tripeptide **45** (790 mg, 3.22 mmol, 1.30 eq.) in a 1:4 mixture of AcN/H₂O (18 mL) was added and the reaction mixture was stirred for 2 h at r.t.. Afterwards, the mixture was diluted with EtOAc (100 mL), 10% KHSO₄-solution (20 mL) was added and the aqueous layer was extracted with EtOAc (2x50 mL). The organic layer was washed with brine (2 x 50 mL), dried over Na₂SO₄ and evaporated under reduced pressure which afforded pentapeptide **52** as a yellow solid (2.15 g, 93%).
**HRMS** (ESI): *m*/*z* calc. for (M+H)⁺ C₃₆H₅₁Cl₃N₆O₁₁S 851.4039, found 851.4058.

### Fully protected cyclic pentapeptide 53:

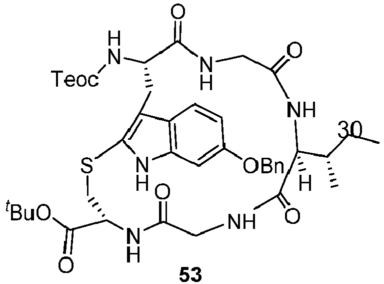

Pentapeptide **51** (151 mg, 0.180 mmol, 1.00 eq.) was dissolved in 1 mL of a solution of p-toluenesulfonic acid in THF (1.8 M) and was stirred for 4 h at r.t. Then, the reaction mixture was neutralized by the addition of DIPEA (320 µL, 1.84 mmol, 10 eq) and diluted with DCM (180 mL). Afterwards, DIPEA (60.2 µL, 354 µmol, 2.00 eq.) and T3P (50% in EtOAc, 210 µL, 354 µmol, 0.34 eq.) were added. After the solution was stirred for 16 h at r.t. 2/3 of the the solvent was concentrated under reduced pressure. The organic phase was washed with 10% KHSO₄-solution (20 mL), sat. NaHCO₃-solution (20 mL), water (20 mL) and brine (20 mL). The organic layer was dried over Na₂SO₄ and the solvent was removed under reduced pressure. The crude product was purified by C18 reverse phase chromatography (AcN/H₂O 50% to 100% gradient) to afford cyclic pentapeptide **53** as a yellow solid (82 mg, 70%)
**HRMS** (ESI): *m*/*z* calc. for C₄₁H₅₈N₆O₉SSi (M+H)⁺ 839.3828, found 839.3839.

### Fully protected cyclic pentapeptide (54):

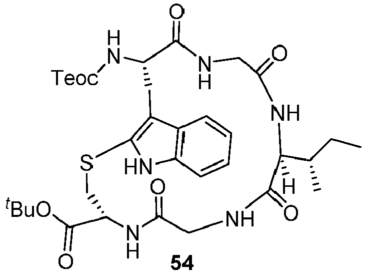

Pentapeptide **52** (700 mg, 0.822 mmol, 1.00 eq.) was dissolved in 10 mL of 2 M HCl in dioxane and stirred for 40 min at r.t. Then, the reaction mixture diluted with 40 mL of dioxane and the solvent was evaporated under reduced pressure. The precipitate was dissolved in 8 mL DMF and diluted with 82 mL DCM. Afterwards, DIPEA (279 µL, 1.64 mmol, 2.00 eq.) and T3P (50% in EtOAc, 977 µL, 1.64 mmol, 2.00 eq.) were added. After the solution was stirred for 5 h at r.t., 1/3 of the the solvent was concentrated under reduced pressure. The organic phase was washed with 10% KHSO₄-solution (20 mL), sat. NaHCO₃-solution (20 mL), water (20 mL) and brine (20 mL). The organic layer was dried over Na₂SO₄ and the solvent was removed under reduced pressure. The crude product was purified by C18 reverse phase chromatography (AcN/H₂O 50% to 100% gradient) to afford cyclic pentapeptide **54** as a yellow solid (420 mg, 72%).
**HRMS** (ESI): *m*/*z* calc. for C₃₄H₅₂N₆O₈SSi (M+H)⁺ 733.3409, found 733.3409.

### Fully deprotected monocyclic pentapeptide 55:

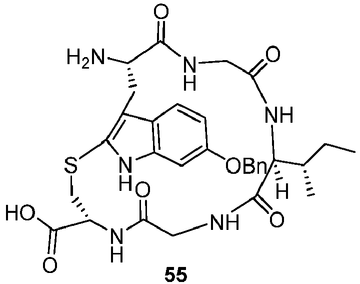

Monocyclic Pentapeptide 53 (125 mg, 0.17 mmol, 1.00 eq.) was stirred in TFA/DCM/TIPS (8:1.5:0.5) for 2 h at r.t. The solvent was removed under reduced pressure and the crude product was purified by C18 reverse phase chromatography (AcN/H₂O 20% to 100%) to afford the fully deprotected monocyclic pentapeptide 55 as a white powder (100 mg, quant.).
**HRMS** (ESI): *m*/*z* calc. for C₃₁H₃₈N₆O₇S (M+H)⁺ 639.2595, found 639.2590.

### Fully deprotected monocyclic pentapeptide 56:

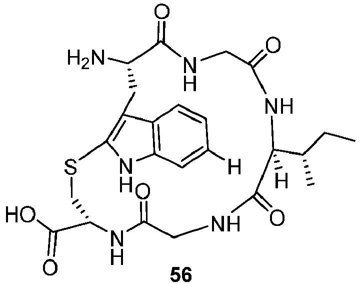

Monocyclic Pentapeptide **54** (250 mg, 0.34 mmol, 1.00 eq.) was stirred in TFA/DCM/TIPS (8:1.5:0.5) for 2 h at r.t. The solvent was removed under reduced pressure and the crude product was purified by C18 reverse phase chromatography (AcN/H₂O 10% to 30%) to afford the fully deprotected monocyclic pentapeptide **56** as a white powder (200 mg, quant.).
**HRMS** (ESI): *m*/*z* calc. for C₂₄H₃₂N₆O₆S (M+H)⁺ 533.2177, found 533.2188.

### Tripeptide (48):

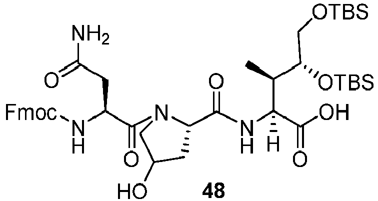

**HRMS** (ESI): *m*/*z* calc. for C₄₂H₆₄N₄O₁₀Si₂ (M+H)⁺ 841.4233, found 841.4253.

### Monocyclic octapeptide 57:

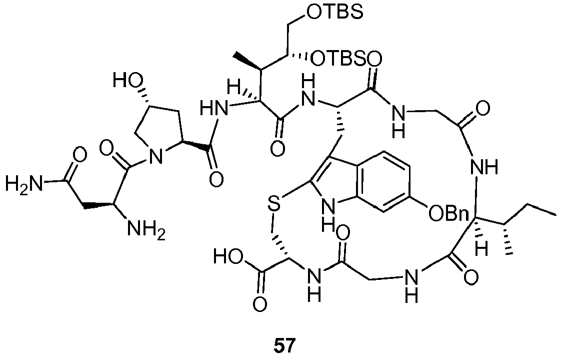

A solution of fully deprotected monocyclic pentapeptide **55** (50.0 mg, 0.078 mmol, 1.00 eq.) and MSA (13.8 µL, 0.86 mmol, 1.1 eq.) in DMA (1.5 mL) was stirred for 2 h at 50°C. Separately, a solution of Fmoc-Asn-Hyp-DHIIe(TBS)₂-OH (98.8 mg, 0.12 mmol, 1.50 eq.), COMU (36.9 mg, 0.086 mmol, 1.10 eq.) and DIPEA (15.0 µL, 0.083 mmol, 1.10 eq.) in DMA (0.4 mL) was stirred for 30 min at 0°C. The silylated monocyclic peptide was then added to the activated tripeptide and stirred for 1 h at 0°C then at 35°C for 3 h. Et₂NH (82.0 µL, 0.078 mmol, 10.0 eq.) was added and stirred for 1 h at r.t. The solvent was removed under reduced pressure and the crude product was purified using preparative HPLC (Sunfire Prep C18 OBD 10µm, 50x150 mm column, gradient A) to afford octapeptide **57** as a white solid (65.5 mg, 68%).
**HRMS** (ESI): *m*/*z* calc. for C₅₈H₉₀N₁₀O₁₄SSi₂ (M+H)⁺ 1239.5970, found 1239.5980.

### Monocyclic octapeptide 58:

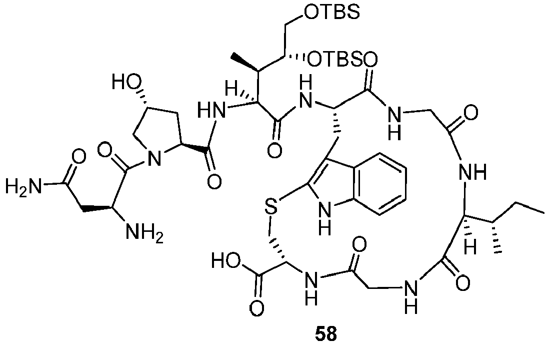

A solution of fully deprotected monocyclic pentapeptide **56** (40.0 mg, 0.075 mmol, 1.00 eq.) and MSA (12 µL, 0.075 mmol, 1.00 eq.) in DMA (1.5 mL) was stirred for 2 h at 50°C. Similtaneously, a solution of Fmoc-Asn-Hyp-DHIIe(TBS)₂-OH (95 mg, 0.11 mmol, 1.50 eq.), COMU (35.0 mg, 0.083 mmol, 1.10 eq.) and DIPEA (14.0 µL, 0.083 mmol, 1.10 eq.) in DMA (0.4 mL) was stirred for 30 min at 0°C. The silylated monocyclic peptide was then added to the activated tripeptide and stirred for 1 h at 0°C then at 35°C for 3 h. Et₂NH (77 µL, 0.75 mmol, 10 eq.) was added and stirred for 2 h at r.t. The solvent was removed under reduced pressure and the crude product was purified using preparative HPLC (Sunfire Prep C18 OBD 10µm, 50x150 mm column, gradient A) to afford octapeptide **58** as a white powder (70 mg, 68%).
**HRMS** (ESI): *m*/*z* calc. for C₅₁H₈₄N₁₀O₁₃SSi₂ (M+H)⁺ 1133.5551, found 1133.5549.

### Monocyclic octapeptide 59:

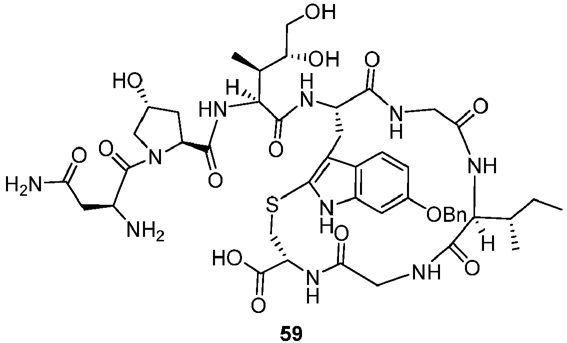

A solution of TBAF in THF (1 M, 0.52 mL, 10.0 eq) was added to a solution of octapeptide **57** (70 mg, 62 mmol, 1.0 eq) in THF (61.8 mL) and stirred for 2 h at r.t.. The solvent was evaporated *in vacuo* and the crude product purified by C18 reverse phase chromatography (AcN/H₂O 5% to 30%) to afford the product **59** as a white solid (45 mg, 85%).
**HRMS** (ESI): *m*/*z* calc. for C₄₆H₆₂N₁₀O₁₄S (M+H)⁺ 1011.4240, found 1001.4247.

### Monocyclic octapeptide 60:

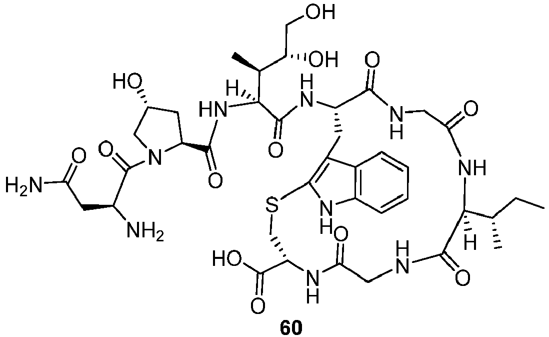

A solution of TBAF in THF (1 M, 0.62 mL, 10.0 eq) was added to a solution of octapeptide **58** (70 mg, 62 mmol, 1.0 eq) in THF (0.62 mL) and stirred for 2 h at r.t.. The solvent was evaporated *in vacuo* and the crude product purified by C18 reverse phase chromatography (AcN/H₂O 5% to 60%) to afford the product **61** as a white solid (49 mg, 88%).
**HRMS** (ESI): *m*/*z* calc. for C₃₉H₅₆N₁₀O₁₃S (M+H)⁺ 905.3822, found 905.38113.

### 2-((9H-fluoren-9-yl)methyl) 1-(tert-butyl) (2S,4R)-4-hydroxypyrrolidine-1,2-dicarboxylate (64):

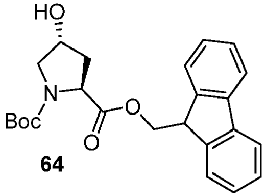

A solution of *N*-Boc-protected (2*S*,4*R*)-4-hydroxyproline **63** (5.0 mg, 22 mmol, 1.0 eq.) in DMF (20 mL) was added dropwise to a solution of 9-Fluorenemethanol (8.5 mg, 43 mmol, 2.0 eq.), EDC*HCl (8.3 g, 43 mmol, 2.0 eq.) and DMAP (396 mg, 3.24 mmol, 0.150 eq.) in DCM (220 mL). The reaction mixture was stirred at r.t. for 2 h. Then, 10% KHSO₄ solution (50 mL) was added. The organic phase was washed with brine (50 mL) and dried over NaSO₄. Afterwards, the solvent was removed under reduced pressure and the crude product was purified by column chromatography on silica gel (hexane/ethyl acetate = 1:1) to afford compound **64** as a white solid (5.0 g, 56%).

### (9H-fluoren-9-yl)methyl (2S,4R)-4-hydroxypyrrolidine-2-carboxylate (64a):

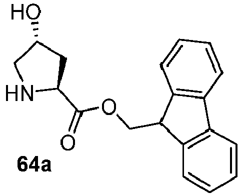

**64** (5.0 g, 22 mmol, 1.0 eq.) was treated with 4 M HCI in Dioxane (30 mL) at r.t. for 1 h. Afterwards, the solvent was evaporated under reduced pressure to afford the product (**64a**) as a white solid (3.7 g, quant.).
**HRMS** (ESI): *m*/*z* calc. for C₁₉H₁₉NO₃ (M+H)⁺ 310.1438, found 310.1426.

### (9H-fluoren-9-yl)methyl (2S,4R)-1-((tert-butoxycarbonyl)-L-asparaginyl)-4-hydroxypyrrolidine-2-carboxylate (65)

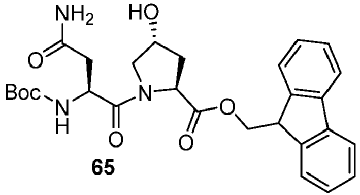

N-Boc-protected asparagine (1.7 g, 7.3 mmol, 1.5 eq.), **64a** (1.5 g, 4.8 mmol, 1.0 eq.), EDC*HCl (1.4 g, 7.3 mmol, 1.5 eq.) and HOBt*H₂O (1.5 g, 9.7 mmol, 2.0 eq.) were dissolved in DMF (72 mL) and stirred at r.t. for 16 h. The reaction mixture was diluted with EtOAc (200 mL). Then, 10% KHSO₄ solution (50 mL) was added. The organic phase was washed with 10% KHSO₄ solution (50 mL) and brine (2 x 50 mL). After drying over NaSO₄ and removal of the solvent under reduced pressure the crude product was purified by C18 reversed phase chromatography (AcN/H₂O 20% to 70%) to afford dipeptide **65** as a white powder (1.6 g, 78%).
**HRMS** (ESI): *m*/*z* calc. for C₂₈H₃₃N₃O₇ (M+H)⁺ 524.2391, found 524.2396.

### (9H-fluoren-9-yl)methyl (2S,4R)-1-(L-asparaginyl)-4-hydroxypyrrolidine-2-carboxylate (66)

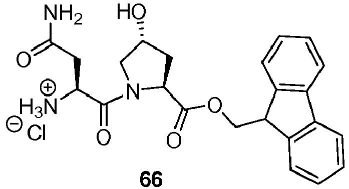

**65** (1.0 g, 1.9 mmol, 1.0 eq.) was treated with 4 M HCI in Dioxane (30 mL) at r.t. for 1 h. Afterwards, the solvent was evaporated under reduced pressure to afford the product (**66**) as a white solid (870 mg, quant.).
**HRMS** (ESI): *m*/*z* calc. for C₂₃H₂₅N₃O₅ (M+H)⁺ 424.1866, found 424.1858.

### Monocyclic hexapeptide 67:

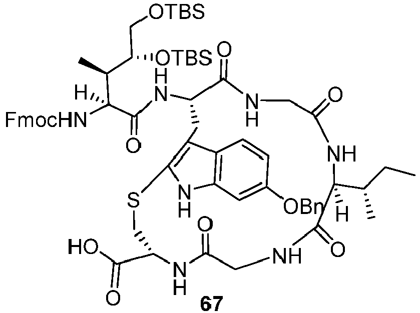

A solution of fully deprotected monocyclic pentapeptide **55** (42 mg, 0.66 mmol, 1.00 eq.) and MSA (11.6 µL, 0.723 mmol, 1.10 eq.) in DMA (2 mL) was stirred for 2 h at 50°C. Simultaneously, a solution of Fmoc-DHIIe(TBS)₂-OH (**13**, 52 mg, 0.85 mmol, 1.30 eq.), COMU (36 mg, 0.85 mmol, 1.30 eq.) and DIPEA (15 µL, 0.85 mmol, 1.30 eq.) in DMA (0.4 mL) was stirred for 30 min at 0°C. The silylated monocyclic peptide was then added to the activated dihydroxyisoleucine derivative and stirred for 1 h at 0°C then at r.t. overnight. Afterwards, the mixture was diluted with EtOAc (50 mL) and washed with 10% KHSO₄ solution (3 x 5 mL). The organic phase was washed with brine (2 x 20 mL), dried over NaSO₄ and evaporated under reduced pressure. The crude product of **67** was used in the next step without any further purification.
**HRMS** (ESI): *m*/*z* calc. for C₆₄H₈₇N₇O₁₂SSi₂ (M+H)⁺ 1234.5745, found 1234.5745.

### Monocyclic hexapeptide 68:

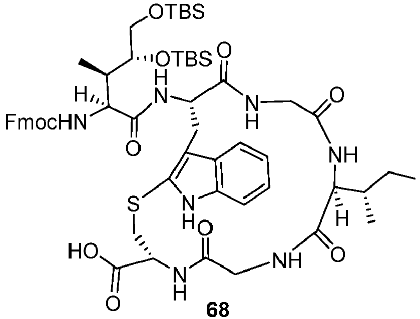

A solution of fully deprotected monocyclic pentapeptide **56** (100 mg, 0.188 mmol, 1.00 eq.) and MSA (33.2 µL, 0.207 mmol, 1.10 eq.) in DMA (4 mL) was stirred for 2 h at 50°C. Simultaneously, a solution of Fmoc-DHIIe(TBS)₂-OH (**13,** 149 mg, 0.244 mmol, 1.30 eq.), COMU (104 mg, 0.244 mmol, 1.30 eq.) and DIPEA (42.5 µL, 0.244 mmol, 1.30 eq.) in DMA (1.25 mL) was stirred for 30 min at 0°C. The silylated monocyclic peptide was then added to the activated dihydroxyisoleucine derivative and stirred for 1 h at 0°C then at r.t. overnight. Afterwards, the mixture was diluted with EtOAc (100 mL) and washed with 10% KHSO₄ solution (3 x 10 mL). The organic phase was washed with brine (2 x 25 mL), dried over NaSO₄ and evaporated under reduced pressure. The crude product of **68** was used in the next step without any further purification.
**HRMS** (ESI): *m*/*z* calc. for C₅₇H₈₁N₇O₁₁SSi₂ (M+H)⁺ 1128.5326, found 1128.5316.

### Monocyclic octapeptide 69:

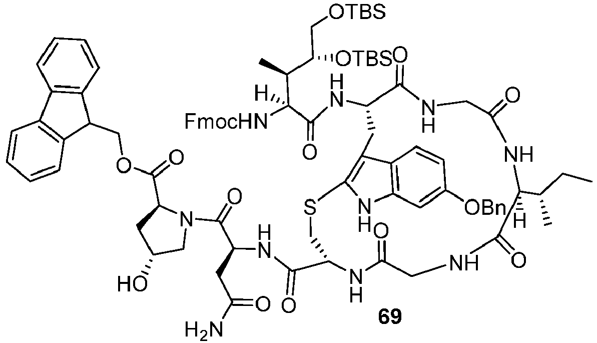

Crude monocyclic hexapeptide **67** (0.66 mmol, 1.0 eq.), dipeptide **66** (42 mg, 0.10 mmol, 1.50 eq.) were dissolved in DMF (1.5 mL). Then, DIPEA (17.3 mL, 0.10 mmol, 1.50 eq.) and HATU (38 mg, 0.10 mmol, 1.5 eq) were added at 0°C. The reaction mixture was allowed to warm to r.t. overnight and concentrated under reduced pressure. The crude product was purified by C18 reversed phase chromatography (AcN/H₂O 60% to 100%) to fully protected octapeptide **69** as a white solid (60 mg, 55% over two steps).
**HRMS** (ESI): *m*/*z* calc. for C₈₇H₁₁₀N₁₀O₁₆SSi₂ (M+H)⁺ 1639.7433, found 1639.7404.

### Monocyclic octapeptide 70:

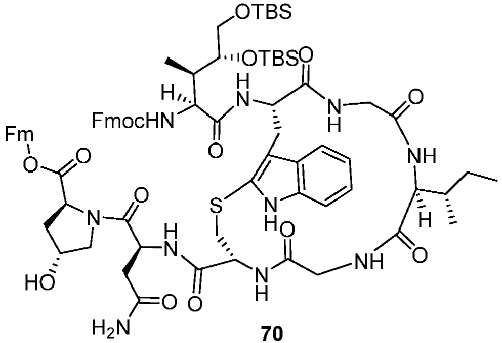

Crude monocyclic hexapeptide **68** (0.188 mmol, 1.00 eq.), dipeptide **68** (119 mg, 0.282 mmol, 1.50 eq.) were dissolved in DMF (3 mL). Then, DIPEA (49.1 mL, 0.282 mmol, 1.50 eq.) and HATU (108 mg, 0.282 mmol, 1.50 eq) were added at 0°C. The reaction mixture was allowed to warm to r.t. overnight and concentrated under reduced pressure. The crude product was purified by C18 reversed phase chromatography (AcN/H2O 60% to 100%) to fully protected octapeptide **70** as a white solid (170 mg, 59% over two steps).
**HRMS** (ESI): *m*/*z* calc. for C₈₀H₁₀₄N₁₀O₁₅SSi₂ (M+H)⁺ 1533.7015, found 1533.7004.

### Monocyclic octapeptide 71:

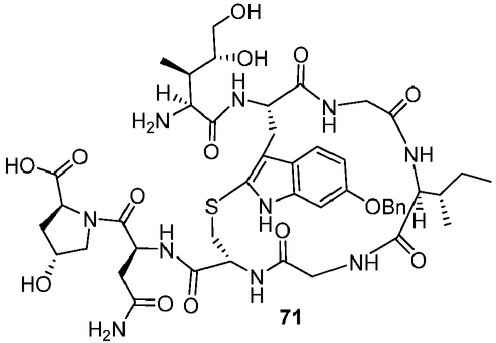

Monocyclic octapeptide **69** (15 mg, 10.3 µmol, 1.00 eq.) was dissolved in DMF (0.1 mL). Et₂NH (10.8 µL, 0.103 mmol, 10 eq.) was added and stirred for 2 h at r.t. The solvent was removed under reduced pressure and the precipitate was redissolved in THF (0.2 mL). Then, a solution of TBAF in THF (1 M, 0.10 mL, 10 eq) was added and the reaction mixture was stirred for 4 h at r.t.. The solvent was evaporated *in vacuo* and the crude product purified by C18 reverse phase chromatography (AcN/H₂O 5% to 70%) to afford the product **71** as a white solid (8 mg, 77%).
**HRMS** (ESI): *m*/*z* calc. for C₄₆H₆₂N₁₀O₁₄S (M+H)⁺ 1011.4240, found 1001.4241.

### Monocyclic octapeptide 72:

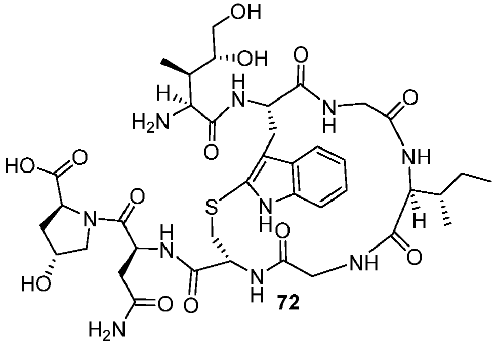

Monocyclic octapeptide **69** (20.0 mg, 14.8 µmol, 1.00 eq.) was dissolved in DMF (0.15 mL). Et₂NH (15.5 µL, 0.148 mmol, 10 eq.) was added and stirred for 2 h at r.t. The solvent was removed under reduced pressure and the precipitate was redissolved in THF (0.3 mL). Then, a solution of TBAF in THF (1 M, 0.15 mL, 10 eq) was added and the reaction mixture was stirred for 4 h at r.t.. The solvent was evaporated *in vacuo* and the crude product purified by C18 reverse phase chromatography (AcN/H₂O 5% to 30%) to afford the product **72** as a white solid (10 mg, 75%).
**HRMS** (ESI): *m*/*z* calc. for C₃₉H₅₆N₁₀O₁₃S (M+H)⁺ 905.3822, found 905.3810.

### (S)-Deoxy-(O)-benzyl-α-amanitin (61a) :

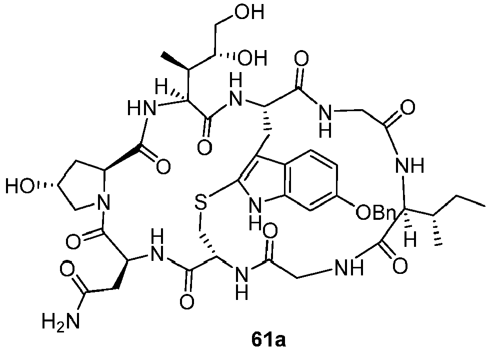

Monocyclic octapeptide **59** or **71** (20.0 mg, 19.2 µmol, 1.00 eq.) was dissolved in DMF (19 mL). Then, DIPEA (6.71 µL, 38.5 µmol, 2.00 eq.) and HATU (4.98 mg, 38.5 µmol, 2.00 eq) were added at 0°C. The reaction mixture was allowed to warm to r.t. overnight and concentrated under reduced pressure. The crude product was purified using preparative HPLC (Sunfire Prep C18 OBD 10µm, 50x150 mm column, gradient **C**) to afford *(O)*-Benzyl-α-amanitin (**61-a,** 13 mg, 68%) as a white powder.
**HRMS** (ESI): *m*/*z* calc. for C₄₆H₆₀N₁₀O₁₃S (M+H)⁺ 993.4135, found 993.4145.

### (S)-Deoxyamaninamide (72a) :

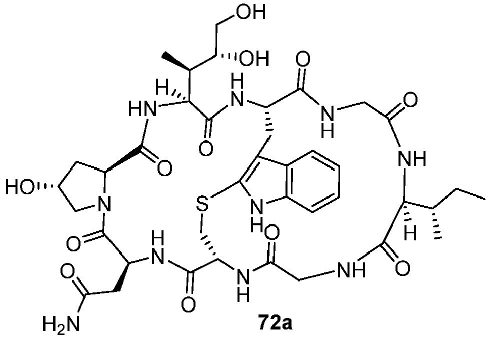

Monocyclic octapeptide **60** or **72** (20.0 mg, 21.4 µmol, 1.00 eq.) was dissolved in DMF (21 mL). Then, DIPEA (7.47 µL, 42.9 µmol, 2.00 eq.) and HATU (16.3 mg, 42.9 µmol, 2.00 eq) were added at 0°C. The reaction mixture was allowed to warm to r.t. overnight and concentrated under reduced pressure. The crude product was purified using preparative HPLC (Sunfire Prep C18 OBD 10µm, 50x150 mm column, gradient B) to afford (S)-Deoxyamaninamide (**72a,** 14 mg, 74%) as a white powder.
**HRMS** (ESI): *m*/*z* calc. for C₃₉H₅₄N₁₀O₁₂S (M+H)⁺ 887.3716, found 887.3718.

### (O)-Benzyl-α-amanitin (61b):

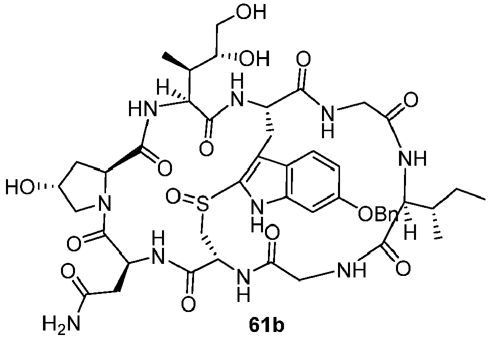

The prophyrine derived ligand (22 µg, 0.45 µmol, 0.3 eq.) and MnOTf₂ (16 µg, 0.45 µmol, 0.3 eq.) were dissolved in DCM (1 mL) and stirred for 3 h at r.t. Then Octapeptide **61a** (1.5 mg, 1.5 µmol, 1.0 eq.) dissolved in DMF (500 µL), AcOH (0.21 µL, 3.8 µmol, 2.5 eq.) and H₂O₂ (0.11 µL, 4.5 µmol, 3.0 eq.) were added. The reaction mixture was cooled down to 0°C and was stirred for 16 h at 0°C. The solvent was removed under reduced pressure and the crude product was used in the next step without further purification.
**HRMS** (ESI): *m*/*z* calc. for C₄₆H₆₀N₁₀O₁₄S (M+H)⁺ 1009.4084 found 1009.4118.

### Amaninamide (62):

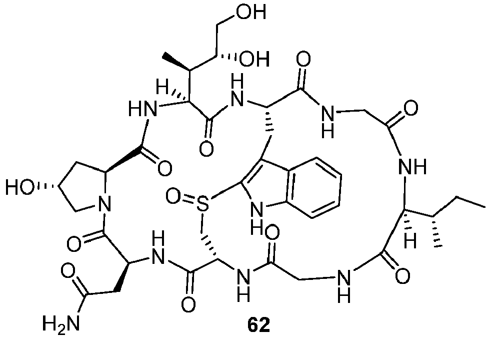

The prophyrine derived ligand (73 µg, 1.5 µmol, 0.3 eq.) and MnOTf₂ (53 µg, 1.5 µmol, 0.3 eq.) were dissolved in DCM (1 mL) and stirred for 3 h at r.t. Then Octapeptide **72** (5 mg, 5 µmol, 1.0 eq.) dissolved in DMF (500 µL), AcOH (0.700 µL, 12.7 µmol, 2.5 eq.) and H₂O₂ (0.37 µL, 15.0 µmol, 3.0 eq.) were added. The reaction mixture was cooled down to 0°C and was stirred for 16 h at 0°C. The solvent was removed under reduced pressure and the crude product was purified using preparative HPLC (Sunfire Prep C18 OBD 10µm, 50x150 mm column, gradient D) to afford amaninamide **62** as a white powder.
**HRMS** (ESI): *m*/*z* calc. for C₃₉H₅₄N1₀O₁₃S (M+H)⁺, found.

### α-Amanitin (61):

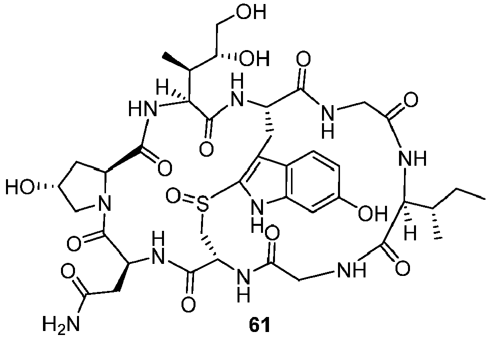

The crude product of **61b** was dissolved in THF/H2O (2:1) and Pd/C (1 mg) was added. The reaction mixture was flushed with N₂ for 10 min, then with H₂ for 10 min and was stirred for 2 h at r.t. The solvent was removed under reduced pressure and the crude product was purified using preparative HPLC (Sunfire Prep C18 OBD 10µm, 50x150 mm column) to afford α-*Amanitin* (**61**) as a white powder.
**HRMS** (ESI): *m*/*z* calc. for C₃₉H₅₄N₁₀O₁₄S (M+H)⁺919.3614 found 919.3614.

### Preparative HPLC purification gradients:

Gradient **A:** 0-25 min 40%-60% B, 25-35 min 100% B; 35-40 min 40%B
   0.1% formic acid in water (Solvent A) and 0.1% formic acid in ACN (Solvent B).
Gradient **B:** 0-30 min 10%-30% B, 30-40 min 100% B; 40-50 min 10%B
   0.1% formic acid in water (Solvent A) and 0.1% formic acid in ACN (Solvent B).
Gradient **C:** 0-60 min 5%-50% B, 60-65 min 100% B; 65-70 min 5%B
   0.1% formic acid in water (Solvent A) and 0.1% formic acid in ACN (Solvent B).

### Solid-phase peptide synthesis

Automated or manual solid-phase peptide synthesis was performed in 50 µmol scale. Loading: To a 10 ml syringe reactor with frit and cap 1g of tritylchloride polystyrene (TCP) resin (0.9 mmol/g) were added and 7 ml drydichloromethane(DCM) . For resin loading with the first amino acids, the resin was pre-swollen for 10 min and the solvent was removed by evaporation in vacuum. A mixture of the amino acids (0.6 mmol) and 3 equivalents of N,N-diisopropylamine (DIPEA) dissolved in 5 ml dry DCM was added to the resin. The syringe was agitated for 30 min at room temperature. The solution was removed and the resin was washed (2x 5ml N,N-dimethylformamide(DMF), 2x 5 mlDCM). Capping of non-reacted functional groups of the resin was performed with DCM, methanol and DIPEA 80:15:5 (2x 10 ml, 10min). After washing (5x 5 ml DMF), Fmoc-removal was achieved with DMF/piperidine (4:1, 5 ml, 1x 2 min, 1x 20 min). After final washing (2x 5 ml DMF, 1x 5ml methanol, 3x 5 ml DCM), the resin was dried in vacuo. Coupling of Fmoc protected amino acids: To 200 mg of the resin (∼0.5mmol/g), a 0.25M solution of the amino acid in DMF (2.5 eq relative to resin loading) was added. After addition of a 0.5M solution of DIPEA in DMF (2.5eq) and a 0.25 M solution of O-(benzotriazol-1-yl)-N,N,N',N'-tetramethyluronium tetrafluoroborate TBTU in DMF (2.5 eq), the reaction solution was mixed for 15min. A second coupling was performed for 15min. Fmoc removal: DMF/piperidine (4:1, 2.5ml) was added to the resin and mixed for 2.5min. The procedure was repeated 4 times. The resin was washed with DMF (6x 2.5 ml). After the final coupling cycle, the resin was washed with DCM (6x 2 ml).Cleavage: After addition of the cleavage cocktail (DCM/HFIP 4:1, the syringe was shaken for 30min. The solution was transferred to a flask and the solvent was removed *in vacuo..* Further instructions can be found in (Amblard M, Fehrentz JA, Martinez J, Subra G. Mol Biotechnol. 2006 Jul;33(3):239-54).

### Abbreviations

BF₃*Et₂O: boron trifluoride etherate
Bn: benzyl
Boc: tert-butyloxycarbonyl
BMIM-PF₆: 1-butyl-3-methylimidazolium hexafluorophosphate
Cbz: benzyloxycarbonyl
COMBU: 4-{[1,3-Dimethyl-2,4,6-trioxotetrahydropyrimidin-5(6*H*)ylidenaminooxy](dimethylamino)methylen}morpholin-4-iumhexafluorophosphate
COMU: (1-Cyano-2-ethoxy-2-oxoethylidenaminooxy)dimethylamino-morpholino-carbenium-hexafluorophosphate
[(p-cymene) RuCl₂]₂: (cymene)ruthenium dichloride dimer
DCM: dichloromethane
DHIL: dihydroxy-isoleucine
DIPEA: N,N-diisopropylethylamine
DMA: dimethylacetamide
DMF: dimethylformamide
(DHQD)₂PHAL: hydroquinidine 1,4-phthalazinediyl diether
Fm - 9-Fluorenylmethyl
Fmoc: fluorenylmethyloxycarbonyl
Fmoc-OSu: 9-Fluorenylmethyl N-succinimidyl carbonate
HATU: 1-[Bis(dimethylamino)methylene]-1H-1,2,3-triazolo[4,5-b]pyridinium 3-oxid hexafluorophosphate; Hexafluorophosphate Azabenzotriazole Tetramethyl Uronium
HBTU: 2-(1*H*-Benzotriazol-1-yl)-1,1,3,3-tetramethyluronium-hexafluorophosphate
HCTU: 2-(6-Chlor-1*H*-benzotriazol-1-yl)-1,1,3,3-tetramethylaminium-hexafluorophosphate
Hyp: trans-4-hydroxy-proline
LHMDS: lithium bis(trimethylsilyl)amide
MSA: N-Methyl-N-trimethylsilylacetamid
NMO: 4-methylmorpholine 4-oxide
PPh₃: triphenylphosphine
PPO: Phthaloyl peroxide
T3P: 2,4,6-tripropyl-1,3,5,2,4,6-trioxatriphosphorinane-2,4,6-trioxide
TBAF: tetra-n-butylammonium fluoride
tBuOAc: tert-butyl-acetate
TBS: tert.-butyldimethylsilyl
TBTU: 2-(1H-Benzotriazole-1-yl)-1,1,3,3-tetramethylaminium tetrafluoroborate
Tce: trichloroethyl
Teoc: 2-(Trimethylsilyl)ethoxycarbonyl
TFA: trifluoroacetic acid
THF: tetrathydrofuran
TMSOTf: trimethylsilyl trifluoromethanesulfonate
TOMBU: N-{[1,3-Dimethyl-2,4,6-trioxotetrahydropyrimidin-5(6H)-ylidenaminooxy](dimethylamino)methylen}-N-methylmethanaminiumhexafluorophosphate

## Claims

1. A method for preparation of a compound of formula (lox) 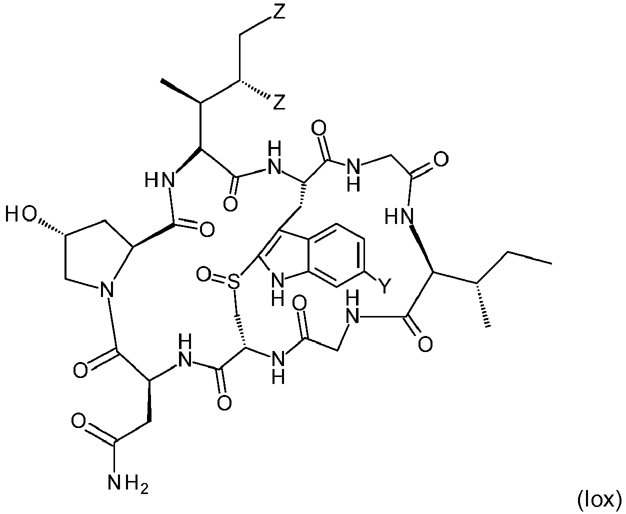 wherein
a) a compound of formula (IIox) 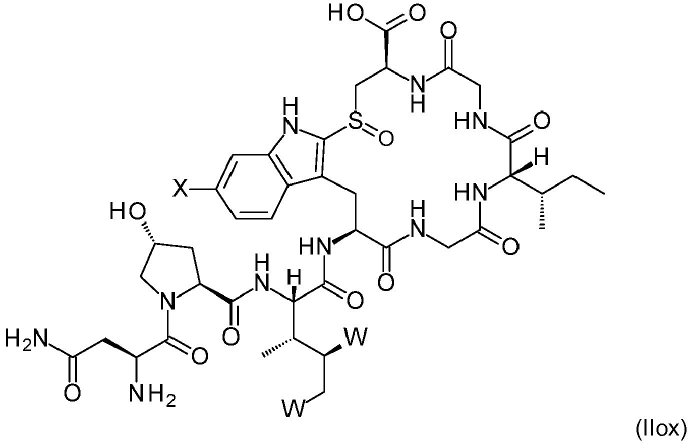 wherein
• X and Y are H, or
Y is OH and X is OR^{PGP} wherein R^{PGP} is a protecting group for phenolic OH groups, particularly a phenolic OH-protecting group not acid- or alkali-labile, more particularly cleavable under reductive conditions, or
X and Y are selected from F, Cl, Br, and I,
particularly X and Y are H, or Y is OH and X is OR^{PGP}
• Z and W are H, or
Z is OH and W is OR^{PGOH}, wherein R^{PGOH} is a protecting group for hydroxyl-groups, particularly a hydroxyl-protecting group cleavable with fluoride ions,is reacted with a peptide bond forming reagent,
particularly with a coupling reagent selected from a carbodiimide, an imidazolinium reagent, a phosphonium salt, an organo-phosphorous reagent, an uronium salt, a pyridinium reagent, and a phosphonic acid,
more particularly with HATU, COMU, HBTU, TBTU, TOMBU, COMBU, or HCTU,
in a reaction step (a1),
and for Y being OH and/or Z being OH, the compound is reacted with a deprotection agent removing R^{PGP} and/or R^{PGOH},
or wherein
b) the compound of formula (II) 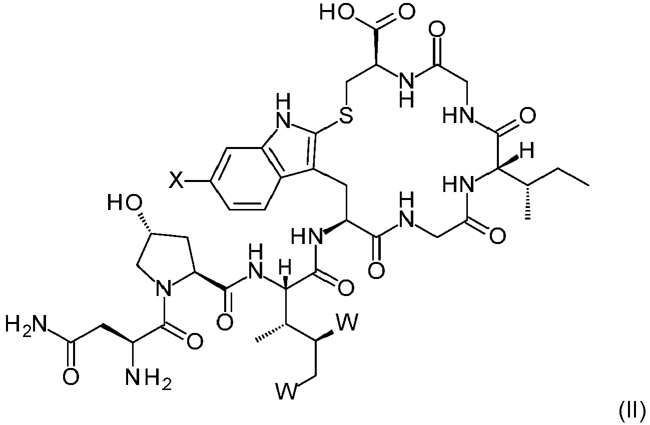 wherein
• X, Y, Z and W have the same meanings as defined above,
is reacted with a peptide bond forming reagent, particularly with HATU, in a reaction step (a2),
yielding a compound of formula (I) 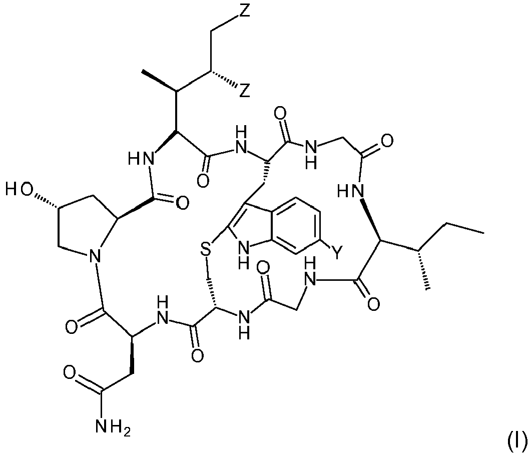 wherein the sulfur atom is subsequently oxidized, particularly
i. using manganese ions, more particularly the compound is reacted with a compound of formula (XXII) 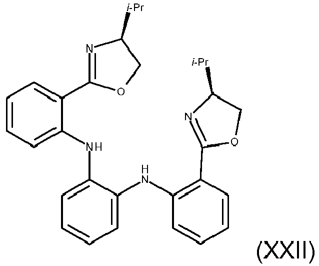 and with Mn(OTf)₂ and H₂O₂,
ii. using PPO, dibenzyolperoxide, tert-butyl peroxybenzoate, or lauroyl peroxide; or
iii. using iodine and oxygen;
in a reaction step (b2);
and for Y being OH and/or Z being OH, the compound is reacted with a deprotection agent removing R^{PGP} and/or R^{PGOH}, particularly for R^{PGP} with reductive conditions and for R^{PGOH} with fluoride ions,
to yield the compound **characterized by** (lox).

2. A method for preparation of a compound of formula (I) 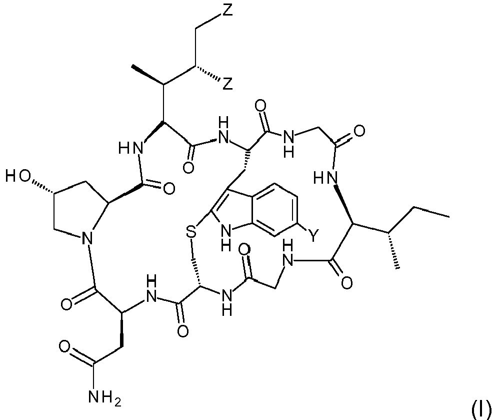 wherein a compound of formula (II) 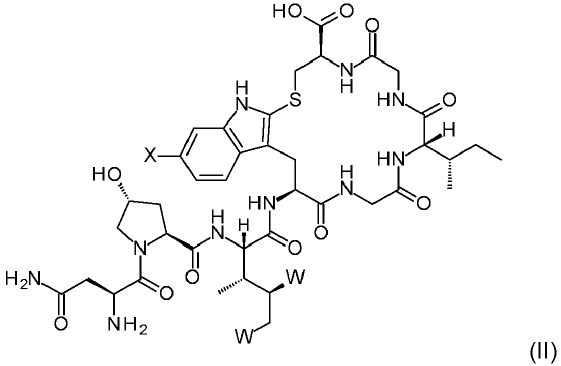 wherein
• X and Y are H, or
Y is OH and X is OR^{PGP} wherein R^{PGP} is a protecting group for phenolic OH groups, particularly a phenolic OH-protecting group not acid- or alkali-labile, more particularly cleavable under reductive conditions, or
X and Y are selected from F, Cl, Br, and I,
particularly X and Y are H or Y is OH and X is OR^{PGP}
• Z and W are H, or
Z is OH and W is OR^{PGOH}, wherein R^{PGOH} is a protecting group for hydroxyl-groups, particularly a hydroxyl-protecting group cleavable with fluoride ions,
is reacted with a peptide bond forming reagent,
particularly with a coupling reagent selected from a carbodiimide, an imidazolinium reagent, a phosphonium salt, an organo-phosphorous reagent, an uronium salt, a pyridinium reagent, and a phosphonic acid,
more particularly with HATU, COMU, HBTU, TBTU, TOMBU, COMBU, or HCTU in a reaction step (a),
and for Y being OH and/or Z being OH, the compound is reacted with a deprotection agent removing R^{PGP} and/or R^{PGOH} in a reaction step (b)
to yield the compound **characterized by** (I).

3. The method according to claim 1 or 2, wherein a compound of formula (III) 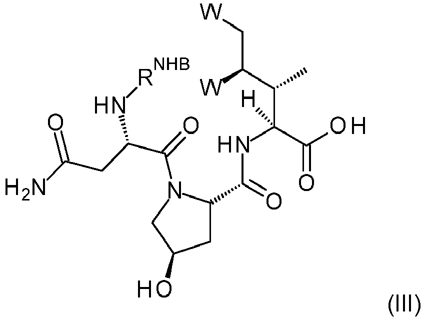 and a compound of formula (IV) or (IVox) 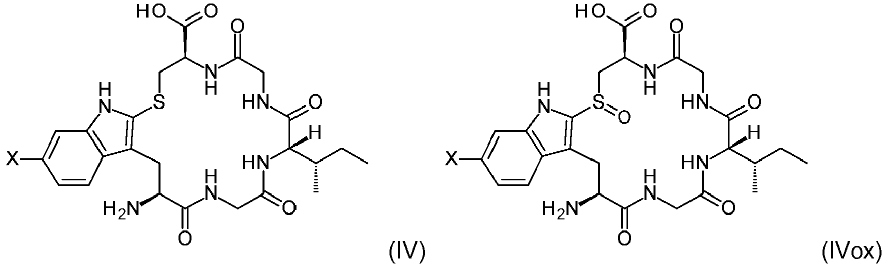 wherein
- R^{NHB} is an amino protecting group, particularly an amino protecting group cleavable under alkaline conditions, more particularly Fmoc, or an amino protecting group cleavable with hydrogenolysis, particularly Cbz, most particularly R^{NHB} is Fmoc,
- W and X have the same meaning as outlined in claim 1,
wherein
• the amino-group of (IV) or (IVox) is preactivated, particularly with MSA, and preactivated (IV) or preactivated (IVox) and (III) are reacted with a peptide bond forming reagent, particularly with HATU, or
• the amino-group of (IV) or (IVox) is preactivated, particularly with MSA, and the carboxyl-group of compound (III) is preactivated, particularly with an O-PFP-ester, O-PCP-ester, or OSu-ester, and preactivated (IV) or preactivated (IVox) and preactivated (III) are reacted,
in a reaction step (c),
and the compound is reacted with a deprotection agent removing R^{NHB} in a reaction step (d), particularly with a base if R^{NHB} is Fmoc, or with hydrogenolysis if R^{NHB} is Cbz,
to yield the compound **characterized by** (II) or (IIox).

4. The method according to claim 3, wherein a compound of formula (IV) 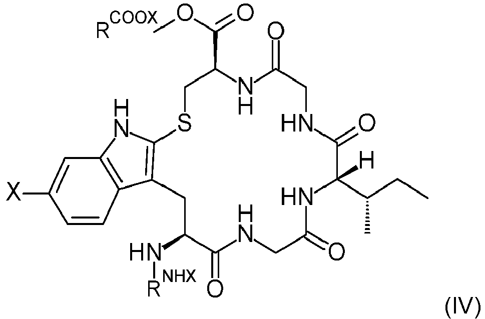 wherein
- R^{COOX} is a carboxyl-protecting group, particularly *t*Butyl,
- R^{NHX} is an amino-protecting group, particularly Teoc or Fmoc, more particularly Teoc,
- X has the same meaning as outlined in claim 1,
wherein the sulfur atom is subsequently oxidized, particularly
i. using manganese ions, more particularly the compound is reacted with a compound of formula (XXII) 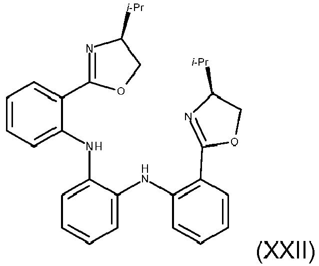 and with Mn(OTf)₂ and H₂O₂,
ii. using PPO, dibenzyolperoxide, tert-butyl peroxybenzoate, or lauroyl peroxide; or
iii. using iodine and oxygen;
in a reaction step (d2),
and the compound is reacted with a deprotection agent removing R^{COOX}, particularly with a strong acid, more particularly with TFA,
and with a deprotection agent removing R^{NHX}, particularly in case of R^{NHX} being Teoc with a strong acid, more particularly with TFA, or in case of R^{NHX} being Fmoc with alkaline conditions,
to yield the compound **characterized by** (IVox).

5. The method according to claim 4, wherein a compound of formula (V) 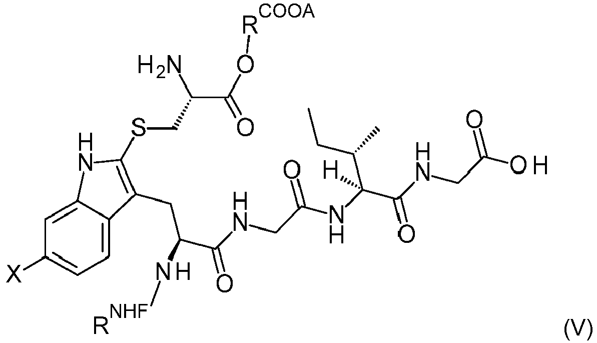 wherein
- R^{NHF} is an amino protecting group, particularly an amino protecting group cleavable with fluoride ions or strong acids, more particularly Teoc, or an amino protecting group cleavable with alkaline conditions, more particularly Fmoc,
- R^{COOA} is a carboxyl-protecting group, particularly a carboxyl-protecting group cleavable under strongly acidic conditions, more particularly tert-butyl,
- X has the same meaning as outlined in claim 1,
is reacted with a peptide bond forming reagent, particularly with a coupling reagent selected from a carbodiimide, an imidazolinium reagent, a phosphonium salt, an organo-phosphorous reagent, an uronium salt, a pyridinium reagent, and a phosphonic acid, more particularly with T3P, HATU, COMU, HBTU, TBTU, TOMBU, COMBU, or HCTU, in a reaction step (e),
and the compound is reacted with a deprotection agent removing R^{NHF} and R^{COOA} in a reaction step (f), particularly with TFA,
to yield the compound **characterized by** (IV).

6. The method according to claim 5, wherein a compound of formula (VI) 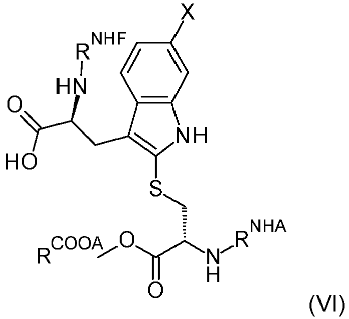 and a compound of formula (VII) 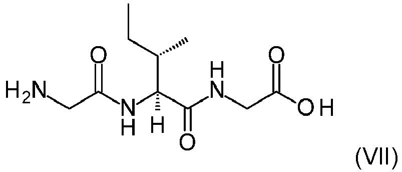 wherein
- R^{NHA} is an amino protecting group, particularly an amino protecting group cleavable under acidic conditions, more particularly Boc,
- R^{COOA} , R^{NHF} and X have the same meaning as outlined in claim 1 and 5,
wherein compound (VI) is
• preactivated with a peptide bond forming reagent, particularly with HATU, COMU, HBTU, TBTU, TOMBU, COMBU, or HCTU, followed by a reaction with the silylated compound (VII), or
• is preactivated as in OSu-ester, followed by a reaction with the compound (VII) in a reaction step (g),
and the compound is reacted with a deprotection agent removing R^{NHA} in a reaction step (h), particularly with acidic conditions,
to yield the compound **characterized by** (V).

7. The method according to claim 6, wherein a compound of formula (VIII) 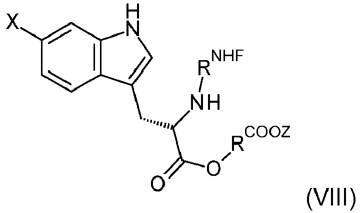 and a compound of formula (IX) 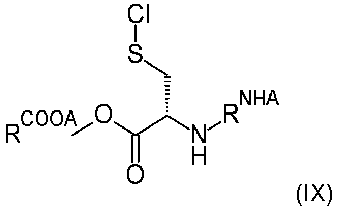 wherein
- R^{COOZ} is a carboxyl-protecting group, particularly a carboxyl-protecting group cleavable with Zn, more particularly Tce, or R^{COOZ} is H,
- R^{COOA}, R^{NHF}, R^{NHA} and X have the same meaning as outlined in claim 1, 5, and 6,
are reacted in a reaction step (i), and if R^{COOZ} is a carboxyl-protecting group, the compound is reacted with a deprotection agent removing R^{COOZ} in a reaction step (j), particularly with Zn,
to yield the compound **characterized by** (VI).

8. The method according to claim 3, wherein a compound of formula (X) 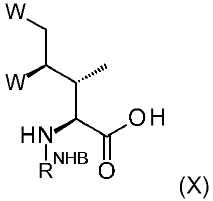 and a compound of formula (XI) 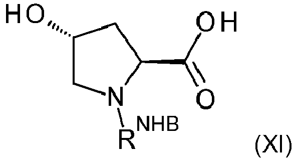 and a compound of formula (XII) 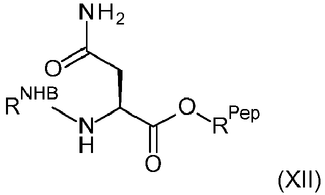 wherein
- R^{Pep} is an active ester, particularly O-pentafluorophenol or OSu-ester,
- R^{NHB} is an amino protecting group, particularly an amino protecting group cleavable under alkaline conditions, more particularly Fmoc,
are reacted with solid phase peptide synthesis in a reaction step (k),
to yield the compound **characterized by** (III).

9. A method for preparation of a compound of formula (XIII), (XIIIC), (XIIIN), or (XIIICN) 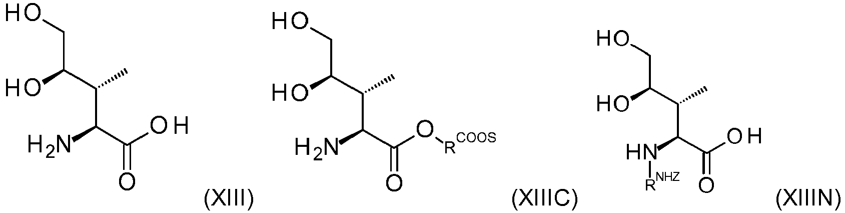 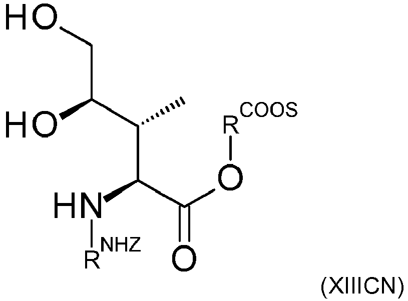 wherein a compound of formula (XIV) 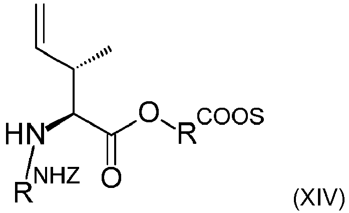 wherein
- R^{COOS} is a carboxyl-protecting group, particularly tert-butyl,
- R^{NHZ} is an amino protecting group, particularly an amino protecting group cleavable under alkaline conditions, more particularly Fmoc, or an amino protecting group cleavable under reductive conditions, more particularly trifluoroacetyl;
is reacted with Osmium(IV)-oxide in a reaction step (I), particularly in CHCl₃/H₂O, and optionally, the compound is reacted with a deprotection agent removing R^{NHR} and/or R^{COOS} in a reaction step (m),
particularly with silylating agents for R^{COOS} and reductive conditions or alkaline conditions for R^{NHR},
more particularly with TMSOTf and lutidine for R^{COOS} and/or sodium borohydride or alkaline conditions for R^{NHZ}
to yield the compound **characterized by** (XIII), (XIIIC), (XIIIN), or (XIIICN).

10. A method for preparation of a compound of formula (XV) 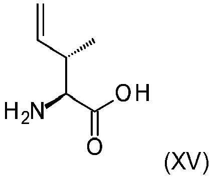 wherein a compound of formula (XVI) 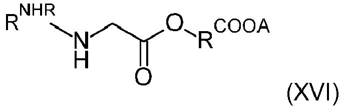 and a compound of formula (XVII) or (XVIIs) 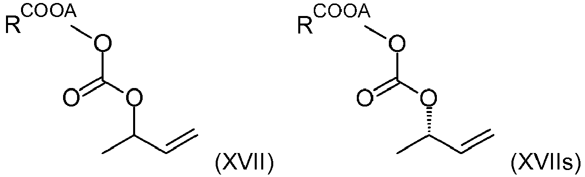 wherein
- R^{NHR} an amino protecting group cleavable under reductive conditions, more particularly trifluoroacetyl,
- R^{COOA} is a carboxyl-protecting group, particularly a carboxyl-protecting group cleavable under strongly acidic conditions, more particularly *tert*-butyl,
are reacted with [(p-cymene)RuCl₂]₂ in a reaction step (n) yielding the compound (XXIII) or (XXIV) 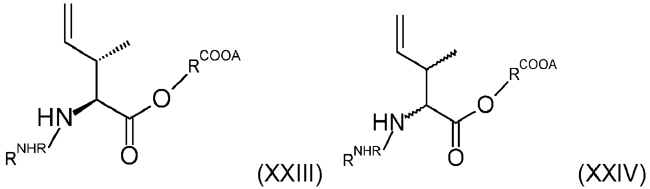
a) the compound (XXVI) is reacted with a deprotection agent removing R^{COOA} in a reaction step (o), and is reacted with acylase in a reaction step (p), or
b) the compound (XXIII) is reacted with a deprotection agent removing R^{COOA} in a reaction step (o), and is reacted with a deprotection agent removing R^{NHR} in a reaction step (q), particularly with reductive conditions,
to yield the compound **characterized by** (XV).

11. A method for preparation of a compound of formula (XVIII) 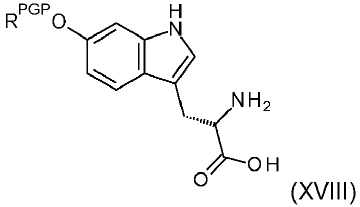 wherein a compound of formula (XIX) 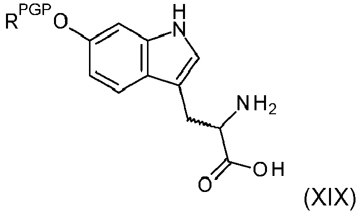 and a compound of formula (XX) 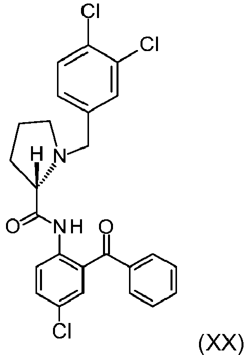 wherein
- R^{PGP} is a protecting group for phenolic OH groups, particularly a phenolic OH-protecting group not acid- or alkali-labile, more particularly cleavable under reductive conditions,
are reacted with Ni²⁺ in a reaction step (r)
to yield the compound **characterized by** (XVIII).

12. A method for preparation of a compound of formula (lox), wherein a compound of formula (I) 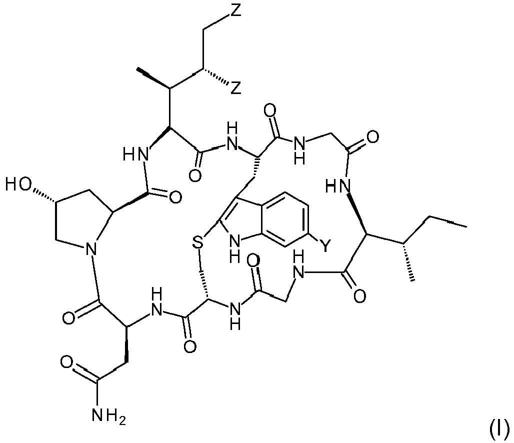 wherein the sulfur atom is oxidized,
i. using manganese ions, more particularly the compound is reacted with a compound of formula (XXII) 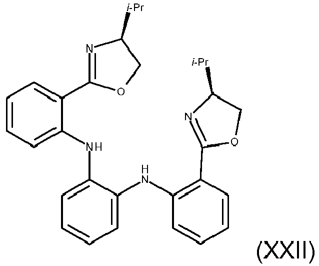 and with Mn(OTf)₂ and H₂O₂,
ii. using PPO, dibenzyolperoxide, tert-butyl peroxybenzoate, or lauroyl peroxide; or
iii. using iodine and oxygen;
yielding the compound (lox).

13. A method for preparation of a compound of formula (XXIII) or (XXIIIox) 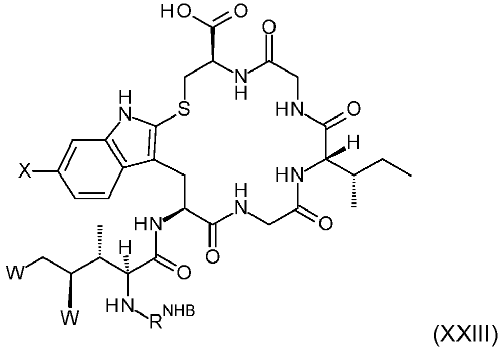 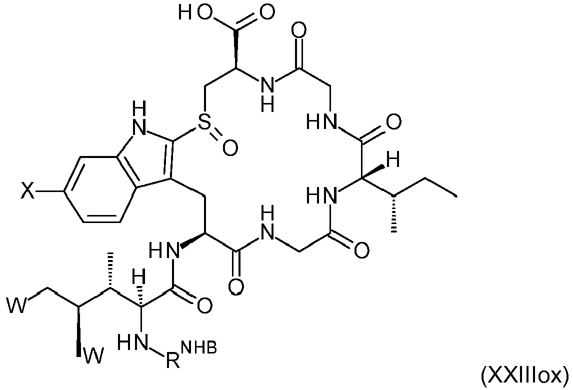 wherein a compound of formula (IV) or (IVox), respectively, 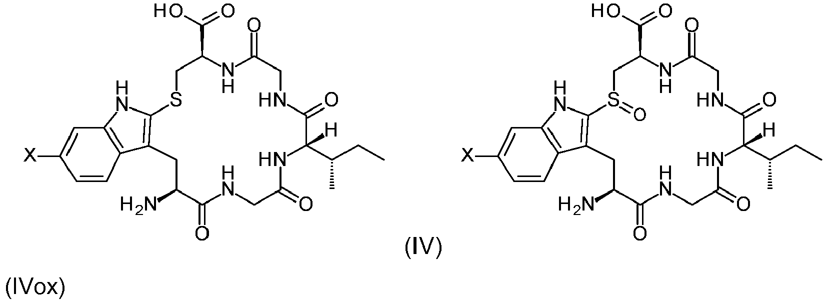 and a compound of formula (X) 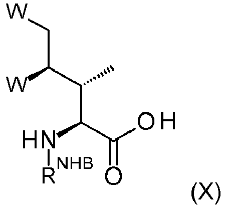 wherein X, W, and R^{NHB} have the same meanings as defined in claim 1 and 3, wherein the amino-group of (IV) or (IVox) is preactivated, particularly with MSA, and preactivated (IV) or preactivated (IVox) and (X) are reacted with a peptide bond forming reagent, particularly with HATU, COMU, HBTU, TBTU, TOMBU, COMBU, or HCTU, more particularly with COMU, in a reaction step (s) to yield the compound (XXIII) or (XXIIIox), respectively.

14. A method for preparation of a compound of formula (XXVI) or (XXVIox) 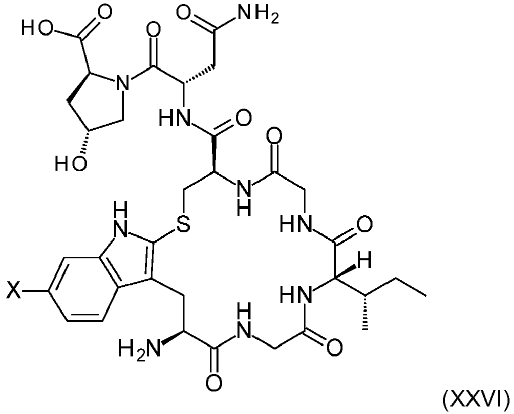 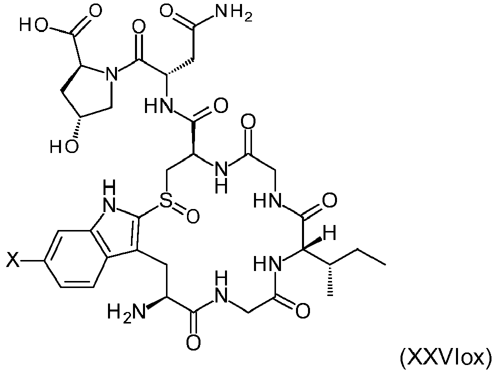 wherein a compound of formula (XXVIII) or (XXVIIIox), respectively, 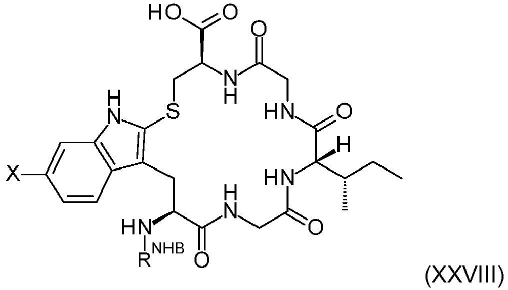 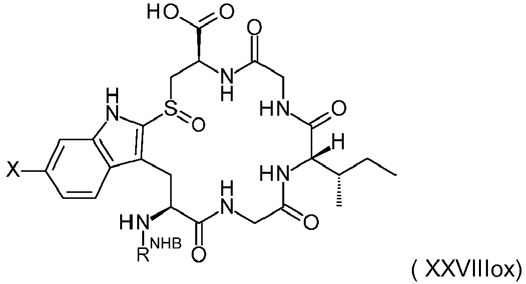 and a compound of formula (XXV) 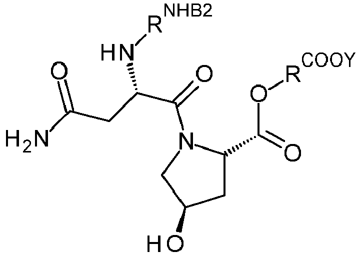 wherein
• X, W, and R^{NHB} have the same meanings as defined in claim 1 and 3,
• R^{NHB2} is an amino-protecting group, particularly an amino-protecting group cleavable under acidic conditions, more particularly Boc;
• R^{COOY} is a carboxyl-protecting group, particularly fluorenylmethyl or benzyl, more particularly fluorenylmethyl;
wherein (IV) or (IVox) and (XXV) are reacted with a peptide bond forming reagent, particularly with HATU, COMU, HBTU, TBTU, TOMBU, COMBU, or HCTU, in a reaction step (t) to yield the compound (XXVI) or (XXVIox), respectively.

15. A method for preparation of a compound of formula (XXVII) or (XXVIIox) 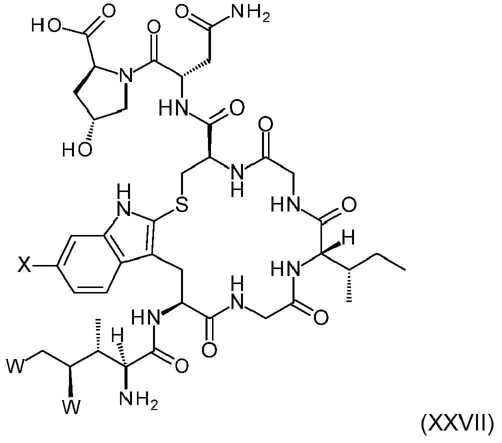 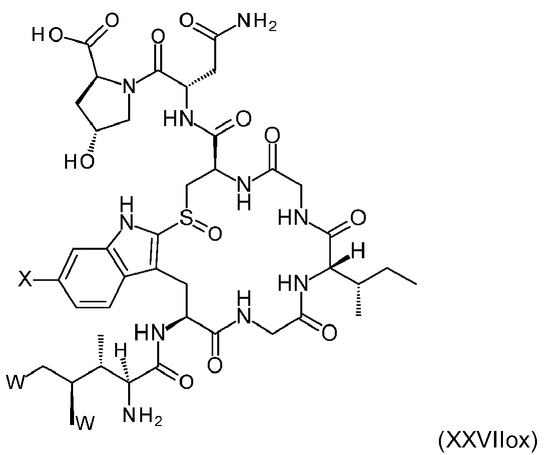 wherein
a) a compound of formula (IV) or (IVox), 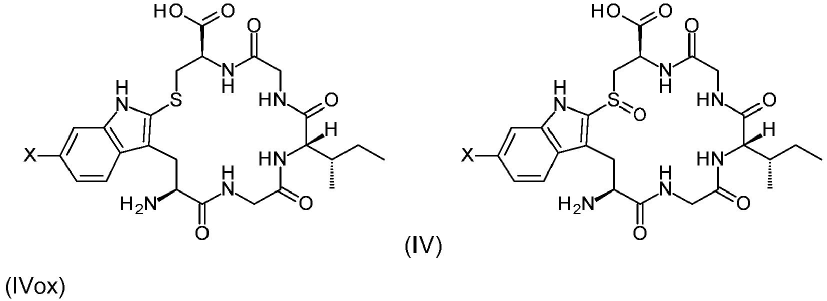 and a compound of formula (X) 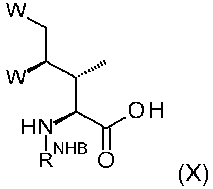
wherein X, W, and R^{NHB} have the same meanings as defined in claim 1 and 3,
wherein the amino-group of (IV) or (IVox) is preactivated, particularly with MSA, and preactivated (IV) or preactivated (IVox) and (X) are reacted with a peptide bond forming reagent, particularly with COMU, in a reaction step (s) to yield the compound (XXIII) or (XXIIIox), respectively,
and subsequently compound (XXIII) or (XXIIIox) and compound (XXV) are reacted with a peptide bond forming reagent, particularly with HATU, COMU, HBTU, TBTU, TOMBU, COMBU, or HCTU
in a reaction step (u) to yield the compound (XXVII) or (XXVIlox), respectively;
or
b) a compound (XXIII) or (XXIIIox) and a compound (XXV) are reacted with a peptide bond forming reagent, particularly with HATU, COMU, HBTU, TBTU, TOMBU, COMBU, or HCTU
in a reaction step (u) to yield the compound (XXVII) or (XXVIlox), respectively;
or
c) a compound of formula (XXVIII) or (XXVIIIox), respectively, 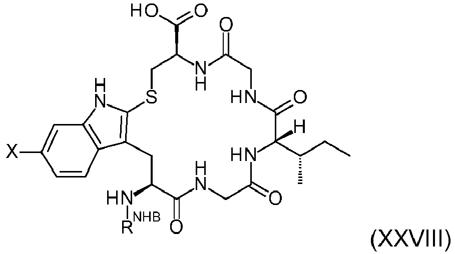 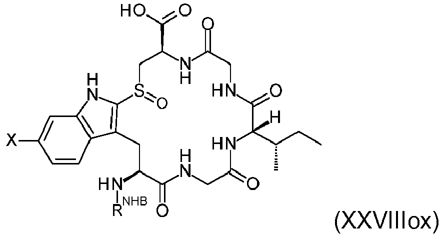 and a compound of formula (XXIX) 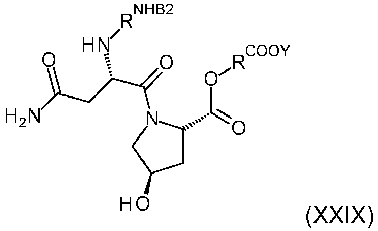 wherein
• X, W, and R^{NHB} have the same meanings as defined in claim 1 and 3,
• R^{NHB2} is an amino-protecting group, particularly an amino-protecting group cleavable under acidic conditions, more particularly Boc;
• wherein R^{COOY} is a carboxyl-protecting group, particularly fluorenylmethyl or benzyl, more particularly fluorenylmethyl;
wherein (XXVIII) or (XXVIIIox) and (XXV) are reacted with a peptide bond forming reagent, particularly with HATU, COMU, HBTU, TBTU, TOMBU, COMBU, or HCTU,
in a reaction step (t) to yield the compound (XXVI) or (XXVIox), respectively and subsequently compound (XXVI) or (XXVIox) and compound (X) are reacted with a peptide bond forming reagent, particularly with HATU, in a reaction step (v) to yield the compound (XXVII) or (XXVIlox), respectively
or
d) a compound (XXVI) or (XXVIox) and a compound (X) are reacted with a peptide bond forming reagent, particularly with HATU, in a reaction step (v) to yield the compound (XXVII) or (XXVIlox), respectively.

16. A compound of the general formula (I) 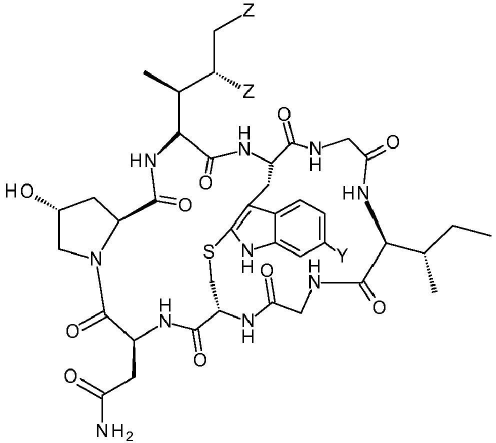 wherein
- Y is H and Z is H,
- Y is H and Z is OH,
- Y is OH and Z is H
- Y is F, Cl, I or Br, and Z is OH, or
- Y is F, Cl, I or Br, and Z is H,
- particularly Y and Z are independently selected from OH and H;
or a compound of the general formula (II) 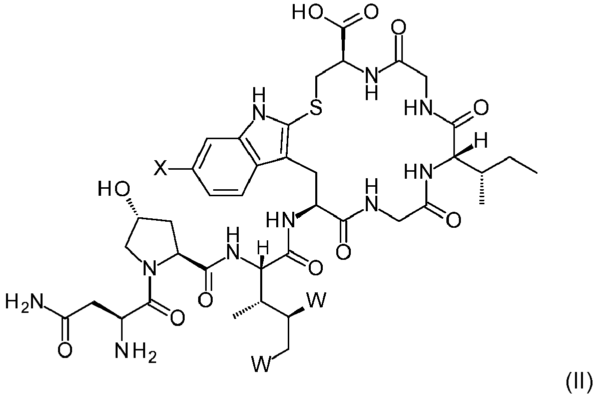 wherein
- X is H and W is H,
- X is OH and W is OH,
- X is H and W is OH,
- X is OH and W is H,
- X is F, Cl, I or Br, and W is OH, or
- X is F, Cl, I or Br, and W is H,
- particularly X and W are independently selected from OH and H;
or a compound of the general formula (IIox) 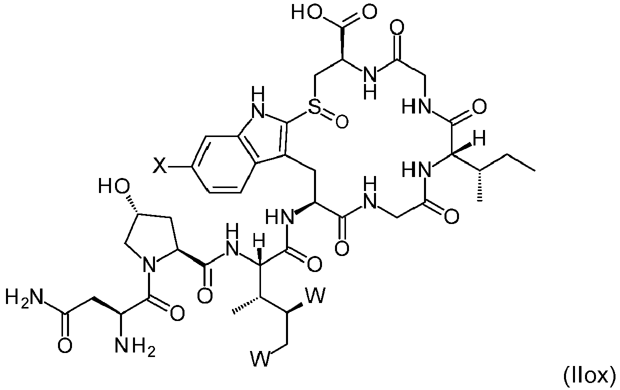 wherein
- X is H and W is H,
- X is OH and W is OH,
- X is H and W is OH,
- X is OH and W is H,
- X is F, Cl, I or Br, and W is OH, or
- X is F, Cl, I or Br, and W is H,
- particularly X and W are independently selected from OH and H;
or a compound of the general formula (IVox) 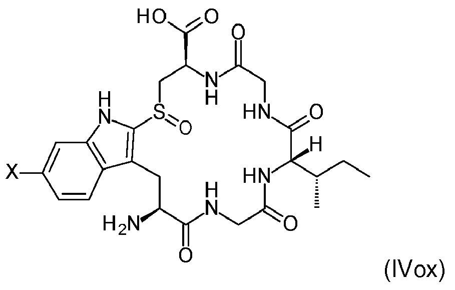 wherein
- X is H or OH, or
- X is F, Cl, I or Br
- particularly X is H or OH;
or a compound of the general formula (XXVIII) 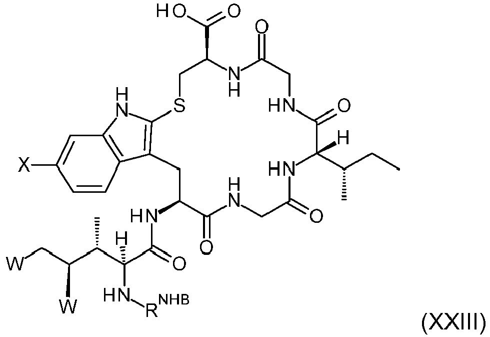 wherein
- X is H and W is H,
- X is OH and W is OH,
- X is H and W is OH,
- X is OH and W is H,
- X is F, Cl, I or Br, and W is OH, or
- X is F, Cl, I or Br, and W is H,
- particularly X and W are independently selected from OH and H;
or a compound of the general formula (XXVIIIox) 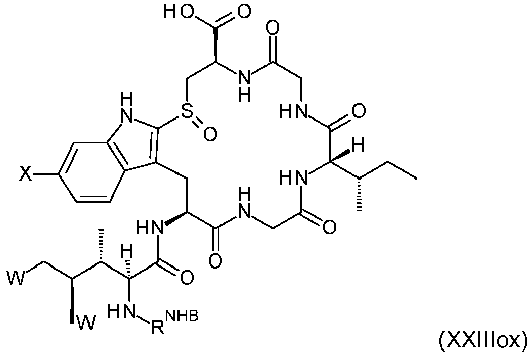 wherein
- X is H and W is H,
- X is OH and W is OH,
- X is H and W is OH,
- X is OH and W is H,
- X is F, Cl, I or Br, and W is OH, or
- X is F, Cl, I or Br, and W is H,
- particularly X and W are independently selected from OH and H;
or a compound of the general formula (XXVI) 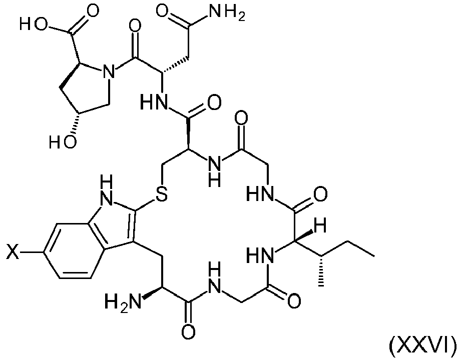 wherein
- X is H and W is H,
- X is H and W is OH,
- X is OH and W is H,
- X is F, Cl, I or Br, and W is OH, or
- X is F, Cl, I or Br, and W is H,
- particularly X is H and W is H, or X is H and W is OH, or X is OH and W is H;
or a compound of the general formula (XXVIox) 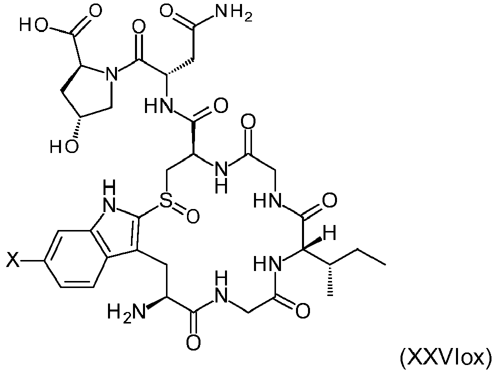 wherein
- X is H and W is H,
- X is OH and W is OH,
- X is H and W is OH,
- X is OH and W is H,
- X is F, Cl, I or Br, and W is OH, or
- X is F, Cl, I or Br, and W is H,
- particularly X and W are independently selected from OH and H.
